# EUROPEAN PATENT APPLICATION

(11) **EP 2 017 287 A2**
(43) Date of publication of application: **21.01.2009**
(21) Application number: 08012908.3
(22) Date of filing: 11.09.2003
(51) Int. Cl.: C07K 14/505, C07K 17/10, C08B 31/00, C07K 17/06, C12N 15/12, A61K 38/18, C07K 14/55, C07K 14/54, C07K 14/56, C07K 14/565, C07K 14/53

(54) **Method of producing hydroxyalkyl starch derivatives**

(30) Priority: 11.09.2002 EP 02020425; 11.09.2002 US 409781 P; 08.08.2003 WO PCT/EP03/08829
(62) Divisional of application: 03020424.2
(71) Applicant: Fresenius Kabi Deutschland GmbH, 61352 Bad Homburg (DE)
(72) Inventor: Zander, Norbert, 38527 Meine (DE)
(74) Representative: Wichmann, Hendrik

(57) **Abstract**

Method of producing a hydroxyalkyl starch derivative, which derivative comprises a functional group X capable of being reacted with a functional group Y of a further compound (M), wherein said functional group Y of the further compound (M) is a thio group.

## Description

The present invention relates to hydroxyalkyl starch derivates, particularly hydroxyalkyl starch derivatives obtainable by a process in which hydroxyalkyl starch is reacted with a primary or secondary amino group of a crosslinking compound or with two crosslinking compounds wherein the resulting hydroxaylkyl starch derivative has at least one functional group X which is capable of being reacted with a functional group Y of a further compound and wherein this group Y is an aldehyd group, a keto group, a hemiacetal group, an acetal group, or a thio group. According to an especially preferred embodiment, the present invention relates to hydroxyalkyl starch derivatives obtainable by a process according to which hydroxyalkyl starch is reacted with a primary or secondary amino group of a crosslinking compound, the resulting reaction product optionally being further reacted with a second crosslinking compound, wherein the resulting hydroxaylkyl starch derivative has at least one functional group X which is capable of being reacted with a functional group Y of a further compound and wherein this group Y is an aldehyd group, a keto group, a hemiacetal group, an acetal group, or a thio group, and the resulting reaction product is reacted with a polypeptide, preferably with a polypeptide such as AT III, IFN-beta or erythropoietin and especially preferably with erythropoietin, which comprises at least one of these functional groups Y. A hydroxyalkyl starch which is especially preferred is hydroxyethyl starch. According to the present invention, the hydroxyalkyl starch and preferably the hydroxyl ethyl starch is reacted with the linker compound at its reducing end which is optionally oxidized prior to said reaction.

Hydroxyethyl starch (HES) is a derivative of naturally occurring amylopectin and is degraded by alpha-amylase in the body. HES is a substituted derivative of the carbohydrate polymer amylopectin, which is present in corn starch at a concentration of up to 95 % by weight. HES exhibits advantageous biological properties and is used as a blood volume replacement agent and in hemodilution therapy in the clinics (Sommermeyer et al., 1987, Krankenhauspharmazie, 8(8), 271-278; and Weidler et al., 1991, Arzneim.-Forschung/Drug Res., 41, 494-498).

Amylopectin consists of glucose moieties, wherein in the main chain alpha-1,4-glycosidic bonds are present and at the branching sites alpha-1,6-glycosidic bonds are found. The physical-chemical properties of this molecule are mainly determined by the type of glycosidic bonds. Due to the nicked alpha-1,4-glycosidic bond, helical structures with about six glucose-monomers per turn are produced. The physicochemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

Some ways of producing a hydroxyethyl starch derivative are described in the art.

DE 26 16 086 discloses the conjugation of hemoglobin to hydroxyethyl starch wherein, in a first step, a cross-linking agent, e.g. bromocyane, is bound to hydroxyethyl starch and subsequently hemoglobin is linked to the intermediate product.

One important field in which HES is used is the stabilisation of polypeptides which are applied, e.g., to the circulatory system in order to obtain a particular physiological effect. One specific example of these polypeptides is erythropoietin, an acid glycoprotein of approximately 34,000 kD which is essential in regulating the level of red blood cells in the circulation.

A well-known problem with the application of polypeptides and enzymes is that these proteins often exhibit an unsatisfactory stability. Especially erythropoietin has a relatively short plasma half live (Spivak and Hogans, 1989, Blood 73, 90; McMahon et al., 1990, Blood 76, 1718). This means that therapeutic plasma levels are rapidly lost and repeated intravenous administrations must be carried out. Furthermore, in certain circumstances an immune response against the peptides is observed.

It is generally accepted that the stability of polypeptides can be improved and the immune response against these polypeptides is reduced when the polypeptides are coupled to polymeric molecules. WO 94/28024 discloses that physiologically active polypeptides modified with polyethyleneglycol (PEG) exhibit reduced immunogenicity and antigenicity and circulate in the bloodstream considerably longer than unconjugated proteins, i.e. have a longer clearance rate. However, PEG-drug conjugates exhibit several disadvantages, e.g. they do not exhibit a natural structure which can be recognized by elements of in vivo degradation pathways. Therefore, apart from PEG-conjugates, other conjugates and protein polymerates have been produced. A plurality of methods for the cross-linking of different proteins and macromolecules such as polymerase have been described in the literature (see e.g. Wong, Chemistry of protein conjugation and cross-linking, 1993, CRCS, Inc.).

In summary, there is still a need for further improved polypeptides with improved stability and/or bioactivity. This applies especially to erythropoietin where isoforms with a high degree of sialic acids and therefore high actvity have to be purified from isoforms with a low degree of sialic acids (see EP 0 428 267 B1). Therefore, it would be highly advantageous if production methods were available which provide highly active polypeptides without requiring extensive purification. Unfortunately, the production of polypeptides in bacteria or insect cells is often difficult, because the polypeptides are often not produced in a properly folded, native confirmation and lack proper glycosylation.

WO 02/08079 A2 discloses compounds comprising a conjugate of an active agent and a hydroxyalkyl starch wherein active agent and hydroxyalykl starch are either linked directly or via a linker compound. As far as the direct linkage is concerned, the reaction of active agent and hydroxyalkyl starch is carried out in an aqueous medium which comprises at least 10 wt.-% of water. No examples are given which are directed to a hydroxyalkyl starch derivative which is linked to a carbonyl group comprised in the active reagent, neither an aldehyd or keto group nor a an acetal or a hemiacetal group.

Consequently, it is an object of the present invention to provide hydroxyalkyl starch derivatives which are capable of forming a chemical linkage to a further compound, e.g. a polypeptide, which comprises, as functional group, a thio group or an aldehyd group, a keto group, a hemiacetal group, or an acetal group. Preferably, the aldehyd group, the keto group, the hemiacetal group, or the acetal group are comprised in a carbohydrate moiety of the further compound.

Therefore, the present invention relates to a method of producing a hydroxyalkyl starch derivative, said hydroxyalkyl starch having a structure according to formula (I) 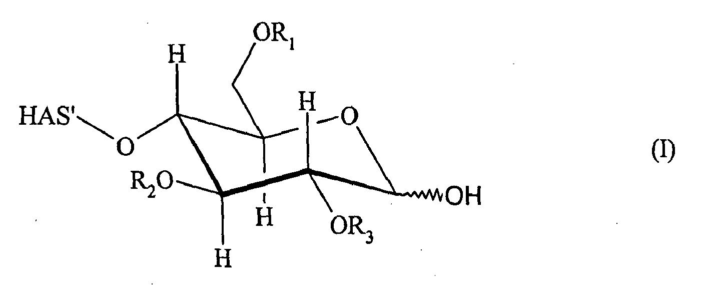 comprising reacting
- hydroxyalkyl starch of formula (I) at its optionally oxidized reducing end or
- a hydroxyalkyl starch derivative, obtainable by reacting hydroxyalkyl starch of formula (I) at its optionally oxidized reducing end with a compound (D), said compound (D) comprising
   -- at least one functional group Z₁ capable of being reacted with the optionally oxidized reducing end of the hydroxyalkyl starch, and
   -- at least one functional group W,
   with a compound (L) comprising
- at least one functional group Z₁ capable of being reacted with said hydroxyalkyl starch, or at least one functional group Z₂ capable of being reacted with functional group W comprised in said hydroxyalkyl starch derivative, and
- at least one functional group X capable of being reacted with a functional group Y of a further compound (M),
wherein said functional group Y is selected from the group consisting of an aldehyd group, a keto group, a hemiacetal group, an acetal group, or a thio group.

In the context of the present invention, the term "hydroxyalkyl starch" (HAS) refers to a starch derivative which has been substituted by at least one hydroxyalkyl group. Therefore, the term hydroxyalkyl starch as used in the present invention is not limited to compounds where the terminal carbohydrate moiety comprises hydroxyalkyl groups R₁, R₂, and/or R₃ as depicted, for the sake of brevity, in formula (1), but also refers to compounds in which at least one hydroxy group present anywhere, either in the terminal carbohydrate moiety and/or in the remaining part of the starch molecule, HAS', is substituted by a hydroxyalkyl group R₁, R₂, or R₃.

In this context, the alkyl group may be a linear or branched alkyl group which may be suitably substituted. Preferably, the hydroxyalkyl group contains 1 to 10 carbon atoms, more preferably from 1 to 6 carbon atoms, more preferably from 1 to 4 carbon atoms, and even more preferably 2-4 carbon atoms. "Hydroxyalkyl starch" therefore preferably comprises hydroxyethyl starch, hydroxypropyl starch and hydroxybutyl starch, wherein hydroxyethyl starch and hydroxypropyl starch are particularly preferred.

Hydroxyalkyl starch comprising two or more different hydroxyalkyl groups is also comprised in the present invention..

The at least one hydroxyalkyl group comprised in HAS may contain two or more hydroxy groups. According to a preferred embodiment, the at least one hydroxyalkyl group comprised HAS contains one hydroxy group.

The expression "hydroxyalkyl starch" also includes derivatives wherein the alkyl group is mono- or polysubstituted. In this context, it is preferred that the alkyl group is substituted with a halogen, especially fluorine, or with an aryl group, provided that the HAS remains soluble in water. Furthermore, the terminal hydroxy group a of hydroxyalkyl group may be esterified or etherified.

Furthermore, instead of alkyl, also linear or branched substituted or unsubstituted alkene groups may be used.

Hydroxyalkyl starch is an ether derivative of starch. Besides of said ether derivatives, also other starch derivatives can be used in the context of the present invention. For example, derivatives are useful which comprise esterified hydroxy groups. These derivatives may be, e.g., derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially derivatives of acetic acid. In this context, acetyl starch, butyl starch and propyl starch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

Hydroxyethyl starch (HES) is most preferred for all embodiments of the present invention.

Therefore, the present invention also relates to a method as described above wherein the hydroxyalkyl starch is hydroxyethyl starch.

HES is mainly characterized by the molecular weight distribution and the degree of substitution. There are two possibilities of describing the substitution degree:
1. The substitution degree can be described relatively to the portion of substituted glucose monomers with respect to all glucose moieties (DS).
2. The substitution degree can be described as the "molar substitution" (MS), wherein the number of hydroxyethyl groups per glucose moiety are described.

HES solutions are present as polydisperse compositions, wherein each molecule differs from the other with respect to the polymerisation degree, the number and pattern of branching sites, and the substitution pattern. HES is therefore a mixture of compounds with different molecular weight. Consequently, a particular HES solution is determined by average molecular weight with the help of statistical means. In this context, Mₙ is calculated as the arithmetic mean depending on the number of molecules. Alternatively, M_{w}, the weight mean, represents a unit which depends on the mass of the HES.

In the context of the present invention, hydroxyethyl starch may have a mean molecular weight (weight mean) of from 1 to 300 kDa, wherein a mean molecular weight of from 5 to 100 kDa is more preferred. Hydroxyethyl starch can further exhibit a molar degree of substitution of from 0.1 to 0.8 and a ratio between C₂ : C₆ substitution in the range of from 2 to 20 with respect to the hydroxyethyl groups.

As far as the residues R₁, R₂ and R₃ according to formula (I) are concerned there are no specific limitations given that compound (I) remains capable of being reacted with a compound (D) or a compound (L). According to a preferred embodiment, R₁, R₂ and R₃ are independently hydrogen or a hydroxyalkyl group, a hydroxyaryl group, a hydroxyaralkly group or a hydroxyalkarly group having of from 1 to 10 carbon atoms. Hydrogen and hydroxyalkyl groups having of from 1 to 6 carbon atoms are preferred. The alkyl, aryl, aralkyl and/or alkaryl group may be linear or branched and suitably substituted.

Therefore, the present invention also related to a method as described above wherein R₁, R₂ and R₃ are independently hydrogen or a linear or branched hydroxyalkyl group with from 1 to 6 carbon atoms.

Thus, R₁, R₂ and R₃ may be hydroxyhexyl, hydroxypentyl, hydroxybutyl, hydroxypropyl such as 1-hydroxypropyl, 2-hydroxypropyl, 3-hydroxypropyl, 1-hydroxyisopropyl, 2-hydroxyisopropyl, hydroxyethyl such as 1-hydroxyethyl, 2-hydroxyethyl, or hydroxymethyl. Hydrogen and hydroxyethyl groups are preferred, hydrogen and the 2-hydroxyethyl group being especially preferred.

Therefore, the present invention also relates to a method as described above wherein R₁, R₂ and R₃ are independently hydrogen or a 2-hydroxyethyl group.

According to the present invention either compound (D) or compound (L) is reacted with the reducing end of the hydroxyalkyl starch via the reaction of the functional group Z₁ with the reducing end where group Z₁ is comprised in compound (D) or compound (L).

According to a first preferred embodiment of the present invention, compound (D) or compound (L) is reacted with the reducing end of the hydroxyalkyl starch and where the reducing end is oxidized prior to the reaction.

This oxidation of the reducing end leads to hydroxyalkyl starch in which the terminal carbohydrate group comprises a lactone group, or in which the terminal carbohydrate group, depending of the chemical reaction conditions and/or the oxidizing agents, has a non-cyclic structure comprising a carboxy group.

According to one embodiment of the present invention, the hydroxyalkyl starch which is oxidized at its reducing end is present as a mixture of a compound comprising the lactone group and a compound comprising the carboxy group. In the mixture, the respective compounds may be present at any conceivable ratio.

Therefore, the present invention also relates to a method as described above wherein he reducing end of the hydroxyalkyl starch is oxidized prior to the reaction with compound (D) or compound (L), said hydroxyalkyl starch thus having a structure according to formula (IIa) 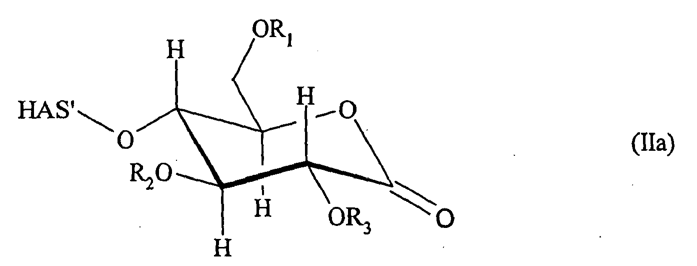 and/or according to formula (IIb)

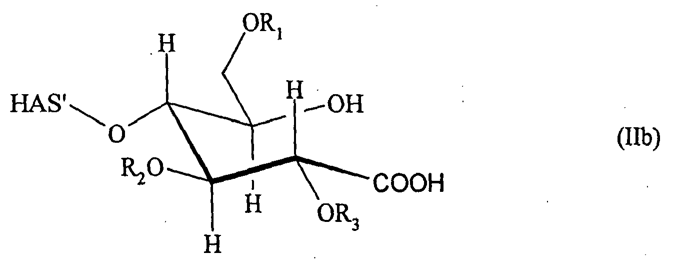

The oxidation of the reducing end of the hydroxyalkyl starch may be carried out according to each method or combination of methods which result compounds having the above-mentioned structures (IIa) and/or (IIb).

Although the oxidation may be carried out according to all suitable method or methods resulting in the oxidized reducing end of hydroxyalkyl starch, it is preferably carried out using an alkaline iodine solution as described, e.g., in 196 28 705 A1.

Therefore, the present invention also relates to a method as mentioned above wherein the reducing end is oxidized by an alkaline iodine solution.

According to a second preferred embodiment of the present invention, compound (D) or compound (L) is reacted with the reducing end of the hydroxyalkyl starch and where the reducing end is not oxidized prior to the reaction.

Therefore, the present invention also relates to a method as mentioned above wherein the reducing end of the hydroxyalkyl starch is not oxidized prior to the reaction with compound (D) or compound (L), said hydroxyalkyl starch thus having a structure according to formula (I)

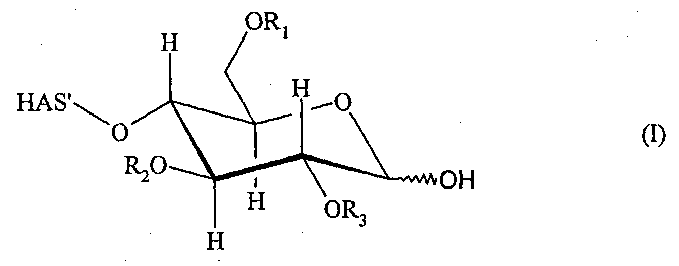

The formation of a chemical linkage between either compound (L) and hydroxyalkyl starch or compound (D) and hydroxyalkyl starch is achieved by reaction of the functional group Z₁ with the optionally oxidized reducing end of the hydroxyalkyl starch.

As functional group Z₁, each functional group may be used which is capable of forming a chemical linkage with the optionally oxidized reducing end of the hydroxyalkyl starch.

According to a preferred embodiment of the present invention, the functional group Z₁ comprises the chemical structure -NH-.

Therefore, the present invention also relates to a method as described above wherein the functional group Z₁ comprises the structure -NH-.

According to one preferred embodiment of the present invention, the functional group Z₁ is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

Therefore, the present invention also relates to a method as described above wherein R' is hydrogen or a methyl or a methoxy group.

According to another preferred embodiment of the present invention, the functional group Z₁ has the structure R'-NH-R"- where R"" preferably comprises the structure unit -NH- and/or the structure unit -(C=G)- where G is O or S, and/or the structure unit -SO₂-. According to more preferred embodiments, the functional group R" is selected from the group consisting of 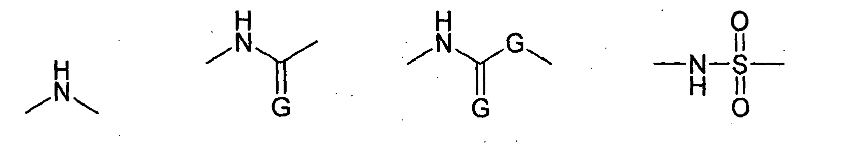 and 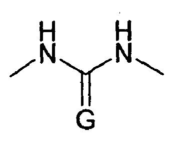 where, if G is present twice, it is independently O or S.

Therefore, the present invention also relates to a method as mentioned above wherein the fuctional group Z₁ is selected from the group consisting of 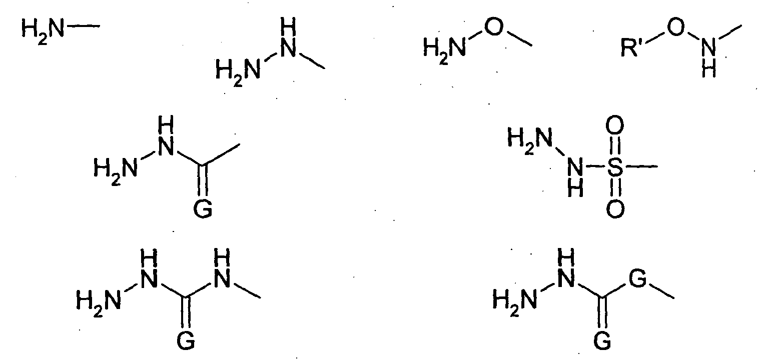 wherein G is O or S and, if present twice, independently O or S, and R' is methyl.

It is an object of the present invention to provide a hydroxyalkyl starch derivative which comprises a functional group X which is capable to react with the functional group Y of a further compound (M) to give as reaction product a hydroxyalkyl starch derivative which comprises the hydroxyalkyl starch, compound (L), optionally compound (D), and the further compound.

As to functional group X, there are no specific restrictions provided that a chemical linkage can be formed with the functional group Y which is comprised in the further compound (M).

If the functional group Y is selected from the group consisting of an aldehyd group, a keto group, a hemiacetal group, and an acetal group, the functional group X, the functional group X preferably comprises the chemical structure -NH-.

Therefore, the present invention also relates to a method as described above wherein the functional group Y is selected from the group consisting of an aldehyd group, a keto group, a hemiacetal group, and an acetal group, and the functional group X comprises the structure -NH-.

According to one preferred embodiment of the present invention, the functional group X is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

Therefore, the present invention also relates to a method as described above wherein R' is hydrogen or a methyl or a methoxy group.

According to another preferred embodiment of the present invention, the functional group X has the structure R'-NH-R"- where R"" preferably comprises the structure unit -NH- and/or the structure unit -(C=G)- where G is O or S, and/or the structure unit -SO₂-. According to more preferred embodiments, the functional group R" is selected from the group consisting of 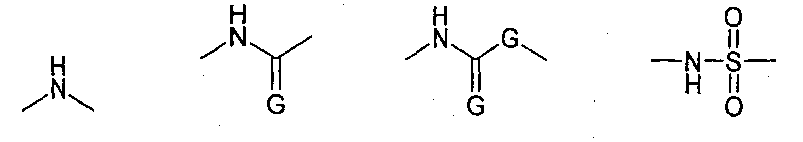 and 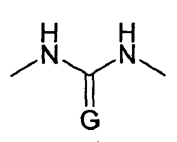 where, if G is present twice, it is independently O or S.

Therefore, the present invention also relates to a method as mentioned above wherein the fuctional group X is selected from the group consisting of 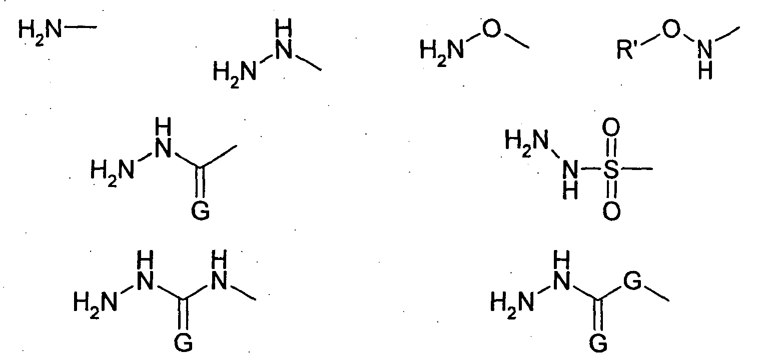 wherein G is O or S and, if present twice, independently O or S, and R' is methyl.

If the functional group Y is a thio group, the functional group X is preferably selected from the groups consisting of 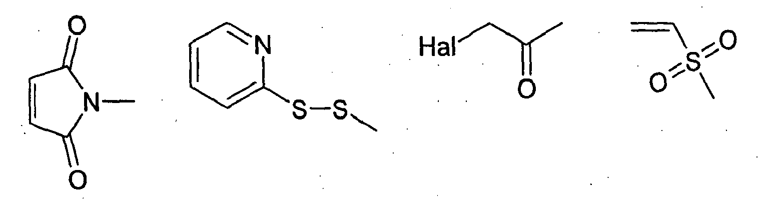 wherein Hal is Cl, Br or I, preferably Br or I.

Therefore, the present invention also relates to a method as described above whereinwherein the functional group Y is -SH and the functional group X is selected from the group consisting of 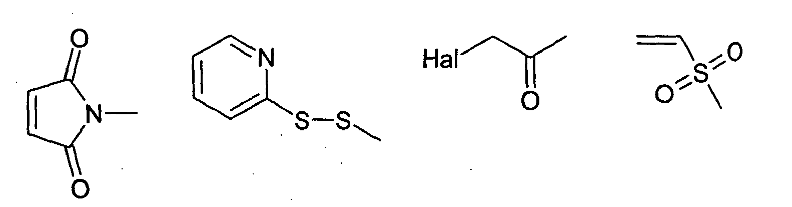 wherein Hal is Cl, Br or I.

According to one embodiment of the present invention, hydroxyalkyl starch is reacted with a compound (D) and the resulting reaction product is further reacted with compound (L) where the chemical linkage between compound (L) and the reaction product is formed by reaction of functional group Z₂ comprised in compound (L) and functional group W comprised in compound (D) being part of the reaction product.

Regarding the functional groups Z₂ and W, there are generally no restrictions provided that the desired chemical linkage is formed.

As possible functional groups W or Z₂, the following functional groups are to be mentioned, among others:
- C-C-double bonds or C-C-triple bonds or aromatic C-C-bonds;
- the thio group or the hydroxy groups;
- alkyl sulfonic acid hydrazide, aryl sulfonic acid hydrazide;
- 1,2-dioles;
- 1,2-aminoalcohols;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trochlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C≡C-H;
- -(C=NH₂Cl)-OAlkyl
- groups -(C=O)-CH₂-Hal wherein Hal is Cl, Br, or I;
- -CH=CH-SO₂
- a disulfide group comprising the structure -S-S-;
- the group 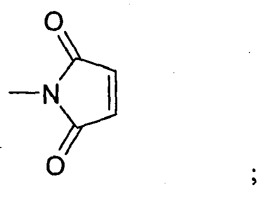
- the group 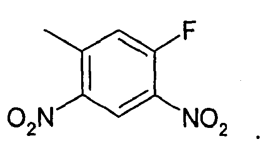
where Z₂ and W, respectively, is a group capable of forming a chemical linkage with one of the above-mentioned groups.

According to preferred embodiments of the present invention, both W and Z₂ are groups from the list of groups given above.

According to a first especially preferred embodiment of the present invention, Z₂ or W is a thio group. In this particular case, the functional group W is preferably selected from the group consisting of 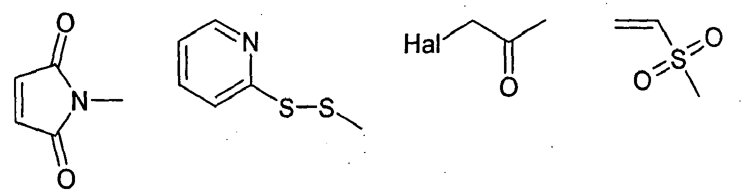 wherein Hal is Cl, Br, or I, preferably Br or I.

Therefore, the present invention also relates to a method as described above wherein the functional group W or the functional group Z₂ is -SH and the functional group Z₂ or the functional group W is selected from the group consisting of 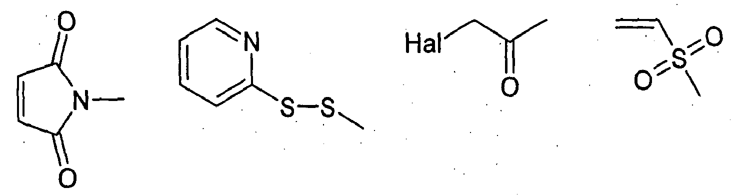 wherein Hal is Cl, Br, or I.

According to a second especially preferred embodiment of the present invention, Z₂ or W is selected from the group consisting of an activated ester, as described above, or a carboxy group which is optionally transformed into an activated ester. In this particular case, the functional group W or Z₂, respectively, comprises the chemical structure -NH-.

Therefore, the present invention also relates to a method as described above wherein Z₂ or W is selected from the group consisting of an activated ester, as described above, or a carboxy group which is optionally transformed into an activated ester, and the functional group W or Z₂, respectively, comprises the chemical structure - NH-.

According to one preferred embodiment of the present invention, the functional group W or Z₂ comprising the structure -NH- is a group having the structure R'-NH-where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

Therefore, the present invention also relates to a method as described above wherein W or Z₂ is selected from the group consisting of an activated ester, as described above, or a carboxy group which is optionally transformed into an activated ester, and the functional group W or Z₂, respectively, is R'-NH- wherein R' is hydrogen or a methyl or a methoxy group.

According to another preferred embodiment of the present invention, the functional group W or Z₂ has the structure R'-NH-R"- where R" preferably comprises the structure unit -NH- and/or the structure unit -(C=G)- where G is O or S, and/or the structure unit -SO₂-. According to more preferred embodiments, the functional group R" is selected from the group consisting of 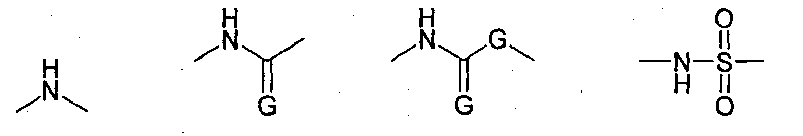 and 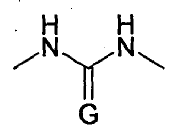 where, if G is present twice, it is independently O or S.

Therefore, the present invention also relates to a method as mentioned above wherein the functional group W or Z₂ is selected from the group consisting of 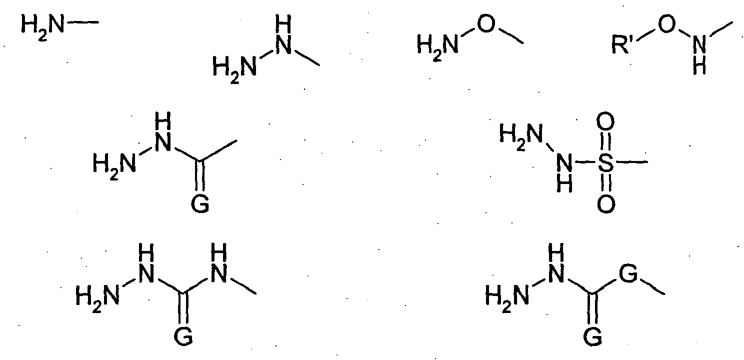 wherein G is O or S and, if present twice, independently O or S, and R' is methyl.

According to yet another aspect of the present invention, the at least one functional group X, Z₂ and/or W may be a group which is not capable of reacting directly with a given further compound, but which may be chemically modified in order to be capable of reacting in the desired way.

As an example of a functional group to be modified prior to the reaction with a further compound, a 1,2-amino alcohol or a 1,2-diol may be mentioned which is modified, e.g., by oxidation to form an aldehyd or a keto group.

Another example for a functional group to be modified prior to the reaction with a further compound is a -NH₂ group which is modified by the reaction with, e.g., a compound according to the following formula 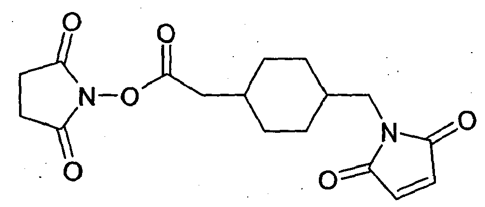 to give a structure of the following formula 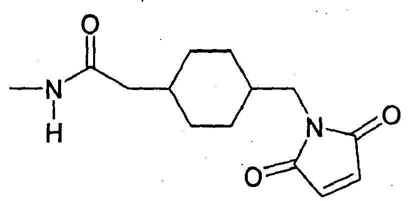 which is, e.g., reactive towards a thio group.

Another example for a functional group to be modified prior to the reaction with a further compound is a -NH₂ group which is modified by the reaction with, e.g., a compound according to the following formula 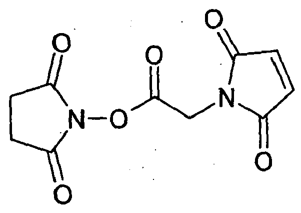 to give a structure of the following formula 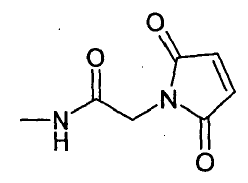 which is, e.g., reactive towards a thio group.

Yet another example for a functional group to be modified prior to the reaction with a further compound is an amino group which is reacted with bromoacetic anhydride or N-succinimidyl iodo acetate.

According to a preferred embodiment of the present invention, a compound (L) has the structure Z₁-L'-X or Z₂-L'-X, L' being an organic residue separating the functional groups and being optionally absent, the structure depending on whether a compound (D) is reacted with the hydroxyalkyl starch or not.

According to a first preferred embodiment, no compound (D) is involved and Y is selected from the group consisting of an aldehyd group, a keto group, a hemiacetal group, and an acetal group.

In this particular case, the following compounds, among others, are preferred as compound (L) having the structure Z₁-L'-X where L' is absent:

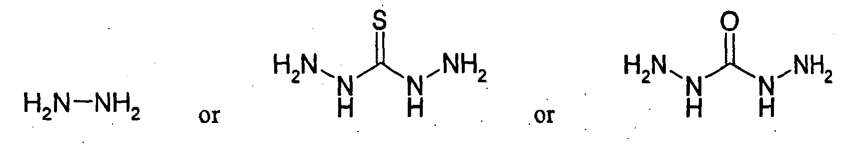

If, in this particular case, L' is not absent, L' may be a linear or branched alkyl or cycloalkyl or aryl or or aralkyl or arylcycloalkyl or alkaryl or cycloalkylaryl group, wherein L' may comprise at least one heteroatom such as N, O, S, and wherein L' may be suitably substituted. The size of the group L' may be adapted to the specific needs. Generally, the separating group L' generally has from 1 to 60, preferably from 1 to 40, more preferably from 1 to 20, more preferably from 1 to 10, more preferably from 1 to 6 and especially preferably from 1 to 4 carbon atoms. If heteroatoms are present, the separating group comprises generally from 1 to 20, preferably from 1 to 8 and especially preferably from 1 to 4 heteroatoms. According to particularly preferred embodiments of the present invention, the separating group L' comprises 1 to 4 oxygen atoms. The separating group L' may comprise an optionally branched alkyl chain or an aryl group or a cycloalkyl group having, e.g., from 5 to 7 carbon atoms, or be a aralkyl group, an alkaryl group where the alkyl part may be a linear and/or cyclic alkyl group. According to an even more preferred embodiment, the separating group is an alkyl chain of from 1 to 20, preferably from 1 to 8, more preferably from 1 to 6, more preferably from 1 to 4 and especially preferably from 2 to 4 carbon atoms. In case heteroatoms are present, a chain comprising 1 to 4 oxygen atoms is particularly preferred.

As to this particular case where Y is selected from the group consisting of an aldehyd group, a keto group, a hemiacetal group, and an acetal group, the following compounds, among others, are preferred as compound (L) having the structure Z₁-L'-X where L' is not absent:

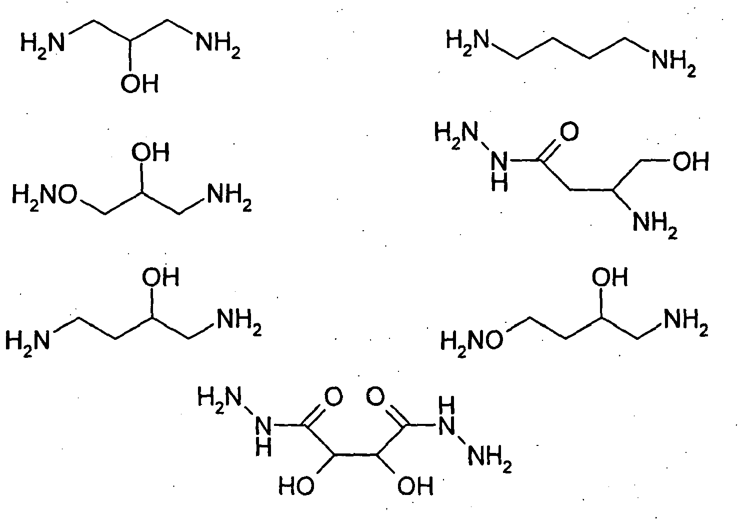

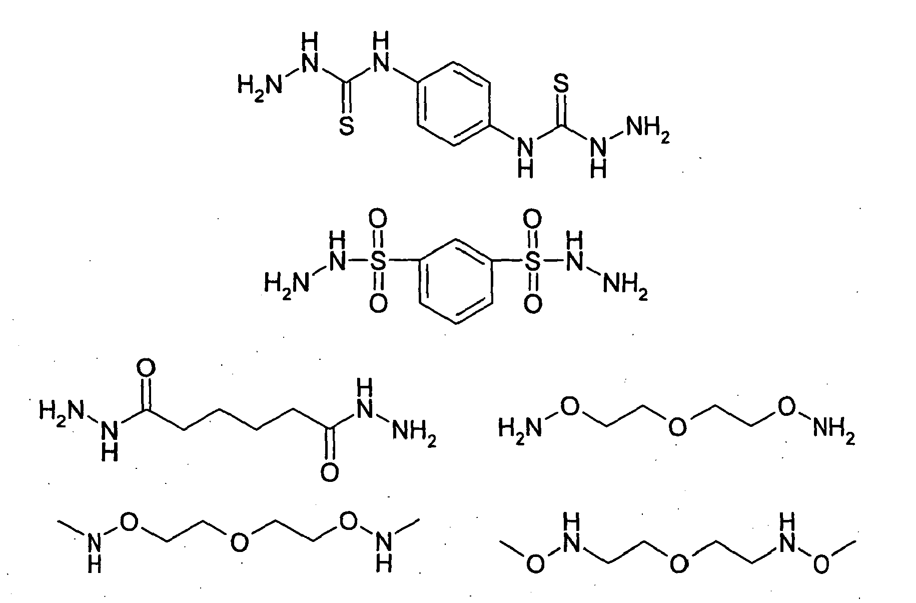

According to a second preferred embodiment, a compound (D) is involved.

According to a further preferred embodiment of the present invention, a compound (D) has the structure Z₁-D'-W, D' being an organic residue separating the functional groups and being optionally absent.

In this particular case, the following compounds, among others, are preferred as compound (D) having the structure Z₁-D'-W where D' is absent:

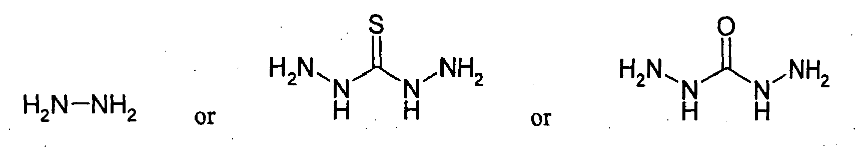

A specific example of a compound D where D' is absent is NH₃.

If, in this particular case, D' is not absent, D' may be a linear or branched alkyl or cycloalkyl or aryl or or aralkyl or arylcycloalkyl or alkaryl or cycloalkylaryl group, wherein D' may comprise at least one heteroatom such as N, O, S, and wherein D' may be suitably substituted. The size of the group D' may be adapted to the specific needs. Generally, the separating group D' generally has from 1 to 60, preferably from 1 to 40, more preferably from 1 to 20, more preferably from 1 to 10, more preferably from 1 to 6 and especially preferably from 1 to 4 carbon atoms. If heteroatoms are present, the separating group comprises generally from 1 to 20, preferably from 1 to 8 and especially preferably from 1 to 4 heteroatoms. According to particularly preferred embodiments of the present invention, the separating group D' comprises 1 to 4 oxygen atoms. The separating group D' may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the separating group is an alkyl chain of from 1 to 20, preferably from 1 to 8, more preferably from 1 to 6, more preferably from 1 to 4 and especially preferably from 2 to 4 carbon atoms. In case heteroatoms are present, a chain comprising 1 to 4 oxygen atoms is particularly preferred.

As to this particular case, preferred compounds (D) having the structure Z₁-D'-W where D' is not absent are:

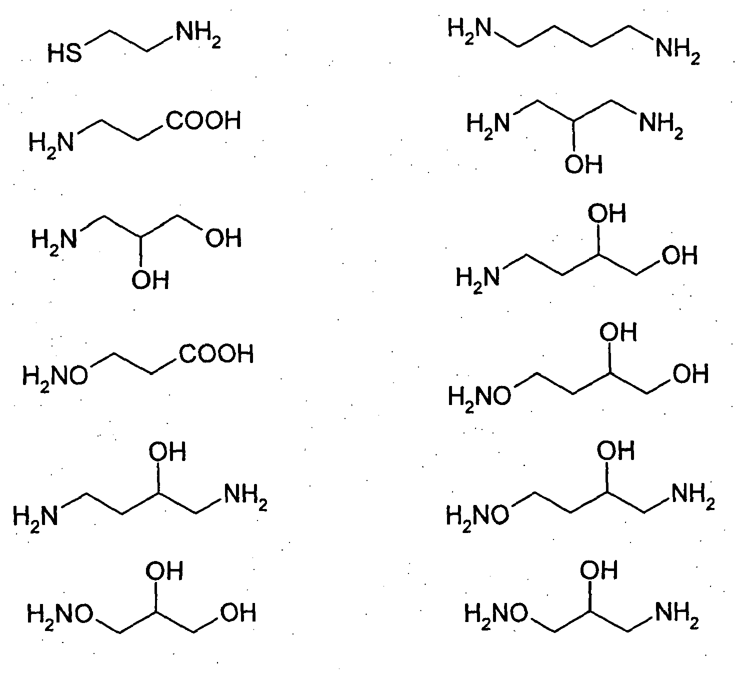

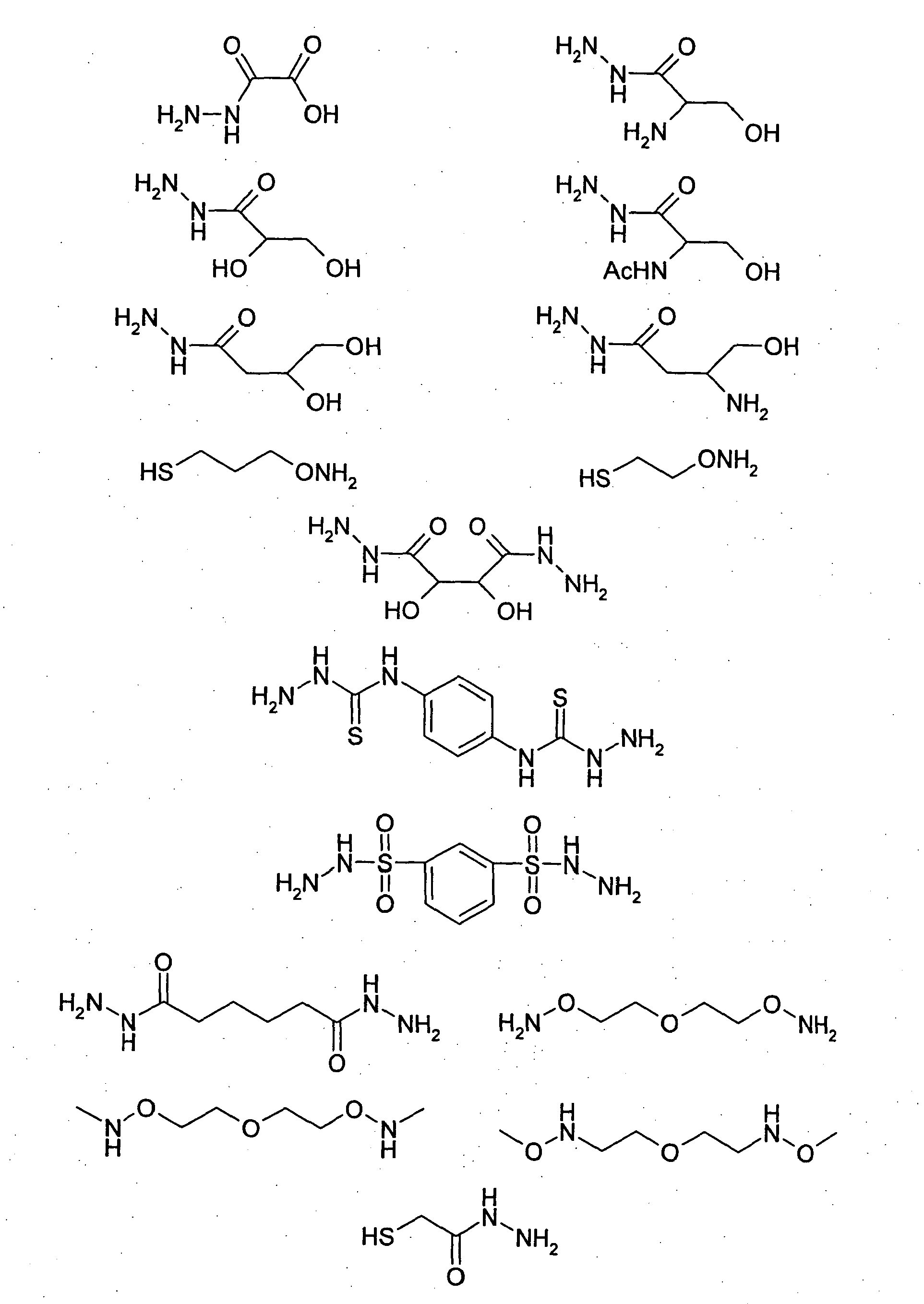

Depending on the chemical nature of the functional group W comprised in compound (D) and the functional group Y, specific compounds (L) may be used according to the specific needs.

If, e.g., the functional group Y is a thio group and the functional group W comprises the structure -NH-, as described above in detail, the following types of compounds (L) are, among others, preferred:

| **Type of compound (L)** | **Functional group X** | **Functional group Z₂** |
|---|---|---|
| C | Iodoalkyl | N-succinimide ester |
| D | Bromoalkyl | N-succinimide ester |
| E | Maleimido | N-succinimide ester |
| F | Pydridyldithio | N-succinimide ester |
| G | Vinylsulfone | N-succinimide ester |

If, e.g., the functional group Y is selected from the group consisting of an aldehyd group, a keto group, a hemiacetal group, and an acetal group, and the functional group W is a thio group, the following types of compounds (L) are, among others, preferred:

| **Type of compound (L)** | **Functional group X** | **Functional group Z₂** |
|---|---|---|
| A | Hydrazide | Maleimido |
| B | Hydrazide | Pyridyldithio |

In Table 1 at the end of the present description, some preferred examples of compounds (L) according to the types given above are listed.

The separating groups L' and/or D' may be suitably substituted. Preferred substituents are, e.g, halides such as F, Cl, Br or I.

The separating groups L' and/or D' may comprise one or more cleavage sites such as 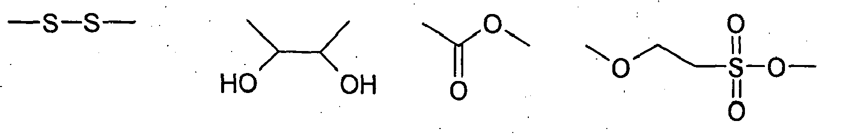 which allow for an easy cleavage of a resulting compound at a pre-determined site.

Especially preferred examples of compounds (L) which may be linked to hydroxyalkyl starch wherein the resulting hydroxyalkyl starch derivative comprises the functional group X capable of being reacted with a functional group Y comprised in a further compound (M) and wherein said functional group Y is selected from the group consisting of an aldehyd group, a keto group, a hemiacetal group, an acetal group, are 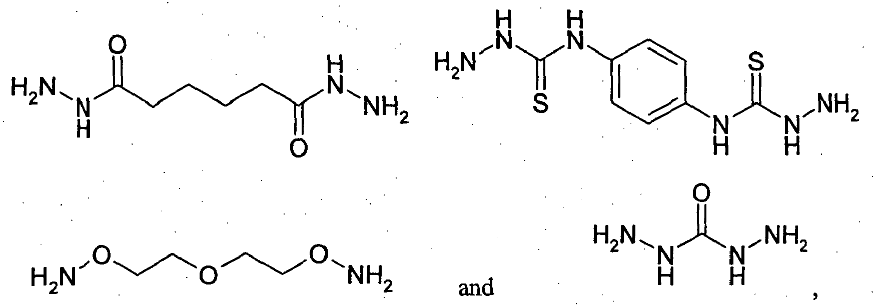 the compounds (L) 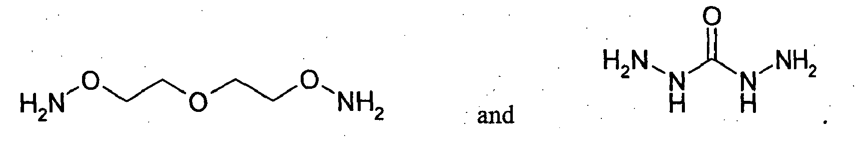 being particularly preferred.

Especially preferred examples of compounds (D) which may be linked to hydroxyalkyl starch wherein the resulting hydroxyalkyl starch derivative comprises the functional group W capable of being reacted with a functional group Z₂ comprised in a compound (L) wherein the resulting hydroxyalkyl starch derivative which comprises hydroxyalkyl starch, compound (D) and compound (L), is capable of being reacted with the functional group Y of a further compound (M) and wherein said functional group Y is a thio group, are 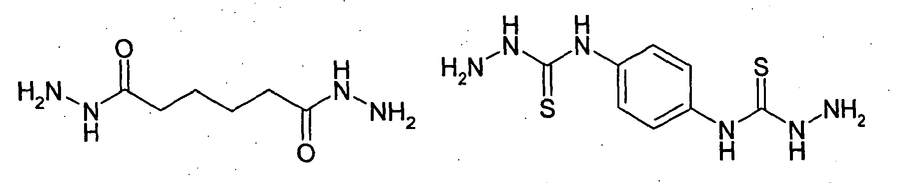 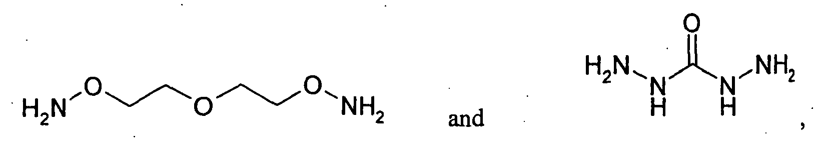 the compounds (D) 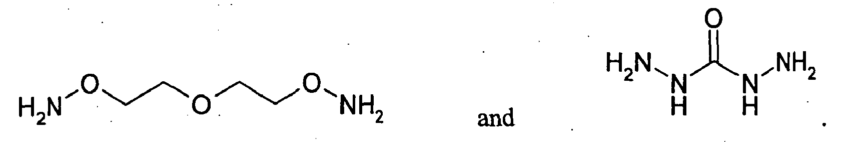 being particularly preferred.

Together with the above-mentioned preferred compounds (D), the following compounds (L) 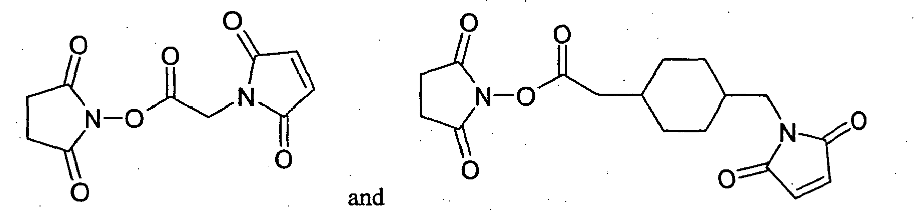 are preferred, the compound (L) 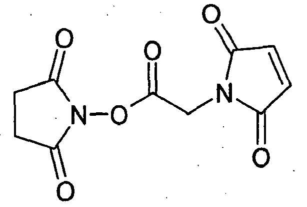 being especially preferred.

According to a first preferred embodiment of the present invention, a compound (D) or a compound (L) is reacted with the reducing end of the hydroxyalkyl starch which is not oxidised.

Depending on the reaction conditions such as the solvent or solvent mixture used, the temperature, pressure or pH of the reaction mixture, the reaction product of a compound (D) or a compound (L) is reacted with the reducing end of the hydroxyalkyl starch which is not oxidised may have different constitutions.

According to a preferred embodiment of the present invention, this reaction is carried out in an aqueous system.

The term "aqueous system" as used in the context of the present invention refers to a solvent or a a mixture of solvents comprising water in the range of from at least 10 % per weight, preferably at least 50 % per weight, more preferably at least 80 % per weight, even more preferably at least 90 % per weight or up to 100 % per weight, based on the weight of the solvents involved. As additional solvents, solvents such as DMSO, DMF, ethanol or methanol may be mentioned.

If the reaction is carried out in an aqueous system and the functional group Z₁ is a group R'-NH-, as described above, the hydroxyalkyl starch derivative may have a constitution according to formula (IIIa)

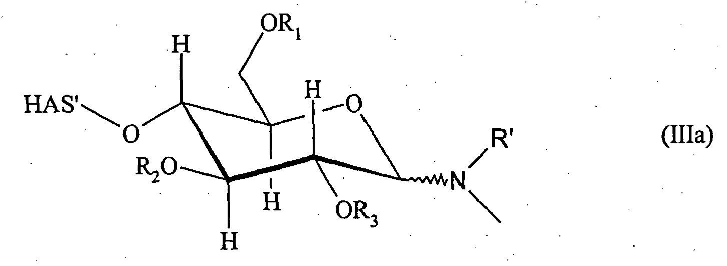

If the reaction is carried out in an aqueous system and the functional group Z₁ is a group R'-NH- with R' = H, as described above, the hydroxyalkyl starch derivative may have a constitution according to formula (IIIa) or formula (IIIb) or be a mixture of compounds according to formulae (IIIa) and (IIIb)

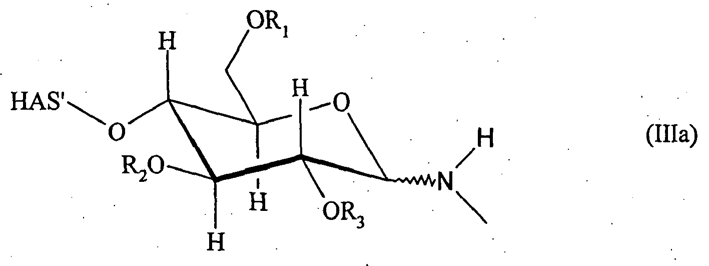

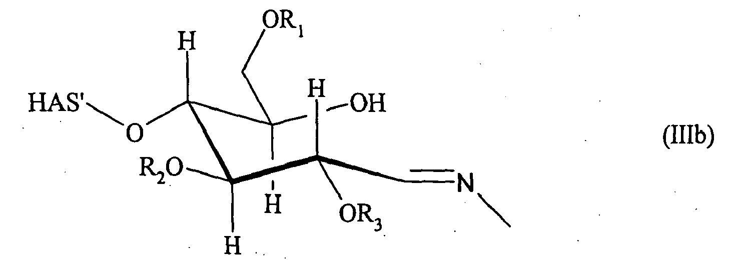

Depending on the reaction conditions and/or the chemical nature of compounds (L) or compound (D) used for the reaction, the compounds according to formula (IIIa) may be present with the N atom in equatorial or axial position where also a mixture of both forms may be present having a certain equilibrium distribution.

Depending on the reaction conditions and/or the chemical nature of compounds (L) or (D) used for the reaction, the compounds according to formula (IIIb) may be present with the C-N double bond in E or Z conformation where also a mixture of both forms may be present having a certain equilibrium distribution.

Therefore, the present invention also relates to a hydroxyalkyl starch derivative as described above having a constitution according to formula (IIIb) or according to formula (IIIb) or according to formulae (IIIa) and (IIIb).

In some cases it may be desirable to stabilize the compound according to formula (IIIa). This is especially the case where the compound according to formula (IIIa) is produced and/or used in an aqueous solution. As stabilizing method, acylation of the compound according to formula (IIIa) is particularly preferred, especially in the case where R' is hydrogen. As acylation reagent, all suitable reagents may be used which result in the desired hydroxyalkyl starch derivative according to formula (IVa)

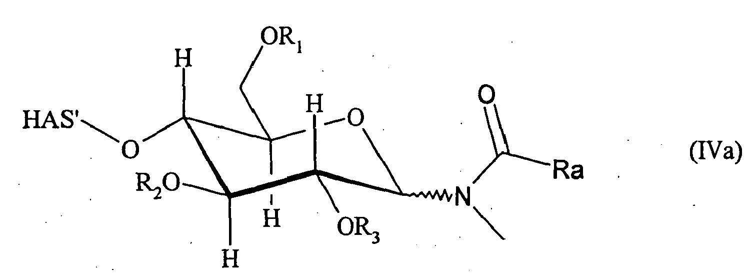

According to especially preferred embodiments of the present invention, the residue Ra being part of the acylation reagent is methyl. As acylation reagents, carboxylic acid anhydrides, carboxylic acid halides, and carboxylic acid active esters are pref erably used.

The acylation is carried at a temperature in the range of from 0 to 30 °C, preferably in the range of from 2 to 20 °C and especially preferably in the range of from 4 to 10 °C.

Therefore, the present invention also relates to a hydroxyalkyl starch derivate obtainable by a method as described above wherein said derivative has a constitution according to formula (IVa).

In other cases it may be desirable to stabilize the compound according to formula (IIIb). This is especially the case where the compound according to formula (IIIb) is produced and/or used in an aqueous solution. As stabilizing method, reduction of the compound according to formula (IIIb) is particularly preferred, especially in the case where R' is hydrogen. As reduction reagent, all suitable reagents may be used which result in the desired hydroxyalkyl starch derivative according to formula (IVb)

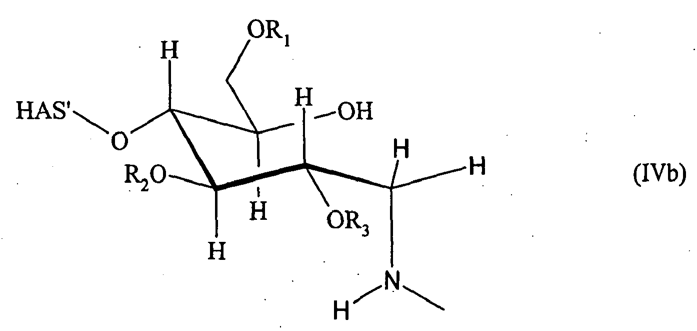

According to especially preferred embodiments of the present invention, as reduction reagents boro hydrides such as NaCNBH₃ or NaBH4 are used.

The reduction is carried at a temperature in the range of from 4 to 100 °C, preferably in the range of from 10 to 90 °C and especially preferably in the range of from 25 to 80 °C.

Therefore, the present invention also relates to a hydroxyalkyl starch derivate obtainable by a method as described above wherein said derivative has a constitution according to formula (IVb).

The present invention further relates to mixtures of compounds having constitutions according to formulae (IIIa) and (IIIb), (IVa) and (IVb), (IIIa) and (IVa), (IIIa) and (IVb), (IIIb) and (IVa), (IIIb) and (IVb), (IIIa) and (IIIb) and (IVa), (IIIa) and (IIIb) and (IVb), (IVa) and (IVb) and (IIIa), and (IVa) and (IVb) and (IIIb) wherein (IIIa) and/or (IVa) may be independently present in a conformation where the N atom in equatorial or axial position and/or wherein (IIIb) may be present with the C-N double bond in E or Z conformation.

According to a second preferred embodiment of the present invention, a compound (D) or a compound (L) is reacted with the reducing end of the hydroxyalkyl starch which is oxidised.

In this case, preferably polar aprotic solvents are used which may also contain a certain amount of water, such as up to 10 wt.-%. Preferred aprotic solvents are, among others, DMSO or DMF. An example of a preferred reaction temperature range is from room 20 to 65 °C, and the reaction times are generally in the range of 1 minute to several hours and up to several days, depending on the chemical nature of the functional group which is reacted with the oxidized reducing end og the hydroxyalkyl starch and the other reaction conditions.

If, in this case, the functional group Z₁ is a group R'-NH-, as described above, the hydroxyalkyl starch derivative may have a constitution according to formula (Va)

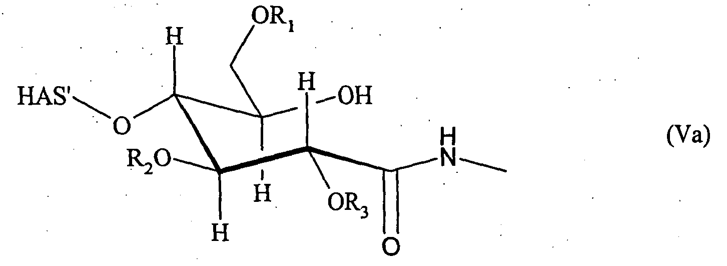

Therefore, the present invention also relates to a hydroxyalkyl starch derivate obtainable by a method as described above wherein said derivative has a constitution according to formula (Va).

As far as the reactions of hydroxyalkyl starch with compound (D) and/or compound (L) as well as compound (M) are concerned, all possible sequences are comprised by the present invention.

A preferred embodiment of the present invention relates to a method as described above wherein hydroxyalkyl starch is reacted with a compound (L) via the reaction of functional group Z₁ with the optionally oxidized reducing end of the hydroxyalkyl starch and the resulting reaction product is reacted with a further compound (M) via the reaction of the functional group X comprised in compound (L) with the functional group Y comprised in compound (M).

Another embodiment of the present invention relates to a method as described above wherein hydroxyalkyl starch is reacted with a compound (L) via the reaction of functional group Z₁ with the optionally oxidized reducing end of the hydroxyalkyl starch, where compound (L), prior to the reaction with hydroxyalkyl starch, is reacted with a further compound (M) via the reaction of functional group X comprised in compound (L) with the functional group Y comprised in compound (M).

Still another embodiment of the present invention relates to a method as described above wherein hydroxyalkyl starch is reacted with a compound (D) via the reaction of the functional group Z₁ comprised in compound (D), with the optionally oxidized reducing end of the hydroxyalkyl starch to give a first hydroxyalkyl starch derivative, and where the first hydroxyalkyl starch derivative is reacted with a compound (L) via the reaction of functional group Z₂ comprised in compound (L) with the functional group W comprised in compound (D) to give a second hydroxyalkyl starch derivative.

Yet another embodiment of the present invention relates to the latter method wherein the second hydroxyalkyl starch derivative is reacted with a further compound (M) via the reaction of functional group X comprised in compound (L) with the functional group Y comprised in compound (M).

Still yet another embodiment of the present invention relates to a method as described above wherein hydroxyalkyl starch is reacted with a compound (D) via the reaction of functional group Z₁ comprised in compound (D) with the optionally oxidized reducing end of the hydroxyalkyl starch to give a first hydroxyalkyl starch derivative, and where the first hydroxyalkyl starch derivative is reacted, via the reaction of the functional group W, comprised in compound (D), and the functional group Z₂, comprised in compound (L), with compound (L), where compound (L), prior to the reaction with the first hydroxyalkyl starch derivative, is reacted with a further compound (M) via the reaction of functional group X comprised in compound (L) with the functional group Y comprised in compound (M).

As far as the reaction conditions of each of the above-described reactions steps are concerned, all parameters such as temperature, pressure, pH, or solvent or solvent mixture may be adapted to the specific needs and the chemical nature of compounds to be reacted.

According to an especially preferred embodiment of the present invention, water is used as solvent, either alone or in combination with at least one other solvent. As at least one other solvent, DMSO, DMF, methanol and ethanol may be mentioned. Preferred solvents other than water are DMSO, DMF, methanol and ethanol. In this embodiment, hydroxylalkyl starch is preferably reacted via the non-oxidized reducing end.

If hydroxyalkyl starch is reacted with compound (D) or compound (L) in an aqueous medium and compound (D) or compound (L) is a hydroxylamine or a hydrazide, the temperature of the reaction is preferably in the range of from 5 to 45 °C, more preferably in the range of from 10 to 30 °C and especially preferably in the range of from 15 to 25 °C.

If hydroxyalkyl starch is reacted with compound (D) or compound (L) in an aqueous medium and the reaction being a reductive amination, the temperature is preferably in the range of up to 100 °C, more preferably in the range of from 70 to 90 °C and especially preferably in the range of from 75 to 85 °C.

During the course of the reaction the temperature may be varied, preferably in the above-given ranges, or held essentially constant.

The reaction time for the reaction of hydroxyalkyl starch with compound (D) or compound (L) may be adapted to the specific needs and is generally in the range of from 1 h to 7 d.

In case compound (D) or compound (L) is a hydroxylamine or a hydrazide, the reaction time is preferably in the range of from 1 h to 3 d and more preferably of from 2 h to 48 h.

In case the reaction of hydroxyalkyl starch with compound (D) or compound (L) is a reductive amination, the reaction time is preferably in the range of from 2 h to 7 d.

The pH value for the reaction of hydroxyalkyl starch with compound (D) or compound (L) may be adapted to the specific needs such as the chemical nature of the reactants.

In case compound (D) or compound (L) is a hydroxylamine or a hydrazide, the pH value is preferably in the range of from 4.5 to 6.5.

In case the reaction of hydroxyalkyl starch with compound (D) or compound (L) is a reductive amination, the pH value is preferably in the range of from 8 to 12.

The suitable pH value of the reaction mixture may be adjusted, for each reaction step, by adding at least one suitable buffer. Among the preferred buffers, sodium acetate buffer, phosphate or borate buffers may be mentioned.

If necessary, the at least one functional group X may be protected with at least one suitable protecting group prior to the reaction of hydroxyalkyl starch with compound (L) or prior to the reaction of compound (D) with compound (L) or prior to the reaction of compound (L) with the reaction product of the reaction of hydroxyalkyl starch with compound (D). In this respect, all conceivable protecting groups are possible which prevent the protected compound (L) from reacting via the at least one functional group X. Hence, the protecting group may be chosen depending from the chemical nature of the functional group X to be protected, from, e.g., the solvent the reaction is carried out in or the pH of the reaction mixture. Preferred protecting groups are, among others, the benzyloxycarbonyl group, the tert-butoxycarbonyl group, the methoxyphenyl group, the 2,4-dimethoxyphenyl group, triarly methyl groups, trityl, the monomethoxytrityl group, the dimethoxytrityl group, the monomethyltrityl group, the dimethyltrityl group, the trifluoracetyl group, phthalimin compounds, 2-(trialkylsilyl)ethoxy carbonyl compounds, Fmoc, the tert-butyl group, or trialkyl silyl groups.

If two or more different functional groups X are present in compound (L), at least one group may be protected whereas at least one other group may be left unprotected.

After the reaction of compound (L), the at least one protecting group may be left in the reaction product or removed by suitable methods such as conventional methods known to the person skilled in the art. If two different functional groups X are protected by suitable protecting groups, it is possible to remove at least one protecting group so as to make at least one functional group X available for further reaction with at least one further compound (M), and leave at least one other functional group protected until the reaction product comprising compound (L) is reacted with the further compound (M). Afterwards, the protecting group of the functional group still protected may be removed to make the remaining functional group X available for reaction with yet a further compound (M).

The use of at least one protecting group may be important for preventing the reaction from resulting in a hydroxyalkyl starch derivative comprising a compound (L) or compound (D) which has been reacted with two or more hydroxyalkyl starch molecules, i.e. a multiple HAS substituted compound (L) or (D). The same result, however, may be achieved by reacting hydroxyalkyl starch with an excess of compound (L) or (D). If an excess amount of compound (L) or (D) is used in the process of the present invention, the molar ratio of compound (L) or (D) to hydroxyalkyl starch is preferably in the range of from 2 to 100.

Once the reaction product of the respective reaction step, as described above, is formed, it may be isolated from the reaction mixture by at least one suitable method. If necessary, the reaction product may be precipitated prior to the isolation by at least one suitable method.

If the reaction product is precipitated first, it is possible, e.g., to contact the reaction mixture with at least one solvent or solvent mixture other than the solvent or solvent mixture present in the reaction mixture at suitable temperatures. According to a particularly preferred embodiment of the present invention where an aqueous system is used as solvent, the reaction mixture is contacted with a mixture of ethanol and acetone, preferably a 1:1 mixture, indicating equal volumes of said compounds, at a temperature, preferably in the range of from -20 to +50 °C and especially preferably in the range of from 0 to 25°C..

Isolation of the reaction product may be carried out by a suitable process which may comprise one or more steps. According to a preferred embodiment of the present invention, the reaction product is first separated off the reaction mixture or the mixture of the reaction mixture with, e.g., the ethanol-acetone mixture, by a suitable method such as centrifugation or filtration. In a second step, the separated reaction product may be subjected to a further treatment such as an after-treatment like dialysis, centrifugal filtration or pressure filtration, ion exchange chromatography, HPLC, MPLC, gel filtration and/or lyophilisation. According to an even more preferred embodiment, the separated reaction product is first dialysed, preferably against water, and then lyophilized until the solvent content of the reaction product is sufficiently low according to the desired specifications of the product. Lyophilisation may be carried out at temperature of from 20 to 35 °C, preferably of from 25 to 30 °C.

According to preferred embodiments of the present invention, the hydroxyalkyl starch derivative comprising hydroxyalkyl starch and compound (L) or comprising hydroxyalkyl starch, compound (D) and compound (L) is further reacted with the further compound (M) which comprises at least one functional group Y.

Generally, there are no limitations regarding compound (M). Preferably, a polypeptide is used as compound (M) in the context of the present invention. However, other compounds (M) are also possible, either polymers or oligomers or monomolecular compounds or mixtures of two or more thereof.

The term "polypeptide" as used in the context of the present invention refers to a compound which comprises at least 2 amino acids which are linked via a peptide bond, i.e. a bond with structure -(C=O)-NH-. The polypeptide may be a naturally occuring compound or a polypeptide which does not occur naturally, the latter comprising naturally occuring amino acids and/or at least one amino acid which does not naturally occur. The backbone of the polypeptide, the polypeptide chain, may be further substituted with at least one suitable substituent thus having at least one side-chain. The at least one functional group Y may be part of the polypeptide backbone or of at least one substituent of the backbone wherein embodiments are possible comprising at least one functional group being part of the polypeptide backbone and at least one functional group being part of at least one substituent of the polypeptide backbone.

As far as the polypeptide is concerned, there exist no restrictions, given that the polypeptide comprises at least one functional group Y. Said functional group Y may be linked directly to the polypeptide backbone or be part of a side-chain of the backbone. Either side-chain or functional group Y or both may be part of a naturally occuring polypeptide or may be introduced into a naturally occuring polypeptide or into a polypeptide which, at least partially, does not occur naturally, prior to the reaction with the functional group X.

Moreover, the polypeptide can be, at least partly, of any human or animal source. In a preferred embodiment, the polypeptide is of human source.

The polypeptide may be a cytokine, especially erythropoietin, an antithrombin (AT) such as AT III, an interleukin, especially interleukin-2, IFN-beta, IFN-alpha, G-CSF, CSF, interleukin-6 and therapeutic antibodies.

According to a preferred embodiment, the polypeptide is an antithrombin (AT), preferably AT III (Levy JH, Weisinger A, Ziomek CA, Echelard Y, Recombinant Antithrombin: Production and Role in Cardiovascular Disorder, Seminars in Thrombosis and Hemostasis 27, 4 (2001) 405-416; Edmunds T, Van Patten SM, Pollock J, Hanson E, Bernasconi R, Higgins E, Manavalan P, Ziomek C, Meade H, McPherson J, Cole ES, Transgenically Produced Human Antithrombin: Structural and Functional Comparison to Human Plasma-Derived Antithrombin, Blood 91, 12 (1998) 4661-4671; Minnema MC, Chang ACK, Jansen PM, Lubbers YTP, Pratt BM, Whittaker BG, Taylor FB, Hack CE, Friedman B, Recombinant human antithrombin III improves survival and attenuates inflammatory responses in baboons lethally challenged with Escherichia coli, Blood 95, 4 (2000) 1117-1123; Van Patten SM, Hanson EH, Bernasconi R, Zhang K, Manavaln P, Cole ES, McPherson JM, Edmunds T, Oxidation of Methionine Residues in Antithrombin, J. Biol. Chemistry 274, 15 (1999) 10268-10276).

According to another preferred embodiment, the polypeptide is human IFN-beta, in particular IFN-beta 1a (cf. Avonex®, REBIF®) and IFN-beta 1b (cf. BETASERON®).

A further preferred polypeptide is human G-CSF (granulocyte colony stimulating factor). See, e.g., Nagata et al., The chromosomal gene structure and two mRNAs for human granulocyte colony-stimulating factor, EMBO J. 5: 575-581, 1986; Souza et al., Recombinant human granulocyte colony-stimulating factor: effects on normal and leukemic myeloid cells, Science 232 (1986) 61-65; and Herman et al., Characterization, formulation, and stability of Neupogen® (Filgrastim), a recombinant human granulocyte-colony stimulating factor, in: Formulalion, characterization, and stability of protein drugs, Rodney Pearlman and Y. John Wang, eds., Plenum Press, New York, 1996,303-328.

If a mixture of at least two different polypeptides is used, the at least two polypeptides may differ, e.g., in the molecular mass, the number and/or sequence of amino acids, the number and/or chemical nature of the substituents or the number of polypeptide chains linked by suitable chemical bonds such as disulfide bridges.

According to a preferred embodiment of the present invention, the reaction product of hydroxyalkyl starch and compound (L) or the reaction product of hydroxyalkyl starch and compound (D) which is further reacted with compound (L) is isolated, preferably according to at least one of the above-mentioned processes, and then reacted with a polypeptide having at least one functional group Y. According to a preferred embodiment of the present invention, the functional group Y is comprised in a carbohydrate moiety of the polypeptide.

In the context of the present invention, the term "carbohydrate moiety" refers to hydroxyaldehydes or hydroxyketones as well as to chemical modifications thereof (see Römpp Chemielexikon, Thieme Verlag Stuttgart, Germany, 9th edition 1990, Volume 9, pages 2281-2285 and the literature cited therein). Furthermore, it also refers to derivatives of naturally occuring carbohydrate moieties like glucose, galactose, mannose, sialic acid and the like. The term also includes chemically oxidized, naturally occuring carbohydrate moieties. The structure of the oxidized carbohydrate moiety may be cyclic or linear.

The carbohydrate moiety may be linked directly to the polypeptide backbone. Preferably, the carbohydrate moiety is part of a carbohydrate side chain. More preferably, the carbohydrate moiety is the terminal moiety of the carbohydrate side chain.

In an even more preferred embodiment, the carbohydrate moiety is a galactose residue of the carbohydrate side chain, preferably the terminal galactose residue of the carbohydrate side chain. This galactose residue can be made available for reaction with the functional group X comprised in the reaction product of hydroxyalkyl starch and compound (L) or the reaction product of hydroxyalkyl starch and compound (D) which is further reacted with compound (L), by removal of terminal sialic acids, followed by oxidation, as described hereinunder.

In a still further preferred embodiment, the reaction product of hydroxyalkyl starch and compound (L) or the reaction product of hydroxyalkyl starch and compound (D) which is further reacted with compound (L) is linked to a sialic acid residue of the carbohydrate side chains, preferably the terminal sialic acid residue of the carbohydrate side chain.

Oxidation of terminal carbohydrate moieties can be performed either chemically or enzymatically.

Methods for the chemical oxidation of carbohydrate moieties of polypeptides are known in the art and include the treatment with perjodate (Chamow et al., 1992, J. Biol. Chem., 267, 15916-15922).

By chemically oxidizing, it is in principle possible to oxidize any carbohydrate moiety, being terminally positioned or not. However, by choosing mild conditions (1 mM periodate, 0 °C in contrast to harsh conditions: 10 mM periodate 1h at room temperature), it is possible to preferably oxidize the terminal sialic acid of a carbohydrate side chain.

Alternatively, the carbohydrate moiety may be oxidized enzymatically. Enzymes for the oxidation of the individual carbohydrate moieties are known in the art, e.g. in the case of galactose the enzyme is galactose oxidase. If it is intended to oxidize terminal galactose moieties, it will be eventually necessary to remove terminal sialic acids (partially or completely) if the polypeptide has been produced in cells capable of attaching sialic acids to carbohydrate chains, e.g. in mammalian cells or in cells which have been genetically modified to be capable of attaching sialic acids to carbohydrate chains. Chemical or enzymatic methods for the removal of sialic acids are known in the art (Chaplin and Kennedy (eds.), 1996, Carbohydrate Analysis: a practical approach, especially Chapter 5 Montreuill, Glycoproteins, pages 175-177; IRL Press Practical approach series (ISBN 0-947946-44-3)).

According to another preferred embodiment of the present invention, the functional group of the polypeptide is the thio group. Therefore, the reaction product of hydroxyalkyl starch and compound (L) or the reaction product of hydroxyalkyl starch and compound (D) which is further reacted with compound (L) may be linked to the polypeptide via a thioether group wherein the S atom can be derived from any thio group comprised in the polypeptide.

In the context of this embodiment, it is particularly preferred to react the polypeptide with a reaction product of hydroxyalkyl starch and compound (D) which is further reacted with compound (L).

Therefore, the present invention also relates to a method as described above wherein the reaction product of hydroxyalkyl starch and compound (D) is further reacted with compound (L) is reacted with the polypeptide via a thio group comprised in the polypeptide.

Therefore, the present invention also relates to a method as described above wherein the reaction product of hydroxyalkyl starch and compound (D) which is further reacted with compound (L) is reacted with the polypeptide via an oxidized carbohydrate moiety and a thio group comprised in the polypeptide.

The thio group may be present in the polypeptide as such. Moreover, it is possible to introduce a thio group into the polyeptide according to a suitbale method. Among others, chemical methods may be mentioned. If a disulfide bridge is present in the polypeptide, it is possible to reduce the -S-S- structure to get a thio group. It is also possible to transform an amino group present in the polypeptide into a SH group by reaction the polypeptide via the amino group with a compound which has at least two different functional groups, one of which is capable of being reacted with the amino group and the other is an SH group or a precursor of an SH group. This modification of an amino group may be regarded as an example where the protein is first reacted with a compound (L) which has at least two different functional groups, one of which is capable of being reacted with the amino group and the other is an SH group, and the resulting reaction product is then reacted with, e.g., a HAS derivative comprising HAS and a compound (D), said derivative comprising a functional group being capable of reacting with the SH group. It is also possible to introduce an SH group by mutation of the polypeptide such as by introducing a cystein or a suitable SH functional amino acid into the polypeptide or such as removing a cystein from the polypeptide so as to disable another cystein in the polypeptide to form a disulfide bridge.

As an especially preferred polypeptide, erythropoietin (EPO) is used.

Therefore, the present invention also relates to a method as described above wherein the polypeptide is erythropoietin.

The EPO can be of any human (see e.g. Inoue, Wada, Takeuchi, 1994, An improved method for the purification of human erythropoietin with high in vivo activity from the urine of anemic patients, Biol. Pharm. Bull. 17(2), 180-4; Miyake, Kung, Goldwasser, 1977, Purification of human erythropoietin., J. Biol. Chem., 252(15), 5558-64) or another mammalian source and can be obtained by purification from naturally occurring sources like human kidney, embryonic human liver or animal, preferably monkey kidney. Furthermore, the expression "erythropoietin" or "EPO" encompasses also an EPO variant wherein one or more amino acids (e.g. 1 to 25, preferably 1 to 10, more preferred 1 to 5, most preferred 1 or 2) have been exchanged by another amino acid and which exhibits erythropoietic activity (see e.g. EP 640 619 B1). The measurement of erythropoietic activity is described in the art (for measurement of activity in vitro see e.g. Fibi et al., 1991, Blood, 77, 1203 ff; Kitamura et al,1989, J. Cell Phys., 140, 323-334; for measurement of EPO activity in vivo see Ph. Eur. 2001, 911-917; Ph. Eur. 2000, 1316 Erythropoietini solutio concentrata, 780- 785; European Pharmacopoeia (1996/2000); European Pharmacopoeia, 1996, Erythropoietin concentrated solution, Pharmaeuropa., 8, 371-377; Fibi, Hermentin, Pauly, Lauffer, Zettlmeissl., 1995, N- and O-glycosylation muteins of recombinant human erythropoietin secreted from BHK-21 cells, Blood, 85(5), 1229-36; (EPO and modified EPO forms were injected into female NMRI mice (equal amounts of protein 50 ng/mouse) at day 1, 2 and 3 blood samples were taken at day 4 and reticulocytes were determined)). Further publications where tests for the measurement of the activity of EPO are Barbone, Aparicio, Anderson, Natarajan, Ritchie, 1994, Reticulocytes measurements as a bioassay for erythropoietin, J. Pharm. Biomed. Anal., 12(4), 515-22; Bowen, Culligan, Beguin, Kendall, Villis, 1994, Estimation of effective and total erythropoiesis in myelodysplasia using serum transferrin receptor and erythropoietin concentrations, with automated reticulocyte parameters, Leukemi, 8(1), 151-5; Delorme, Lorenzini, Giffin, Martin, Jacobsen, Boone, Elliott, 1992, Role of glycosylation on the secretion and biological activity of erythropoietin, Biochemistry, 31(41), 9871-6; Higuchi, Oheda, Kuboniwa, Tomonoh, Shimonaka, Ochi, 1992 ;Role of sugar chains in the expression of the biological activity of human erythropoietin, J. Biol. Chem., 267(11), 7703-9; Yamaguchi, Akai, Kawanishi, Ueda, Masuda, Sasaki, 1991, Effects of site-directed removal of N-glycosylation sites in human erythropoietin on its production and biological properties, J. Biol. Chem., 266(30), 20434-9; Takeuchi, Inoue, Strickland, Kubota, Wada, Shimizu, Hoshi, Kozutsumi, Takasaki, Kobata, 1989, Relationship between sugar chain structure and biological activity of recombinant human erythropoietin produced in Chinese hamster ovary cells, Proc. Natl. Acad. Sci. USA, 85(20), 7819-22; Kurtz, Eckardt, 1989, Assay methods for erythropoietin, Nephron., 51(1), 11-4 (German); Zucali, Sulkowski, 1985, Purification of human urinary erythropoietin on controlled-pore glass and silicic acid, Exp. Hematol., 13(3), 833-7; Krystal, 1983, Physical and biological characterization of erythroblast enhancing factor (EEF), a late acting erythropoietic stimulator in serum distinct from erythropoietin, Exp. Hematol., 11(1), 18-31.

Preferably, the EPO is recombinantly produced. This includes the production in eukaryotic or prokaryotic cells, preferably mammalian, insect, yeast, bacterial cells or in any other cell type which is convenient for the recombinant production of EPO. Furthermore, the EPO may be expressed in transgenic animals (e.g. in body fluids like milk, blood, etc.), in eggs of transgenic birds, especially poultry, preferred chicken, or in transgenic plants.

The recombinant production of a polypeptide is known in the art. In general, this includes the transfection of host cells with an appropriate expression vector, the cultivation of the host cells under conditions which enable the production of the polypeptide and the purification of the polypeptide from the host cells. For detailled information see e.g. Krystal, Pankratz, Farber, Smart, 1986, Purification of human erythropoietin to homogeneity by a rapid five-step procedure, Blood, 67(1), 71.-9; Quelle, Caslake, Burkert, Wojchowski, 1989, High-level expression and purification of a recombinant human erythropoietin produced using a baculovirus vector, Blood, 74(2), 652-7; EP 640 619 B1 and EP 668 351 B1.

In a preferred embodiment, the EPO has the amino acid sequence of human EPO (see EP 148 605 B2).

The EPO may comprise one or more carbohydrate side chains, preferably 1 to 12, more preferably 1 to 9, even more preferably 1 to 6 and particularly 1 to 4, especially preferably 4 carbohydrate side chains, attached to the EPO via N- and/ or O-linked glycosylation, i.e. the EPO is glycosylated. Usually, when EPO is produced in eukaryotic cells, the polypeptide is posttranslationally glycosylated. Consequently, the carbohydrate side chains may have been attached to the EPO during biosynthesis in mammalian, especially human, insect or yeast cells. The structure and properties of glycosylated EPO have been extensively studied in the art (see EP 428 267 B1; EP 640 619 B1; Rush, Derby, Smith, Merry, Rogers, Rohde, Katta, 1995, Microheterogeneity of erythropoietin carbohydrate structure, Anal Chem., 67(8), 1442-52; Takeuchi, Kobata, 1991, Structures and functional roles of the sugar chains of human erythropoietins, Glycobiology, 1(4), 337-46, (Review).

Therefore, the hydroxyalkyl starch derivative according to the present invention may comprise at least one, preferably 1 to 12, more preferably 1 to 9, even more preferably 1 to 6 and particularly preferably 1 to 4 HAS molecules per EPO molecule. The number of HAS-molecules per EPO molecule can be determined by quanatitative carbohydrate compositional analysis using GC-MS after hydrolysis of the product and derivatisation of the resulting monosaccharides (see Chaplin and Kennedy (eds.), 1986, Carbohydrate Analysis: a practical approach, IRL Press Practical approach series (ISBN 0-947946-44-3), especially Chapter 1, Monosaccharides, page 1-36; Chapter 2, Oligosaccharides, page 37-53, Chapter 3, Neutral Polysaccharides, page 55-96).

According to an especially preferred embodiment of the present invention, the carbohydrate moiety linked to EPO, is part of a carbohydrate side chain. More preferably, the carbohydrate moiety is the terminal moiety of the carbohydrate side chain. In an even more preferred embodiment, the carbohydrate moiety is a galactose residue of the carbohydrate side chain, preferably the terminal galactose residue of the carbohydrate side chain. This galactose residue can be made available for reaction with the reaction product of compound (I) and compound (II) by removal of terminal sialic acids, followed by oxidation, as described hereinunder. In a further preferred embodiment, the reaction product of compound (I) and (II) is linked to a sialic acid residue of the carbohydrate side chains, preferably the terminal sialic acid residue of the carbohydrate side chain. The sialic acid is oxidized as described hereinunder.

Particularly preferably this galactose residue is made available for reaction with the reaction product of hydroxyalkyl starch and compound (L) or the reaction product of hydroxyalkyl starch and compound (D) which is further reacted with compound (L) via the functional group X by removal of terminal sialic acid followed by oxidation.

More preferably, this galactose residue is made available for reaction with the reaction product of hydroxyalkyl starch and compound (L) or the reaction product of hydroxyalkyl starch and compound (D) which is further reacted with compound (L) via the functional group X by oxidation wherein terminal sialic acid is not removed.

As mentioned above, the reaction product of hydroxyalkyl starch and compound (L) or the reaction product of hydroxyalkyl starch and compound (D) which is further reacted with compound (L) be reacted with a thio group comprised in EPO.

It is also possible to react the reaction product of hydroxyalkyl starch and compound (L) or the reaction product of hydroxyalkyl starch and compound (D) which is further reacted with compound (L) with a thio group as well as with a carbohydrate moiety, each of them comprised in the at least one further compound, preferably a polypeptide, more preferably erythropoietin.

According to a preferred embodiment, this SH group may be linked to a preferably oxidized carbohydrate moiety, e.g. by using a hydroxylamine derivative, e.g. 2-(aminooxy)ethylmercaptan hydrochloride (Bauer L. et al., 1965, J. Org. Chem., 30, 949) or by using a hydrazide derivative, e.g. thioglycolic acid hydrazide (Whitesides et al., 1977, J. Org. Chem., 42, 332.)

According to a further preferred embodiment, the thio group is preferably introduced in an oxidized carbohydrate moiety of EPO, more preferably an oxidized carbohydrate moiety which is part of a carbohydrate side chain of EPO.

Preferably, the thio group is derived from a naturally occurring cysteine or from an added cysteine. More preferably, the EPO has the amino acid sequence of human EPO and the naturally occurring cysteines are cysteine 29 and/or 33. In a more preferred embodiment, t the reaction product of hydroxyalkyl starch and compound (L) or the reaction product of hydroxyalkyl starch and compound (D) which is further reacted with compound (L) is reacted with cysteine 29 whereas cysteine 33 is replaced by another amino acid. Alternatively, the reaction product of hydroxyalkyl starch and compound (L) or the reaction product of hydroxyalkyl starch and compound (D) which is further reacted with compound (L) is reacted with cysteine 33 whereas cysteine 29 is replaced by another amino acid.

In the context of the present invention, the term "added cysteines" indicates that the polypeptides, preferably EPO, comprise a cysteine residue which is not present in the wild-type polypeptide.

In the context of this aspect of the invention, the cysteine may be an additional amino acid added at the N- or C-terminal end of EPO.

Furthermore, the added cysteine may have been added by replacing a naturally occuring amino acid by cysteine or a suitably substituted cysteine. Preferably, in the context of this aspect of the invention, the EPO is human EPO and the replaced amino acid residue is serine 126.

As to the reaction conditions regarding the reaction of the reaction product of hydroxyalkyl starch and compound (L), optionally with compound (D), with the further compound (M), no specific limitations exist, and the reaction conditions may be adjusted to the specific needs. According to an especially preferred embodiment of the present invention, water is used as solvent, either alone or in combination with at least one other solvent. As at least one other solvent, DMSO, DMF, methanol or ethanol may be mentioned. Preferred solvents other than water are methanol and ethanol. According to other preferred embodiments, DMSO or DMF or methanol or ethanol or a mixture of two or more thereof is used as solvent.

If, e.g., hydroxyalkyl starch is reacted with compound (L) in an aqueous system, as it is the case, e.g., when hydroxyethyl starch is reacted with a hydroxyamine such as O-[2-(2-aminooxy-ethoxy)-ethyl]-hydroxyl amine, via reaction of the non-oxidised reducing end of the starch, and the reaction product is further reacted with a polypeptide, preferably erythropoietin, via an aldehyde, keto, acetal or hemiacetale group, the reaction temperatur is preferably preferably in the range of from 4 to 37 °C, more preferably of from 10 to 30 °C and especially preferably of from 15 to 25 °C.

Isolation of the reaction product comprising the further compound (M), preferably the polypeptide and especially pererably erythropoietin, can be performed by using known procedures for the purification of natural and recombinant EPO (e.g.size exclusion chromatography, ion-exchange chromatography, RP-HPLC, hydroxyapatite chromatography, hydrophobic interaction chromatography or combinations thereof). Isolation of the reaction product may be carried out by a suitable process which may comprise one or more steps. According to a preferred embodiment of the present invention, the reaction product is first separated off the reaction mixture or the mixture of the reaction mixture with, e.g., the ethanol-acetone mixture, by a suitable method such as centrifugation or filtration. In a second step, the separated reaction product may be subjected to a further treatment such as an after-treatment like dialysis, centrifugal filtration or pressure filtration, ion exchange chromatography such as, e.g., by a column containing Q-sepharose, HPLC, MPLC, gel filtration and/or lyophilisation. According to one preferred embodiment, the separated reaction product is first dialysed, preferably against water, and then lyophilized until the solvent content of the reaction product is sufficiently low according to the desired specifications of the product. Lyophilisation may be carried out at temperature of from 20 to 35 °C, preferably of from 25 to 30 °C. According to another preferred embodiment, the reaction mixture comprising the reaction product is applied to a column containing Q-Sepharose to give an eluate which is concentrated, e.g. by centrifugal filtration.

It is another object of the present invention to provide hydroxyalkyl starch derivatives which are produced by one or more of the aforesaid methods.

Therefore, the present invention relates to a hydroxyalkyl starch derivative obtainable by a method of producing a hydroxyalkyl starch derivative, said hydroxyalkyl starch having a structure according to formula (I) 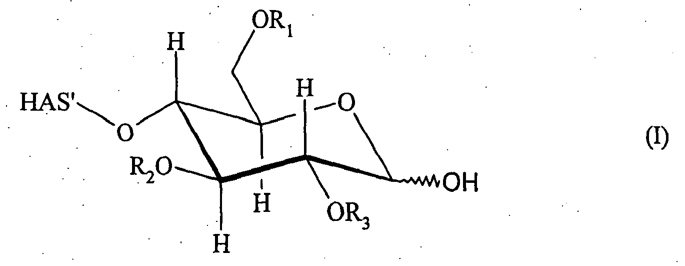 comprising reacting
- hydroxyalkyl starch of formula (I) at its optionally oxidized reducing end or
- a hydroxyalkyl starch derivative, obtainable by reacting hydroxyalkyl starch of formula (I) at its optionally oxidized reducing end with a compound (D), said compound (D) comprising
   -- at least one functional group Z₁ capable of being reacted with the optionally oxidized reducing end of the hydroxyalkyl starch, and
   -- at least one functional group W,
   with a compound (L) comprising
- at least one functional group Z₁ capable of being reacted with said hydroxyalkyl starch, or at least one functional group Z₂ capable of being reacted with functional group W comprised in said hydroxyalkyl starch derivative, and
- at least one functional group X capable of being reacted with a functional group Y of a further compound (M),
wherein said functional group Y is selected from the group consisting of an aldehyd group, a keto group, a hemiacetal group, an acetal group, or a thio group.

According to a preferred embodiment, the present invention relates to a hydroxyalkyl starch derivative as described above wherein R₁, R₂ and R₃ are independently hydrogen or a linear or branched hydroxyalkyl group.

According to an even more preferred embodiment, the present invention relates to a hydroxyalkyl starch derivative as described above wherein R₁, R₂ and R₃ are independently hydrogen or a 2-hydroxyethyl group.

According to a further preferred embodiment, the present invention relates to a hydroxyalkyl starch derivative as described wherein the hydroxyalkyl starch is hydroxyethyl starch.

According to a further preferred embodiment, the present invention relates to a hydroxyalkyl starch derivative as described above wherein the functional group Z₁ comprises the structure -NH-.

According to an especially preferred embodiment, the present invention relates to a hydroxyalkyl starch derivative as described above wherein Z₁ is selected from the group consisting of 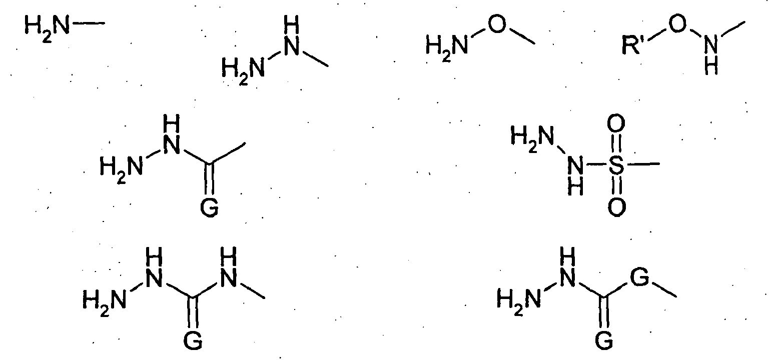 wherein G is O or S and, if present twice, independently O or S, and R' is methyl.

According to a further preferred embodiment, the present invention relates to a hydroxyalkyl starch derivative as described above wherein the functional group Y is selected from the group consisting of an aldehyd group, a keto group, a hemiacetal group, and an acetal group, and the functional group X comprises the structure -NH-.

According to an especially preferred embodiment, the present invention relates to a hydroxyalkyl starch derivative as described above X is selected from the group consisting of 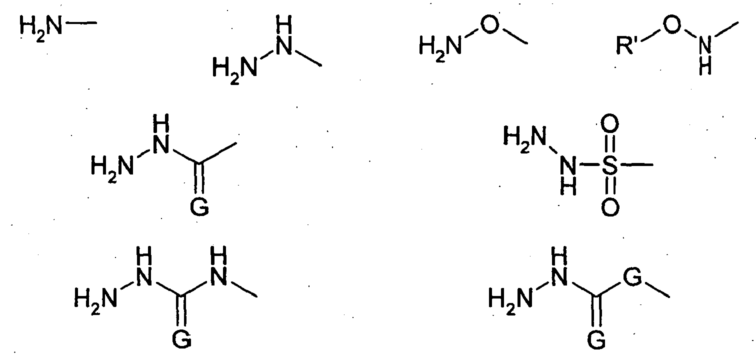 wherein G is O or S and, if present twice, independently O or S, and R is methyl.

According to a further preferred embodiment, the present invention relates to a hydroxyalkyl starch derivative as described above wherein the functional group Y is - SH and the functional group X is selected from the group consisting of 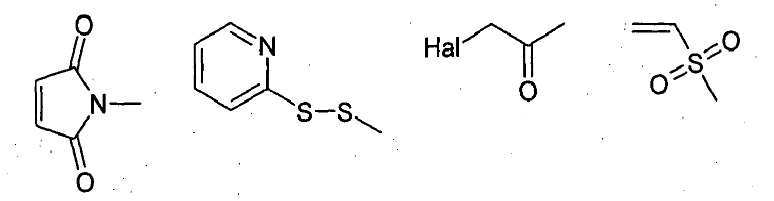 wherein Hal is Cl, Br or I.

According to a further preferred embodiment, the present invention relates to a hydroxyalkyl starch derivative as described above wherein the functional group W or the functional group Z₂ is -SH and the functional group Z₂ or the functional group W is selected from the group consisting of 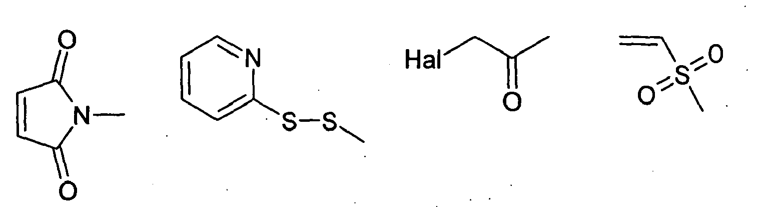 wherein Hal is Cl, Br, or I.

According to a further preferred embodiment, the present invention relates to a hydroxyalkyl starch derivative as described above wherein the functional group W or the functional group Z₂ is selected from the group consisting of an activated ester, as described above, or a carboxy group which is optionally transformed into an activated ester and the functional group Z₂ or the functional group W is selected from the group consisting of 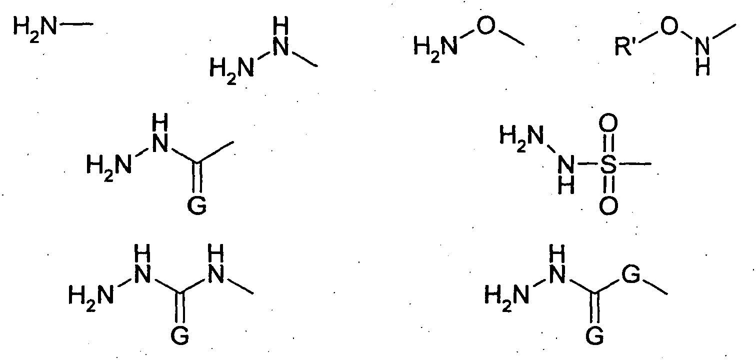 wherein G is O or S and, if present twice, independently O or S, and R is methyl.

According to an especially preferred embodiment, the present invention relates to a hydroxyalkyl starch derivative as described above wherein the reducing end of the hydroxyalkyl starch is not oxidized prior to the reaction with compound (D) or compound (L), said hydroxyalkyl starch thus having a structure according to formula (I)

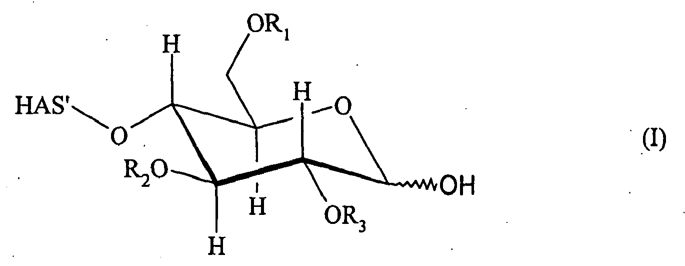

According to another especially preferred embodiment, the present invention relates to a hydroxyalkyl starch derivative as described above wherein the reducing end of the hydroxyalkyl starch is oxidized prior to the reaction with compound (D) or compound (L), said hydroxyalkyl starch thus having a structure according to formula (IIa) 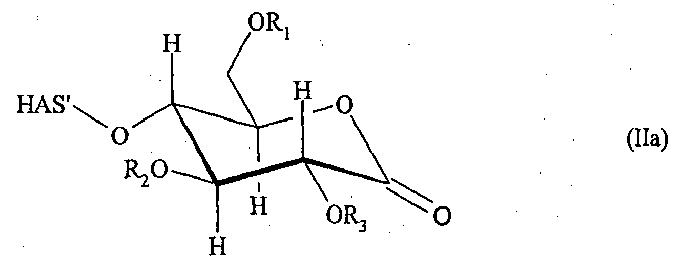 and/or according to formula (IIb)

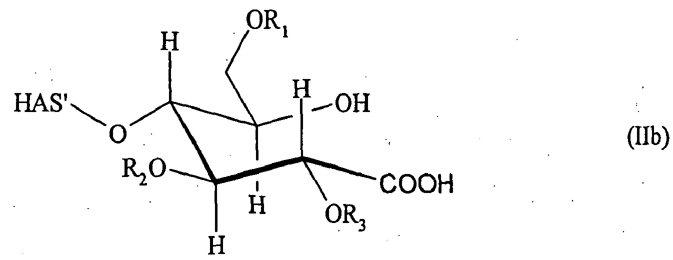

According to a further preferred embodiment, the present invention relates to a hydroxyalkyl starch derivative as described above wherein the reducing end is oxidized by an alkaline iodine solution.

According to a further preferred embodiment, the present invention relates to a hydroxyalkyl starch derivative as described above wherein hydroxyalkyl starch is reacted with a compound (L) via the reaction of functional group Z₁ with the optionally oxidized reducing end of the hydroxyalkyl starch and the resulting reaction product is reacted with a further compound (M) via the reaction of the functional group X comprised in compound (L) with the functional group Y comprised in compound (M).

According to yet a further preferred embodiment, the present invention relates to a hydroxyalkyl starch derivative as described above hydroxyalkyl starch is reacted with a compound (L) via the reaction of functional group Z₁ with the optionally oxidized reducing end of the hydroxyalkyl starch, where compound (L), prior to the reaction with hydroxyalkyl starch, is reacted with a further compound (M) via the reaction of functional group X comprised in compound (L) with the functional group Y comprised in compound (M).

According to still a further preferred embodiment, the present invention relates to a hydroxyalkyl starch derivative as described above wherein hydroxyalkyl starch is reacted with a compound (D) via the reaction of the functional group Z₁ comprised in compound (D), with the optionally oxidized reducing end of the hydroxyalkyl starch to give a first hydroxyalkyl starch derivative, and where the first hydroxyalkyl starch derivative is reacted with a compound (L) via the reaction of functional group Z₂ comprised in compound (L) with the functional group W comprised in compound (D) to give a second hydroxyalkyl starch derivative.

According to an especially preferred embodiment, the present invention relates to the aforesaid hydroxyalkyl starch derivative wherein the second hydroxyalkyl starch derivative is reacted with a further compound (M) via the reaction of functional group X comprised in compound (L) with the functional group Y comprised in compound (M).

According to a further preferred embodiment, the present invention relates to a hydroxyalkyl starch derivative as described above wherein hydroxyalkyl starch is reacted with a compound (D) via the reaction of functional group Z₁ comprised in compound (D) with the optionally oxidized reducing end of the hydroxyalkyl starch to give a first hydroxyalkyl starch derivative, and where the first hydroxyalkyl starch derivative is reacted, via the reaction of the functional group W, comprised in compound (D), and the functional group Z₂, comprised in compound (L), with compound (L), where compound (L), prior to the reaction with the first hydroxyalkyl starch derivative, is reacted with a further compound (M) via the reaction of functional group X comprised in compound (L) with the functional group Y comprised in compound (M).

According to an especially preferred embodiment, the present invention relates to a hydroxyalkyl starch derivative as described above wherein the at least one further compound (M) is a polypeptide.

According to a particularly preferred embodiment, the present invention relates to a hydroxyalkyl starch derivative as described above wherein the polypeptide is erythropoietin.

The hydroxyalkyl starch derivative which in the following is referred to as HAS-EPO conjugate and which is formed by reaction of hydroxyalkyl starch with compound (L) and optionally compound (D) and erythrpoietin, has the advantage that it exhibits an improved biological stability when compared to the erythropoietin before conjugation. Furthermore, it exhibits a higher biological activity than standard BRP EPO. This is mainly due to the fact that this hydroxyalkyl starch derivative is less or even not recognized by the removal systems of the liver and kidney and therefore persists in the circulatory system for a longer period of time. Furthermore, since the HAS is attached site-specifically, the risk of destroying the in-vivo biological activity of EPO by conjugation of HAS to EPO is minimized.

The HAS-EPO conjugate of the invention may exhibit essentially the same in-vitro biological activity as recombinant native EPO, since the in-vitro biological activity only measures binding affinity to the EPO receptor. Methods for determining the in-vitro biological activity are known in the art.

Furthermore, the HAS-EPO exhibits a greater in-vivo activity than the EPO used as a starting material for conjugation (unconjugated EPO). Methods for determining the in vivo biological activity are known in the art.

The HAS-EPO conjugate may exhibit an in vivo activity of from 110 % to 500 %, preferably of from 300 to 400 %, or preferably of from 110 to 300 %, more preferably from 110 % to 200 %, more preferably from 110 % to 180 % or from 110 to 150 %, most preferably from 110 % to 140 %, if the in-vivo activity of the unconjugated EPO is set as 100 %.

Compared to the highly sialylated EPO of Amgen (see EP 428 267 B1), the HAS-EPO exhibits preferably at least 50%, more preferably at least 70 %, even more preferably at least 85 % or at least 95 %, at least 150 %, at least 200 % or at least 300 % of the in vivo activity of the highly sialylated EPO if the in-vivo activity of highly sialylated EPO is set as 100 %. Most preferably, it exhibits at least 95 % of the in vivo activity of the highly sialylated EPO.

The high in-vivo biological activity of the HAS-EPO conjugate of the invention mainly results from the fact that the HAS-EPO conjugate remains longer in the circulation than the unconjugated EPO because it is less recognized by the removal systems of the liver and because renal clearance is reduced due to the higher molecular weight. Methods for the determination of the in-vivo half life time of EPO in the circulation are known in the art (Sytkowski, Lunn, Davis, Feldman, Siekman, 1998, Human erythropoietin dimers with markedly enhanced in vivo activity, Proc. Natl. Acad. Sci. USA, 95(3), 1184-8).

Consequently, it is a great advantage of the present invention that a HAS-EPO conjugate is provided which may be administered less frequently than the EPO preparations commercially available at present. While standard EPO preparations have to be administered at least every 3 days, the HAS-EPO conjugate of the invention is preferable administered twice a week, more preferably once a week.

Furthermore, the method of the invention has the advantage that an effective EPO derivative can be produced at reduced costs since the method does not comprise extensive and time consuming purification steps resulting in low final yield, e.g. it is not necessary to purify away under-sialylated EPO forms which are known to exhibit low or no in-vivo biological activity. Especially Example 8.11 (d) demonstrates that a HES-EPO produced with few modifications steps exhibits a 3-fold activity over standard BRP EPO.

It is yet another object of the present invention to provide a pharmaceutical composition which comprises, in a therapeutically effective amount, the HAS-EPO conjugate of the present invention.

Furthermore, the present invention relates to a pharmaceutical composition comprising, in a therapeutically effective amount, the HAS-polypeptide conjugate, preferably the HAS-EPO conjugate, more preferably the HES-EPO conjugate of the present invention. In a preferred embodiment, the pharmaceutical composition comprises further at least one pharmaceutically acceptable diluent, adjuvant and/or carrier useful in erythropoietin therapy.

Therefore, the present invention also relates to a pharmaceutical composition comprising, in a therapeutically effective amount, a hydroxyalkyl starch derivative obtainable by a method of producing a hydroxyalkyl starch derivative, said hydroxyalkyl starch having a structure according to formula (I) 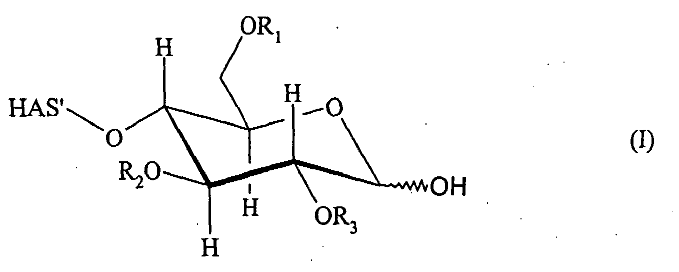 comprising reacting,
- hydroxyalkyl starch of formula (I) at its optionally oxidized reducing end or
- a hydroxyalkyl starch derivative, obtainable by reacting hydroxyalkyl starch of formula (I) at its optionally oxidized reducing end with a compound (D), said compound (D) comprising
   -- at least one functional group Z₁ capable of being reacted with the optionally oxidized reducing end of the hydroxyalkyl starch, and
   -- at least one functional group W,
   with a compound (L) comprising
- at least one functional group Z₁ capable of being reacted with said hydroxyalkyl starch, or at least one functional group Z₂ capable of being reacted with functional group W comprised in said hydroxyalkyl starch derivative, and
- at least one functional group X capable of being reacted with a functional group Y of a further compound (M),
wherein said functional group Y is selected from the group consisting of an aldehyd group, a keto group, a hemiacetal group, an acetal group, or a thio group,
said method of producing a hydroxyalkyl starch derivative further comprising reacting the reaction product comprising hydroxyalkyl starch, compound (L) and optionally compound (D) with a further compound (M) wherein the at least one further compound is a polypeptide.

Moreover, the present invention relates to the use of a hydroxyalkyl starch derivative as described for the preparation of a medicament for the treatment of anemic disorders or hematopoietic dysfunction disoirders or diseases related thereto.

According to a preferred embodiment, the present invention relates to a pharmaceutical composition as described above wherein the polypeptide is an antithrombin (AT), preferably AT III (Levy JH, Weisinger A, Ziomek CA, Echelard Y, Recombinant Antithrombin: Production and Role in Cardiovascular Disorder, Seminars in Thrombosis and Hemostasis 27, 4 (2001) 405-416; Edmunds T, Van Patten SM, Pollock J, Hanson E, Bernasconi R, Higgins E, Manavalan P, Ziomek C, Meade H, McPherson J, Cole ES, Transgenically Produced Human Antithrombin: Structural and Functional Comparison to Human Plasma-Derived Antithrombin, Blood 91, 12 (1998) 4661-4671; Minnema MC, Chang ACK, Jansen PM, Lubbers YTP, Pratt BM, Whittaker BG, Taylor FB, Hack CE, Friedman B, Recombinant human antithrombin III improves survival and attenuates inflammatory responses in baboons lethally challenged with Escherichia coli, Blood 95, 4 (2000) 1117-1123; Van Patten SM, Hanson EH, Bernasconi R, Zhang K, Manavaln P, Cole ES, McPherson JM, Edmunds T, Oxidation of Methionine Residues in Antithrombin, J. Biol. Chemistry 274, 15 (1999) 10268-10276).

According to other preferred embodiments, the present invention relates to pharmaceutical compositions wherein the polypeptide is G-CSF or IFN-beta.

According to an especially preferred embodiment, the present invention relates to a pharmaceutical composition as described above wherein the polypeptide is erythropoietin.

According to a further embodiment, the present invention relates to a pharmaceutical composition as described above wherein the functional group Y is -SH and compound (L) is a compound of general formula Z₁-L'-X where the functional group Z₁ is selected from the group consisting of 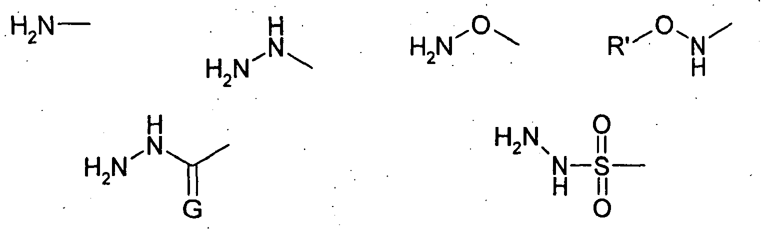 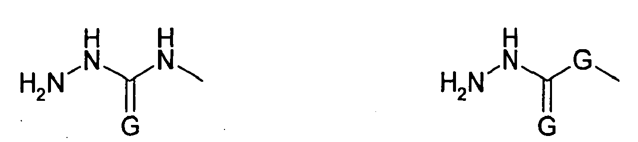 wherein G is O or S and, if present twice, independently O or S, and R' is methyl, and where the functional group X is selected from the group consisting of 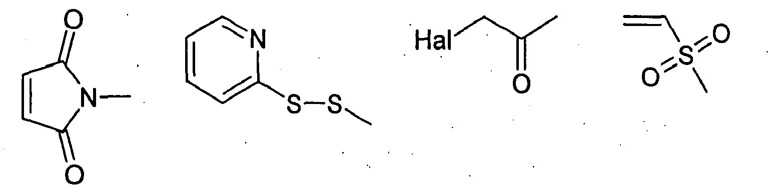 wherein Hal is Cl, Br or I, and where L' is an organic chain bridging Z₁ and X or where L' is absent.

According to a preferred embodiment, the present invention relates to a pharmaceutical composition as described above wherein the functional group Y is selected from the group consisting of an aldehyd group, a keto group, a hemiacetal group, and an acetal group, and compound (L) is a compound of general formula Z₁-L'-X where the functional group Z₁ is selected from the group consisting of 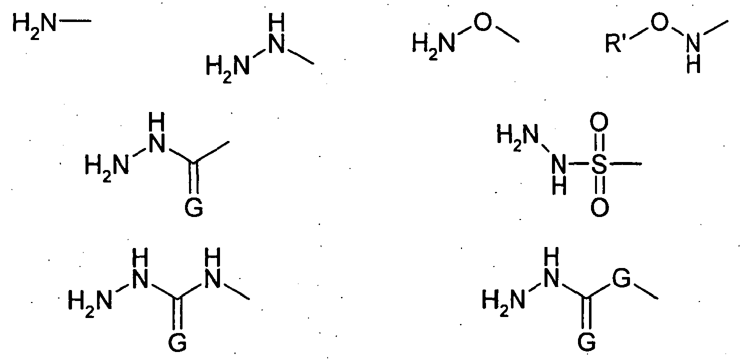 wherein G is O or S and, if present twice, independently O or S, and R' is methyl, and where the functional group X is selected from the group consisting of 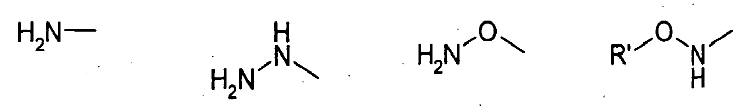 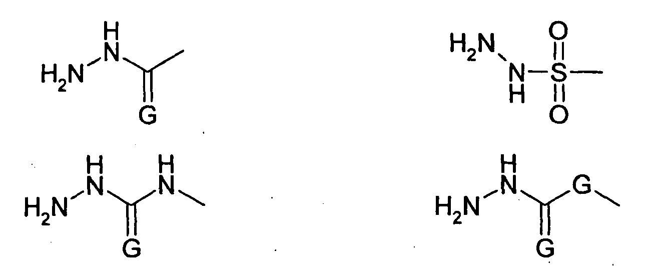 wherein G is O or S and, if present twice, independently O or S, and R' is methyl, and where L' is an organic chain bridging Z₁ and X or where L' is absent.

According to another embodiment, the present invention relates to a pharmaceutical composition as described above wherein the functional group Y is -SH, compound (D) is a compound of general formula Z₁-D'-W, and compound (L) is a compound of general formula Z₂-L'-X, where the functional group Z₁ is selected from the group consisting of 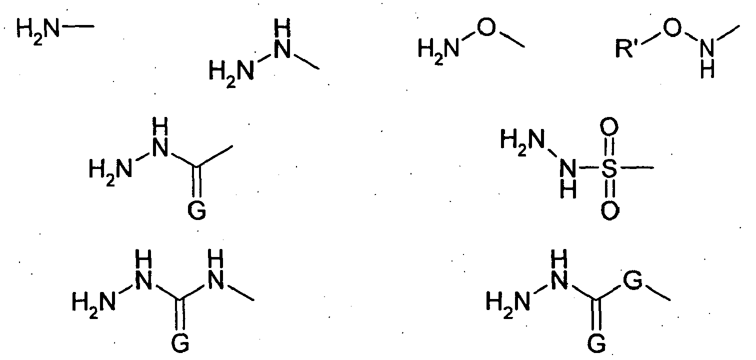 wherein G is O or S and, if present twice, independently O. or S, and R' is methyl, where the functional group X is selected from the group consisting of 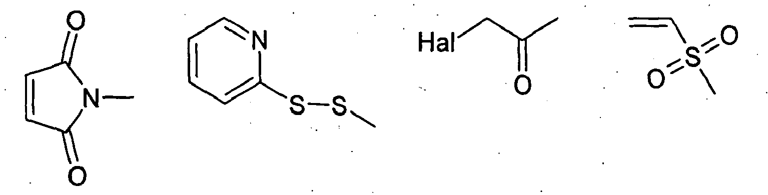 wherein Hal is Cl, Br or I, where the functional group W or the functional group Z₂ is -SH and the functional group Z₂ or the functional group W is selected from the group consisting of 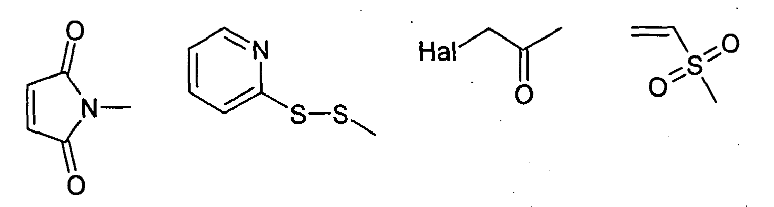 wherein Hal is Cl, Br, or I, or where the functional group W or the functional group Z₂ is selected from the group consisting of an activated ester, as described above, or a carboxy group which is optionally transformed into an activated ester and the functional group Z₂ or the functional group W is selected from the group consisting of 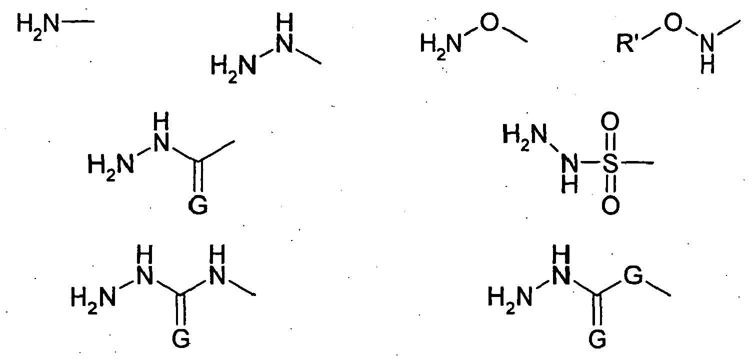 wherein G is O or S and, if present twice, independently O or S, and R' is methyl, and where D' is an organic chain bridging Z₁ and W or where D' is absent and where L' is an organic chain bridging Z₂ and X or where L' is absent.

According to yet another embodiment, the present invention relates to a pharmaceutical composition as described above wherein the functional group Y is selected from the group consisting of an aldehyd group, a keto group, a hemiacetal group, and an acetal group, compound (D) is a compound of general formula Z₁-D'-W, and compound (L) is a compound of general formula Z₂-L'-X, where the functional group Z₁ is selected from the group consisting of 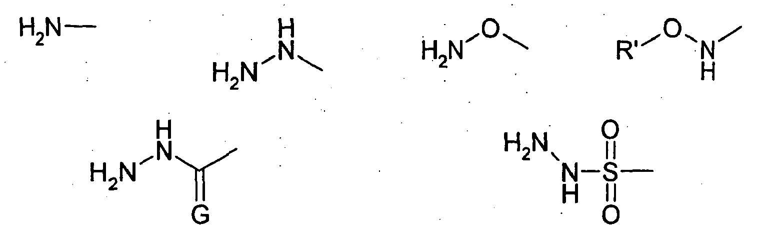 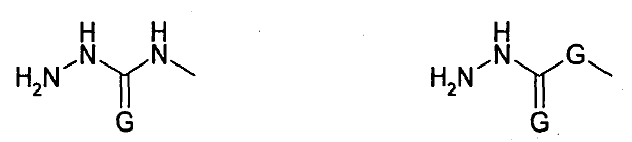 wherein G is O or S and, if present twice, independently O or S, and R' is methyl, where the functional group X is selected from the group consisting of 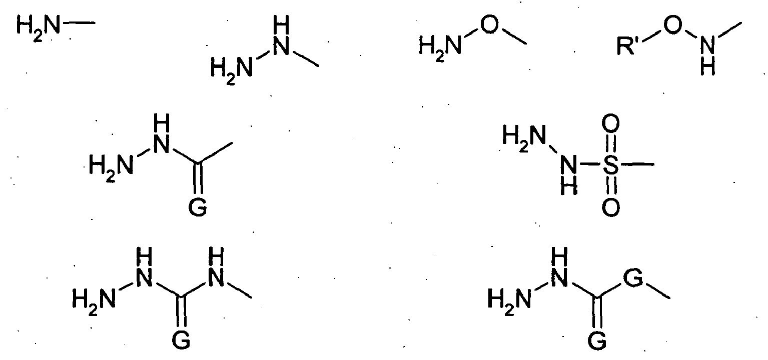 wherein G is O or S and, if present twice, independently O or S, and R' is methyl, the functional group W or the functional group Z₂ is -SH and the functional group Z₂ or the functional group W is selected from the group consisting of 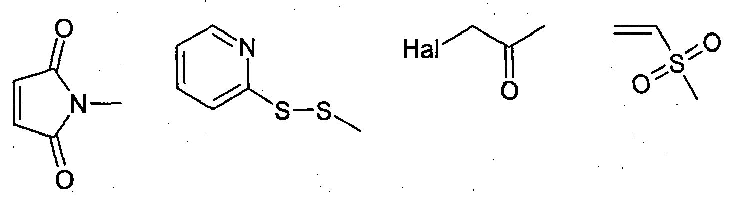 wherein Hal is Cl, Br, or I., or where the functional group W or the functional group Z₂ is selected from the group consisting of an activated ester, as described above, or a carboxy group which is optionally transformed into an activated ester and the functional group Z₂ or the functional group W is selected from the group consisting of 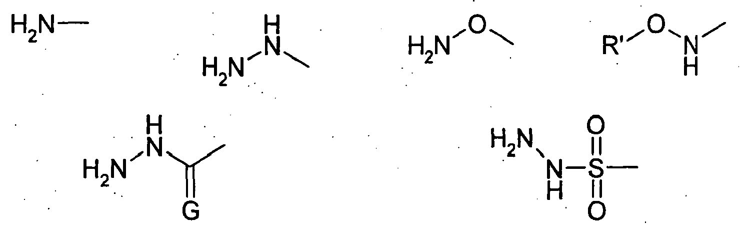 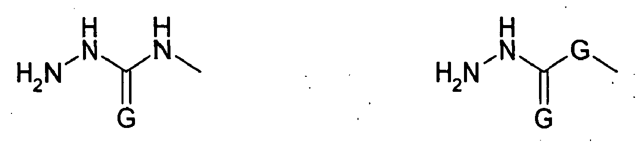 wherein G is O or S and, if present twice, independently O or S, and R' is methyl, and where D' is an organic chain bridging Z₁ and W or where D' is absent and where L' is an organic chain bridging Z₂ and X or where L' is absent.

According to a particularly preferred embodiment, the present invention relates to a pharmaceutical composition as described above wherein hydroxyethyl starch is reacted in an aqueous medium with a compound according to the following formula 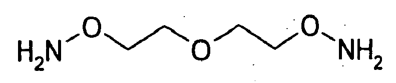 and the reaction product is reacted with erythropoietin.

According to an even more preferred embodiment, the present invention relates to the aformentioned pharmaceutical composition wherein the erythropoietin is oxidised with sodium periodate prior to the reaction.

According to a further preferred embodiment, the present invention relates to pharmaceutical composition as described above wherein the erythropoietin is partially desialylated and subsequently oxidised with sodium periodate prior to the reaction.

According to a further preferred embodiment of the present invention, pharmaceutical compositions comprising a hydroxyalkyl starch derivative which are produced on the basis of a completely reduced Thio-EPO according to Example 6 are excluded.

The above-mentioned pharmaceutical composition is especially suitable for the treatment of anemic disorders or hematopoietic dysfunction disorders or diseases related thereto.

A "therapeutically effective amount" as used herein refers to that amount which provides therapeutic effect for a given condition and administration regimen. The administration of erythropoietin isoforms is preferably by parenteral routes. The specific route chosen will depend upon the condition being treated. The administration of erythropoietin isoforms is preferably done as part of a formulation containing a suitable carrier, such as human serum albumin, a suitable diluent, such as a buffered saline solution, and/or a suitable adjuvant. The required dosage will be in amounts sufficient to raise the hematocrit of patients and will vary depending upon the severity of the condition being treated, the method of administration used and the like.

The object of the treatment with the pharmaceutical composition of the invention is preferably an increase of the hemoglobin value of more than 6.8 mmol/l in the blood. For this, the pharmaceutical composition may be administered in a way that the hemoglobin value increases between from 0.6 mmol/l and 1.6 mmol/l per week. If the hemoglobin value exceeds 8.7 mmol/l, the therapy should be preferably interrupted until the hemoglobin value is below 8.1 mmol/l.

The composition of the invention is preferably used in a formulation suitable for subcutaneous or intravenous or parenteral injection. For this, suitable excipients and carriers are e.g. sodium dihydrogen phosphate, disodium hydrogen phosphate, sodium chlorate, polysorbate 80, HSA and water for injection. The composition maybe administered three times a week, preferably two times a week, more preferably once a week, and most preferably every two weeks.

Preferably, the pharmaceutical composition is administered in an amount of 0.01-10 µg/kg body weight of the patient, more preferably 0,1 to 5 µg/kg, 0,1 to 1 µg/kg, or 0.2-0.9 µg/kg, most preferably 0.3-0.7 µg/kg, and most preferred 0.4-0.6 µg/kg body weight.

In general, preferably between 10 µg and 200 µg, preferably between 15 µg and 100 µg are administered per dosis.

The invention further relates to a HAS-polypeptide according to the present invention for use in method for treatment of the human or animal body.

The invention further relates to the use of a HAS-EPO conjugate of the present invention for the preparation of a medicament for the treatment of anemic disorders or hematopoietic dysfunction disorders or diseases related hereto.

In case compound (L) used in according to the present invention comprises one or more chiral centers, compound (II) may be present in R conformation or in S conformation or as racemic compound with respect to each chiral center.

In case compound (D) optionally used in the present invention comprises one or more chiral centers, compound (D) may be present in R conformation or in S conformation or as racemic compound with respect to each chiral center.

The invention is further illustrated by the following examples, tables an figures which are in no way intended to restrict the scope of the present invention.

### Short description of the Figures

### Figure 1

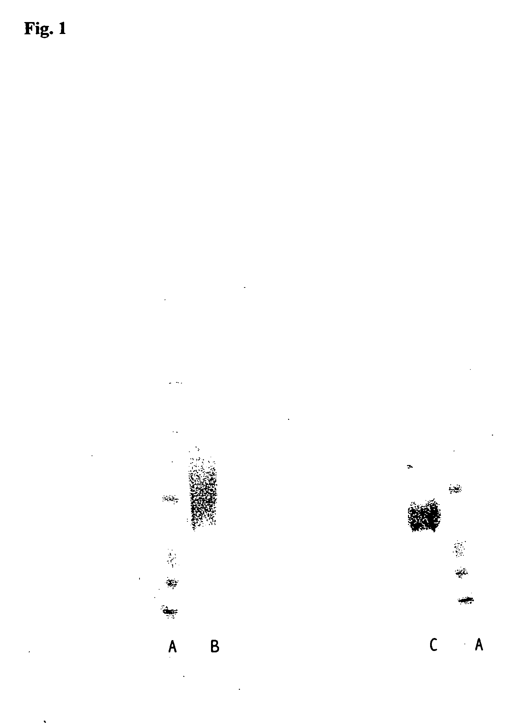

Figure 1 shows an SDS page analysis of the HES-EPO conjugate, produced according to example 5.1.
- Lane A:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.
- Lane B:: Crude product after conjugation according to example 5.1.
- Lane C:: EPO starting material.

### Figure 2

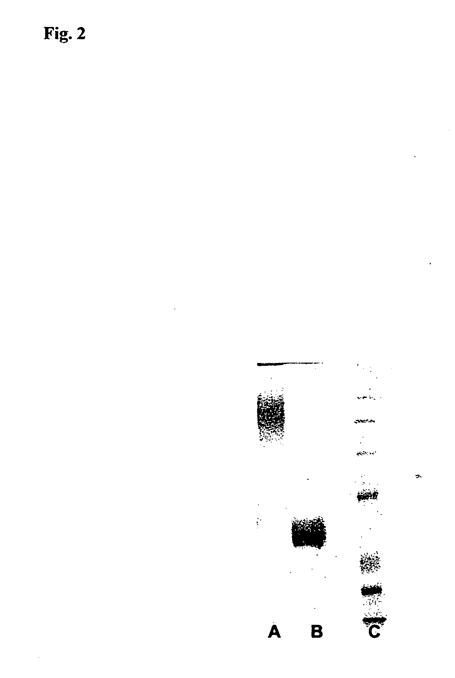

Figure 2 shows an SDS page analysis of the HES-EPO conjugate, produced according to example 5.3.
- Lane A:: Crude product after conjugation according to example 5.3.
- Lane B:: EPO starting material.
- Lane C:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.

### Figure 3

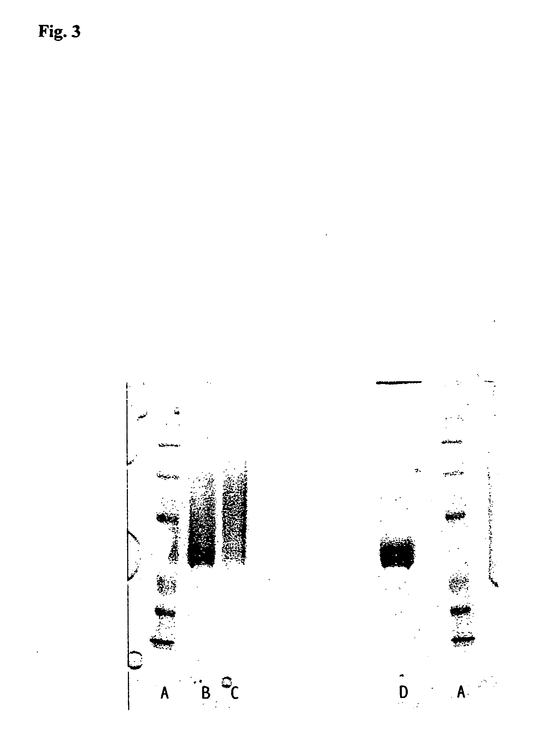

Figure 3 shows an SDS page analysis of the HES-EPO conjugate, produced according to example 5.4 and 5.5.
- Lane A:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.
- Lane B:: Crude product after conjugation according to example 5.4.
- Lane C:: Crude product after conjugation according to example 5.5.
- Lane D:: EPO starting material.

### Figure 4

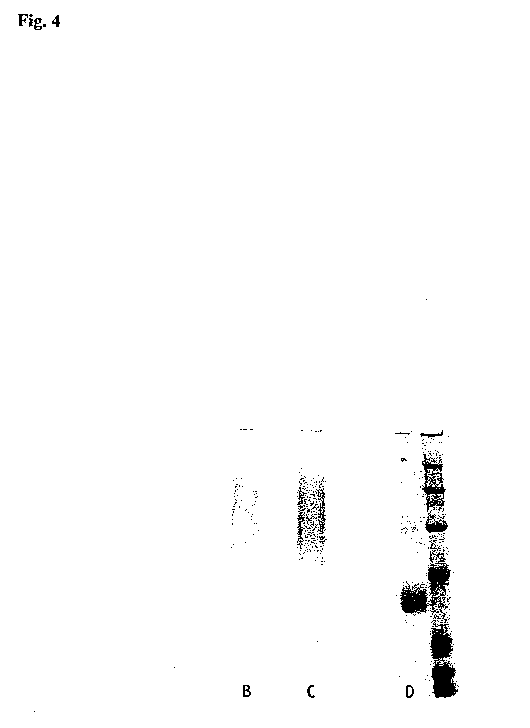

Figure 4 shows an SDS page analysis of HES-EPO conjugates, produced according to examples 7.1 and 7.4.
- Lane A:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.
- Lane B:: Crude product after conjugation according to example 7.4.
- Lane C:: Crude product after conjugation according to example 7.1.
- Lane D:: EPO starting material.

### Figure 5

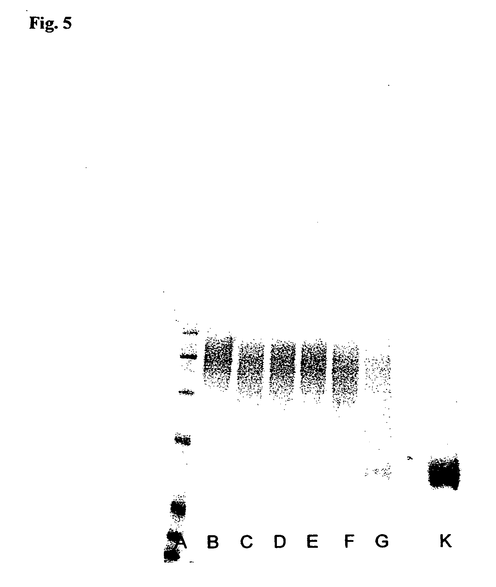

Figure 5 shows an SDS page analysis of HES-EPO conjugates, produced according to examples 7.2, 7.3, 7.5, and 7.6.
- Lane A:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.
- Lane B:: Crude product after conjugation according to example 7.6, based on Example 1.3 b).
- Lane C:: Crude product after conjugation according to example 7.5, based on Example 1.1 b).
- Lane D:: Crude product after conjugation according to example 7.6, based on Example 1.3 a).
- Lane E:: Crude product after conjugation according to example 7.5, based on Example 1.1 a).
- Lane F:: Crude product after conjugation according to example 7.2.
- Lane G:: Crude product after conjugation according to example 7.3.
- Lane K:: EPO starting material.

### Figure 6

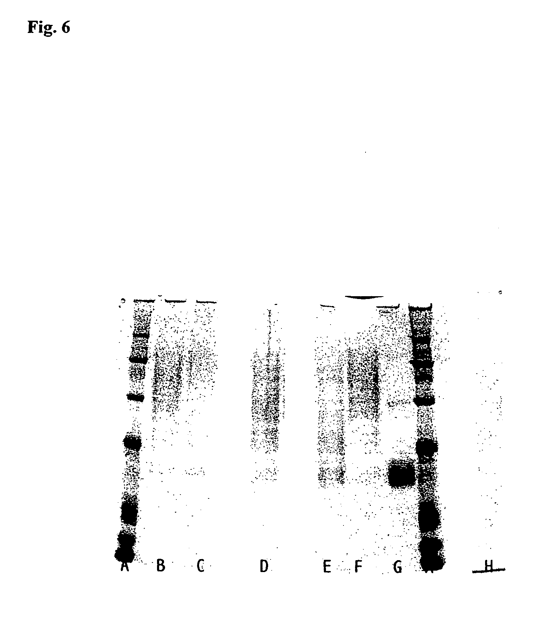

Figure 6 shows an SDS page analysis of HES-EPO conjugates, produced according to examples 7.7, 7.8, 7.9, 7.10, 7.11, and 7.12.
- Lane A:: Protein marker Roti®-Mark PRESTAINED (Carl Roth GmbH+Co, Karlsruhe, D); molecular weights (in kD) of the protein marker from top to bottom: 245, 123, 77, 42, 30, 25.4, and 17.
- Lane B:: Crude product after conjugation according to example 7.11.
- Lane C:: Crude product after conjugation according to example 7.10.
- Lane D:: Crude product after conjugation according to example 7.7.
- Lane E:: Crude product after conjugation according to example 7.8.
- Lane F:: Crude product after conjugation according to example 7.12.
- Lane G:: EPO starting material.
- Lane K:: Crude product after conjugation according to example 7.9.

### Figure 7

SDS-PAGE analyses of EPO-GT-1 subjected to mild acid treatment for 5 min. = lane 2; 10 min. = lane 3; 60 min. = lane 4 and untreated EPO = lane 1; the mobility shift of EPO after removal of N-glycans is shown (**+PNGASE**).

### Figure 8

HPAEC-PAD pattern of oligosaccharides isolated from untreated EPO and from EPO incubated for 5 min., 10 min. and 60 min. under mild acid hydrolysis conditions. Roman numbers **I-V indicate** the elution position of **I** = desialylated diantennary structure, **II** = trisialylated triantennary structures (two isomers), **III** = tetrasialylated tetraantennary structure + 2 N-acetyllactosamine repeats, **IV** = tetrasialylated tetraantennary structure + 1 N-acetyllactosamine repeat; **V** = tetrasialylated tetraantennary structure + without N-acetyllactosamine repeat. The elution area of oligosaccharides structures without, with 1-4 sialic acid is indicated by brackets.

### Figure 9

HPAEC-PAD ofN-linked oligosaccharides after desialylation; the elution position of N-acetylneuraminic acid is shown; numbers 1-9 indicate the elution position of standard oligosaccharides: 1 = diantennary; 2 = triantennary (2-4 isomer), 3 = triantennary (2-6 isomer); 4 = tetraantennary; 5 = triantennary plus 1 repeat; 6 = tetraantennary plus 1 repeat; 7 = triantennary plus 2 repeats; 8 = tetraantennary plus 2 repeats and 9 = tetraantennary plus 3 repeats.

### Figure 10

SDS-PAGE analysis of mild treated and untreated EPO which were subjected to periodate oxidation of sialic acid residues. 1 = periodate oxidized without acid treatment; 2 = periodate oxidized 5 min. acid treatment; 3 = periodate oxidized and acid treatment 10 min.; 4 = periodate oxidized without acid treatment; 5 = BRP EPO standard without periodate and without acid treatment.

### Figure 11

HPAEC-PAD pattern of native oligosaccharides isolated from untreated EPO and from EPO incubated for 5 min and 10 min under mild acid hydrolysis conditions and subsequent periodate treatment. The elution area of oligosaccharides structures without and with 1-4 sialic acid is indicated by brackets 1-5.

### Figure 12

SDS-PAGE analysis of the time course of HES-modification of **EPO-GT-1-A**: 20 µg aliquots of **EPO-GT-1-A** were reacted with hydroxylamine-modified HES derivative **X** for 30 min, 2, 4 and 17 hours. Lane 1 = 30 min reaction time; land 2 = 2 hour reaction time; land 3 = 4 hours reaction time; lane 4 = 17 hours reaction time; lane 5 = **EPO-GT-1-A** without HES-modification. Left figure shows the shift in mobility of **EPO-GT-1-A** with increasing incubation time in the presence of the with hydroxylamine-modified HES derivative (flow rate: 1 ml·min⁻¹) X: Lane 1 = 30 min reaction time; lane 2 = 2 hours reaction time; lane 3 = 4 hours reaction time, land 4 = 17 hours reaction time; lane 5 = **EPO-GT-1-A** with HES modification. The figure on the right shows analysis of the same samples after their treatment with N-glycosidase.

### Figure 13

SDS-PAGE analysis of Q-Sepharose fractions of HES-EPO conjugates. Each 1% of the flow-through and 1% of the fraction eluting at high salt concentrations were concentrated in a Speed Vac concentrator and were loaded onto the gels in sample buffer. EPO protein was stained by Coomassie Blue. A = sample **I; B** = sample **II;** C = sample **III; K** = control EPO-GT-1; **A1, B1,** C1 and **K1** indicated the flow-through fraction; **A2**, **B2**, **C2** and **K2** indicates the fraction eluted with high salt concentration.

### Figure 14a

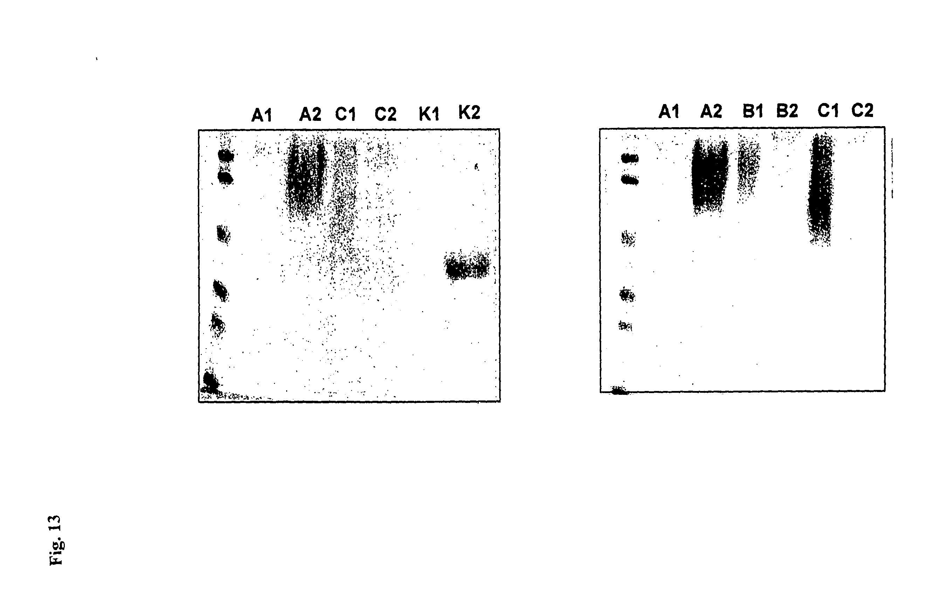

SDS-PAGE analysis of HES-modified EPO sample **A2** (see Fig. **13**), control EPO sample **K2** and **EPO-GT-1-A** EPO preparation were digested in the presence of N-glycosidase in order to remove N-linked oligosaccharides. All EPO samples showed the mobility shift towards low molecular weight forms lacking or containing O-glycan. A lower ratio of the O-glycosylated and nonglycosylated protein band was observed for the HES-modified EPO sample A2 after de-*N*-glycosylation and a diffuse protein band was detected around 30 KDa, presumably representing HES-modification at the sialic acid of O-glycan residue (see arrow marked by an asterisk).

### Figure 14b

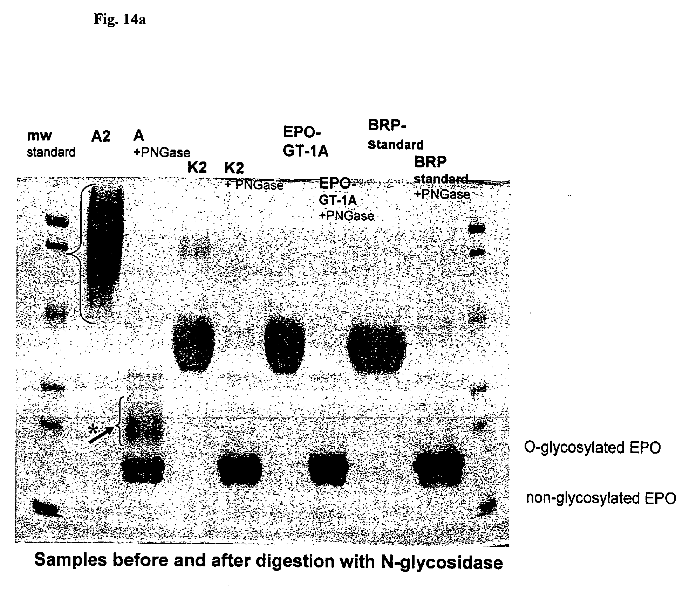

SDS-PAGE analysis after mild hydrolysis of HES-modified EPO sample **A2** (see Fig. **13**), control EPO sample **K2** and **EPO-GT-1A** which were untreated or digested in the presence of N-glycosidase in order to remove N-linked oligosaccharides (**see** **Figure 14a**). Both high molecular weight form of **A2** before and **A** after N.glycosidase treatment (see brackets with and without arrow) disappeared upon acid treatment of the samples. The BRP EPO standard which was run for comparison was not subjected to mild acid treatment.

### Figure 15

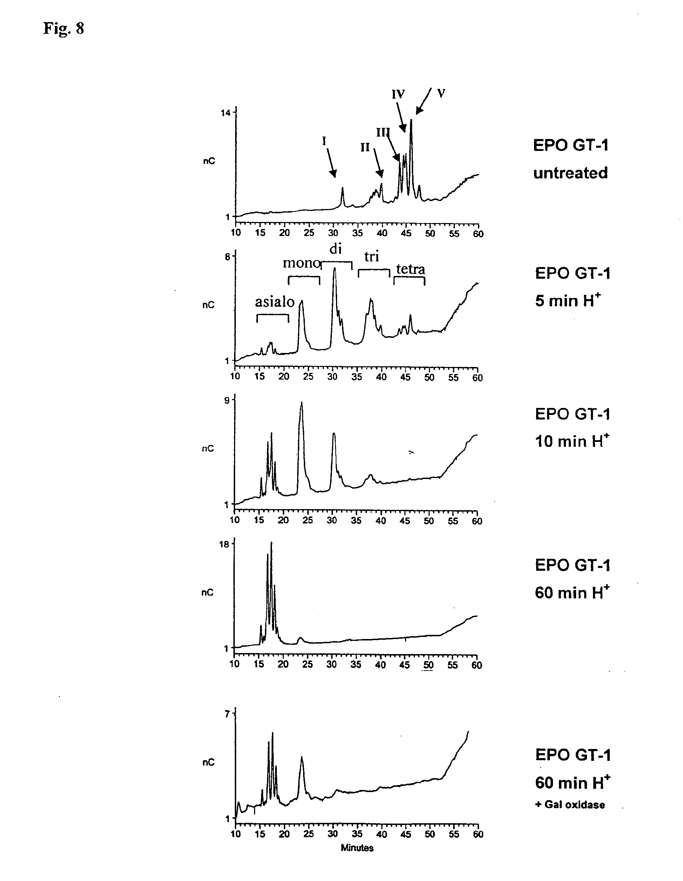

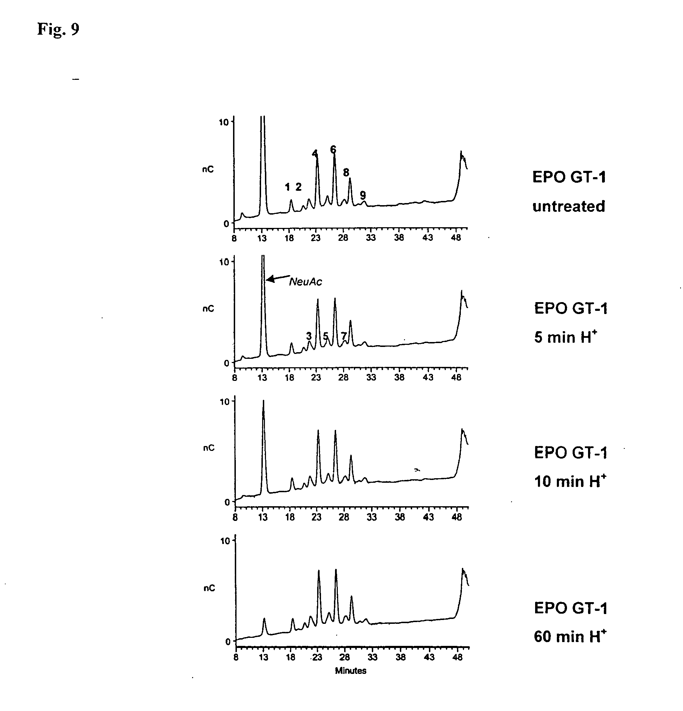

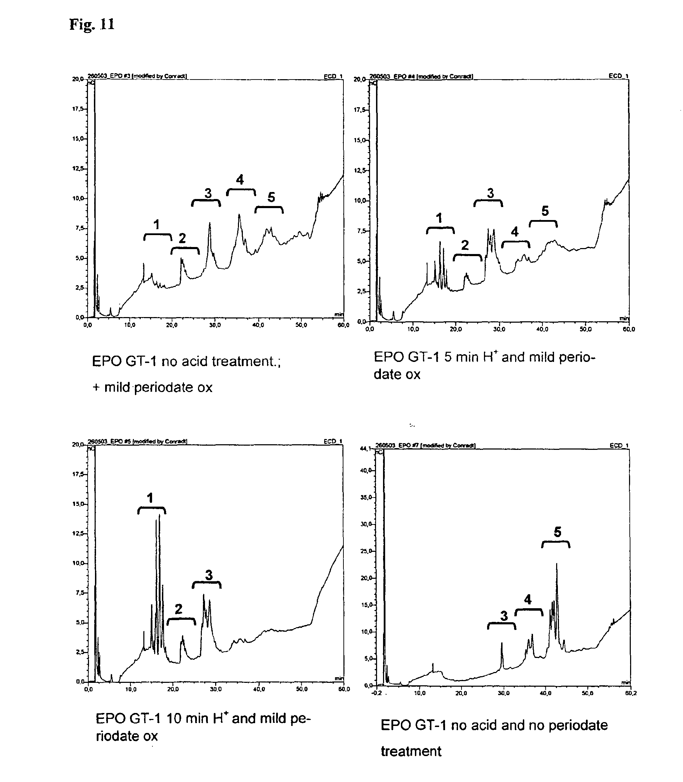

HPAEC-PAD analysis of N-linked oligosaccharide material liberated from HES-modified sample A, from EPO-GT-1-A and from a control EPO sample incubated with unmodified HES (**K**). Roman numbers **I-V indicate** the elution position of **I** = disialylated diantennary structure, **II** = trisialylated triantennary structures (two isomers), **III** = tetrasialylated tetraantennary structure + 2 N-acetyllactosamine repeats, **IV** = tetrasialylated tetraantennary structure + 1 N-acetyllactosamine repeat, **V** = tetrasialylated tetraantennary structure + without N-acetyllactosamine repeat; brackets indicate the elution area of di-, tri- and tetrasialylated N-glycans as reported in the legends of Figs. 8 and 11.

### Figure 16

HPAEC-PAD analysis of N-linked oligosaccharide material liberated from HES-modified sample A, from EPO-GT-lA and from a control EPO sample (**K**) incubated with unmodified HES. The retention times of a mixture of standard oligosaccharides is shown: numbers 1-9 indicate the elution position of standard oligosaccharides: 1 = diantennary; 2 = triantennary (2-4 isomer); 3 = triantennary (2-6 isomer); 4 = tetraantennary; 5 = triantennary plus 1 repeat; 6 = tetraantennary plus 1 repeat; 7 = triantennary plus 2 repeats; 8 = tetraantennary plus 2 repeats and 9 = tetraantennary plus 3 repeats.

### Figures 17 to 23

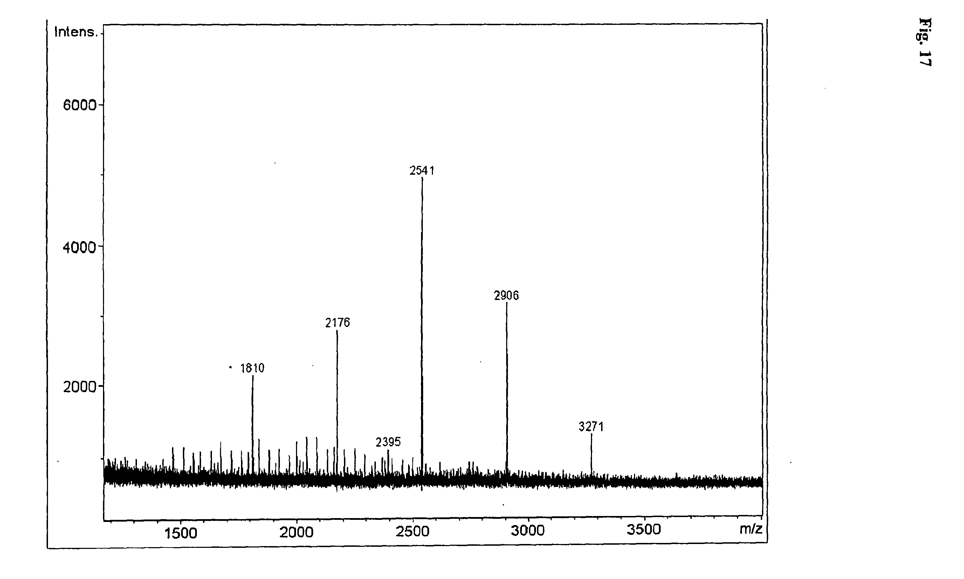

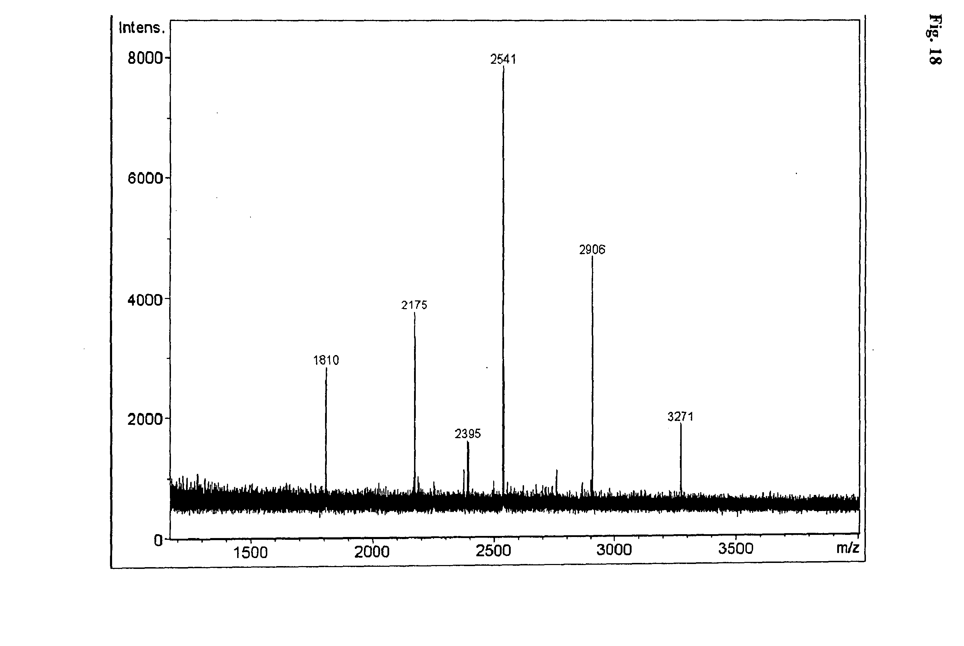

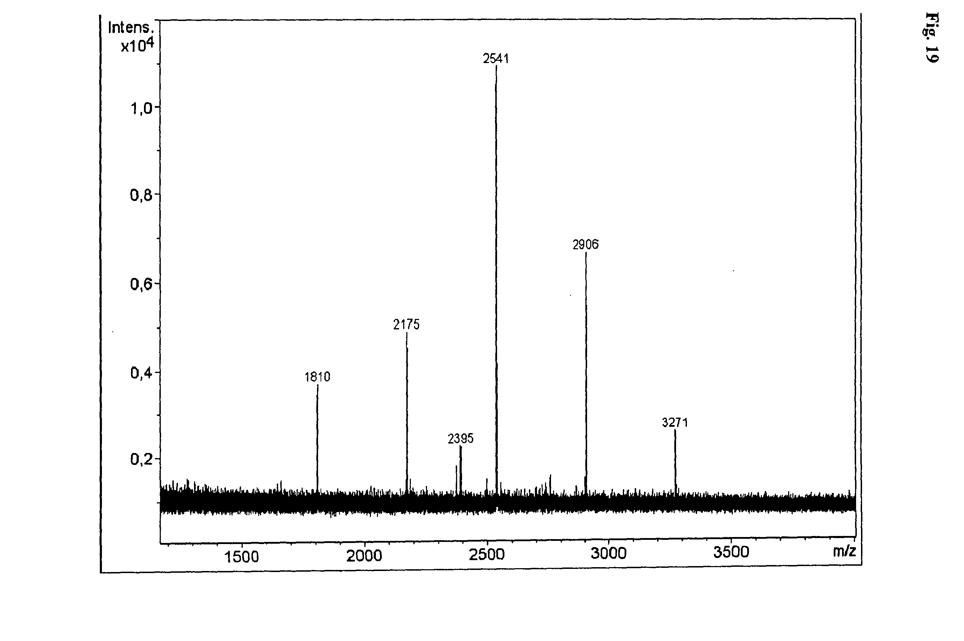

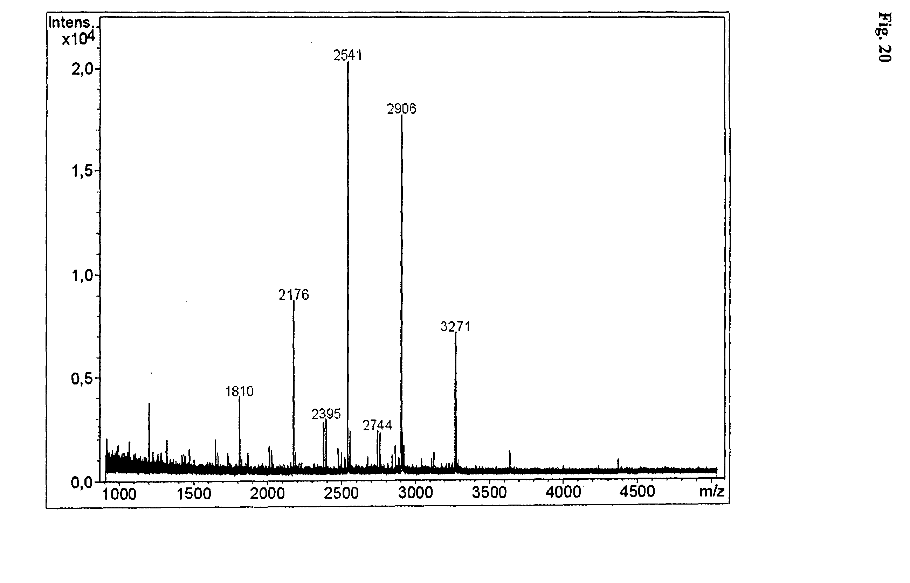

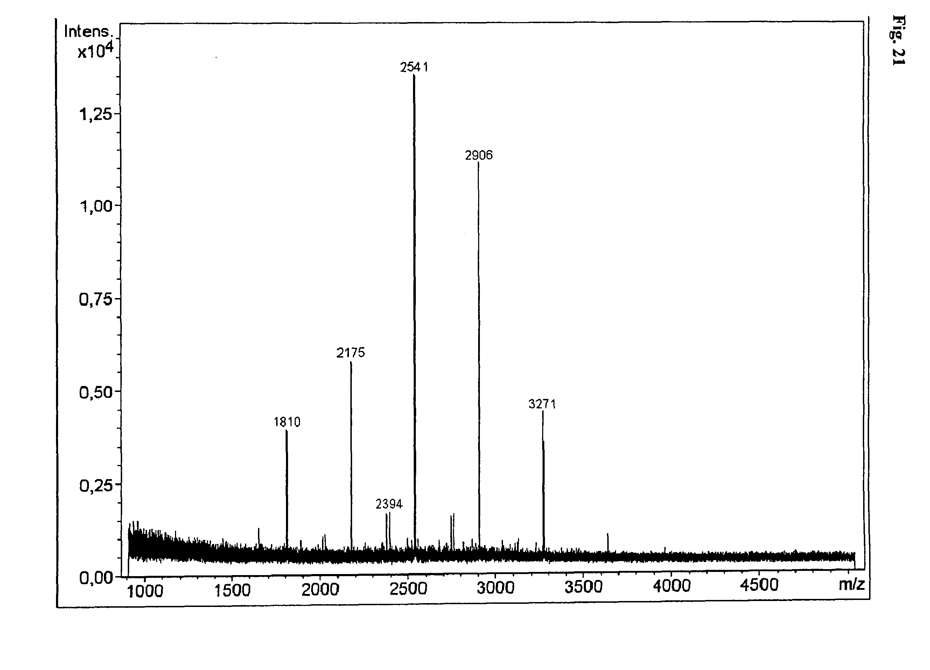

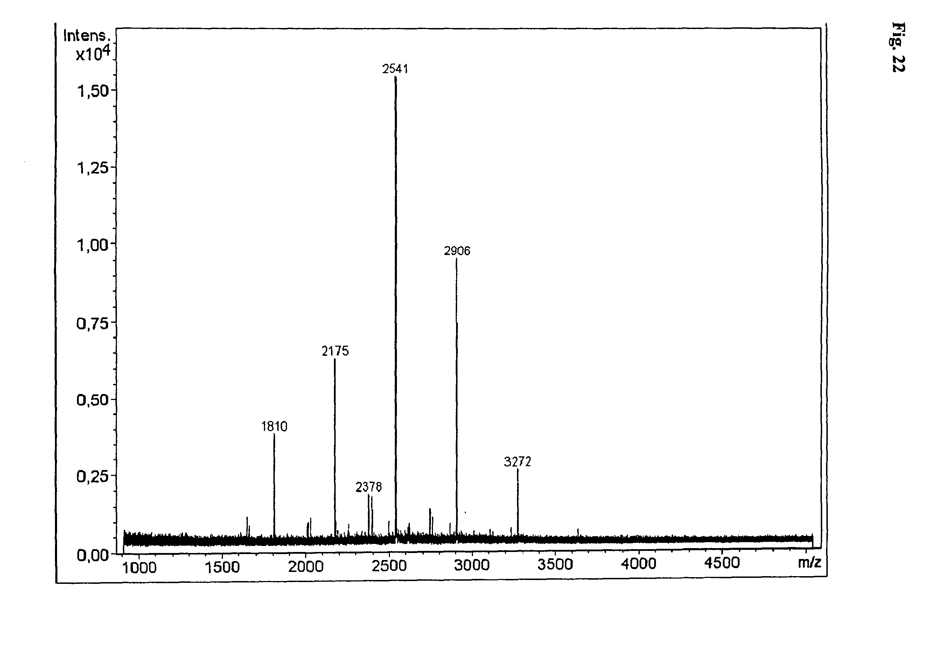

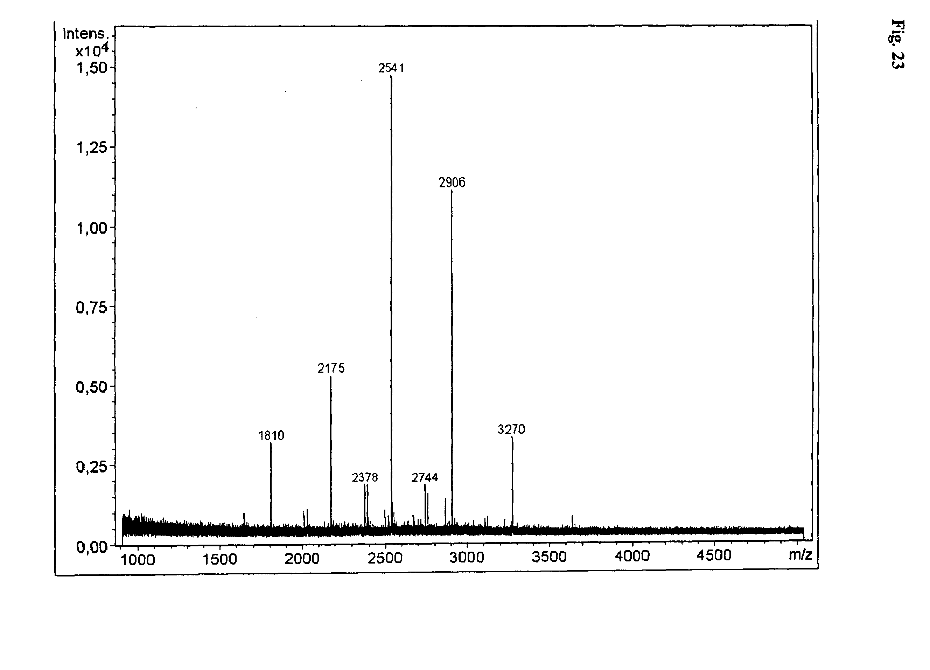

Figures 17 to 23 represent MALDI/TOF mass spectra of the enzymatically liberated and chemically desialylated N-glycans isolated from HES-modified EPO and control EPO preparations. Major signals at *m*/*z* 1809.7, 2174.8, 2539.9, 2905.0 and 3270.1 ([M+Na]⁺) correspond to di- to tetraantennary complex-type N-glycan structures with no, one or two N-acetyllactosamine repeats accompanied by weak signals due to loss of fucose or galactose which are due to acid hydrolysis conditions employed for the desialylation of samples for MS analysis.

### Figure 17

MALDI/TOF spectrum: desialylated oligosaccharides of HES-modified EPO A2.

### Figure 18

MALDI/TOF spectrum: desialylated oligosaccharides of EPO GT-1-A.

### Figure 19

MALDI/TOF spectrum: desialylated oligosaccharides of EPO K2.

### Figure 20

MALDI/TOF spectrum: desialylated oligosaccharides of EPO-GT-1.

### Figure 21

MALDI/TOF spectrum: desialylated oligosaccharides of EPO-GT-1 subjected to acid hydrolysis for 5 min.

### Figure 22

MALDI/TOF spectrum: desialylated oligosaccharides of EPO-GT-1 subjected to acid hydrolysis for 10 min.

### Figure 23

MALDI/TOF spectrum: desialylated oligosaccharides of EPO-GT-1 subjected to acid hydrolysis for 60 min.

### Figure 24

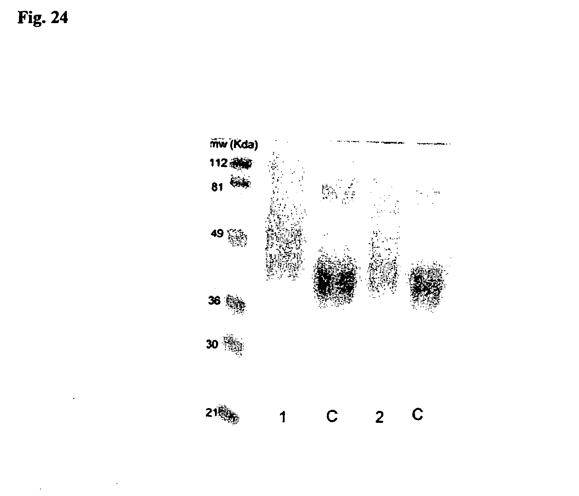

Figure 24 shows an SDS page analysis of two HES-EPO conjugates
- mw:: marker
- Lane 1:: HES-EPO produced according to example protocol 8: EPO is conjugated to hydrazido-HES 12KD L
- Lane 2:: HES-EPO produced according to example protocol 9 : EPO is conjugated to hydroxylamino HES 12 KD K
- C:: control (unconjugated EPO); the upper band represents EPO dimer

### Figure 25

Figure 2 demonstrates that the HES is conjugated to a carbohydrate moiety of a carbohydrate side chain by showing a digestion of HAS modified EPO forms with polyppetide N-glycosidase
- Lane 1:: HES-EPO produced according to example protocol 8 after digestion with N-glycosidase
- Lane 2:: HES-EPO produced according to example protocol 9 after digestion with N-glycosidase
- Lane 3:: BRP EPO standard
- Lane 4:: BRP EPO standard after digestion with N-glycosidase
- mw:: marker (Bio-Rad SDS-PAGE Standards Low range Catalog No 161-0305, Bio-Rad Laboratories, Hercules, CA, USA)

### Examples

### Example 1: Formation of hydroxyethyl starch derivatives by reductive amination of the non-oxidised reducing end

### Example 1.1 Reaction of hydroxyethyl starch with 1,3-diamino-2-hydroxy propane

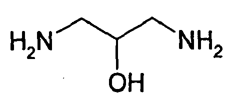

a) To a solution of 200 mg hydroxyethyl starch (HES18/0.4 (MW = 18,000 D, DS=0.4)) in 5 ml water, 0.83 mmol 1,3-diamino-2-hydroxy propane (Sigma Aldrich, Taufkirchen, D) and 50 mg sodium cyanoborohydrate NaCNBH₃ were added. The resulting mixture was incubated at 80 °C for 17 h. The reaction mixture was added to 160 ml of a cold 1:1 mixture of acetone and ethanol (v/v). The precipitate was collected by centrifugation and dialysed for 4 d against water (SnakeSkin dialysis tubing, 3.5 KD cut off, Perbio Science Deutschland GmbH, Bonn, D), and lyophilized.
b) Incubation of the mixture resulting from adding 0.83 mmol 1,3-diamino-2-hydroxy propane and 50 mg sodium cyanoborohydrate NaCNBH₃ to the solution of 200 mg hydroxyethyl starch was also possible and carried out at 25 °C for 3 d.

### Example 1.2 Reaction of hydroxyethyl starch with 1,2-dihydroxy-3-amino propane

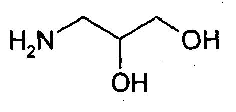

a) To a solution of 200 mg hydroxyethyl starch (HES18/0.4 (MW = 18,000 D, DS=0.4)) in 5 ml water, 0.83 mmol 1,2-dihydroxy-3-amino propane (Sigma Aldrich, Taufkirchen, D) and 50 mg sodium cyanoborohydrate NaCNBH₃ were added. The resulting mixture was incubated at 80 °C for 17 h. The reaction mixture was added to 160 ml of a cold 1:1 mixture of acetone and ethanol (v/v). The precipitate was collected by centrifugation and dialysed for 4 d against water (SnakeSkin dialysis tubing, 3.5 KD cut off, Perbio Science Deutschland GmbH, Bonn, D), and lyophilized.
b) Incubation of the mixture resulting from adding 0.83 mmol 1,2-dihydroxy-3-amino propane and 50 mg sodium cyanoborohydrate NaCNBH₃ to the solution of 200 mg hydroxyethyl starch was also possible and carried out at 25 °C for 3 d.

The reaction of 1,2-dihydroxy-3-amino propane with HES was confirmed indirectly by quantification of formaldehyde, resulting from the oxidative cleavage of the 1,2-diole in the reaction product by periodate as described by G. Avigad, Anal. Biochem. 134 (1983) 449-504.

### Example 1.3 Reaction of hydroxyethyl starch with 1,4-diamino butane

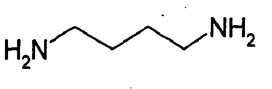

a) To a solution of 200 mg hydroxyethyl starch (HES18/0.4 (MW = 18,000 D, DS=0.4)) in 5 ml water, 0.83 mmol 1,4-diamino butane (Sigma Aldrich, Taufkirchen, D) and 50 mg sodium cyanoborohydrate NaCNBH₃ were added. The resulting mixture was incubated at 80 °C for 17 h. The reaction mixture was added to 160 ml of a cold 1:1 mixture of acetone and ethanol (v/v). The precipitate was collected by centrifugation and dialysed for 4 d against water (SnakeSkin dialysis tubing, 3.5 KD cut off, Perbio Science Deutschland GmbH, Bonn, D), and lyophilized.
b) Incubation of the mixture resulting from adding 0.83 mmol 1,4-diamino butane and 50 mg sodium cyanoborohydrate NaCNBH₃ to the solution of 200 mg hydroxyethyl starch was also possible and carried out at 25 °C for 3 d.

### Example 1.4 Reaction of hydroxyethyl starch with 1-mercapto-2-amino ethane

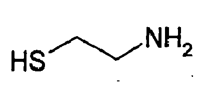

a) To a solution of 200 mg hydroxyethyl starch (HES18/0.4 (MW = 18,000 D, DS=0.4)) in 5 ml water, 0.83 mmol 1-mercapto-2-amino ethane (Sigma Aldrich, Taufkirchen, D) and 50 mg sodium cyanoborohydrate NaCNBH₃ were added. The resulting mixture was incubated at 80 °C for 17 h. The reaction mixture was added to 160 ml of a cold 1:1 mixture of acetone and ethanol (v/v). The precipitate was collected by centrifugation and dialysed for 4 d against water (SnakeSkin dialysis tubing, 3.5 KD cut off, Perbio Science Deutschland GmbH, Bonn, D), and lyophilized.
b) Incubation of the mixture resulting from adding 0.83 mmol 1-mercapto-2-amino ethane and 50 mg sodium cyanoborohydrate NaCNBH₃ to the solution of 200 mg hydroxyethyl starch was also possible and carried out at 25 °C for 3 d.

### Example 2: Formation of hydroxyethyl starch derivatives by conjugation with the non-oxidised reducing end

### Example 2.1: Reaction of hydroxyethyl starch with carbohydrazide

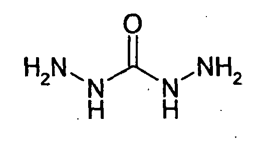

0.96 g of HES18/0.4 (MW = 18,000 D, DS=0.4) were dissolved in 8 ml aqueous 0.1 M sodium acetate buffer, pH 5.2, and 8 mmol carbohydrazide (Sigma Aldrich, Taufkirchen, D) were added. After stirring for 18 h at 25 °C, the reaction mixture was added to 160 ml of a cold 1:1 mixture of acetone and ethanol (v/v). The precipitated product was collected by centrifugation, re-dissolved in 40 ml water, and dialysed for 3 d against water (SnakeSkin dialysis tubing, 3.5 KD cut off, Perbio Science Deutschland GmbH, Bonn, D), and lyophilized.

### Example 2.2: Reaction of hydroxyethyl starch with adepic dihydrazide

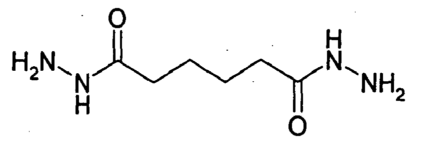

0.96 g of HES 18/0.4 (MW = 18,000 D, DS=0.4) were dissolved in 8 ml aqueous 0.1 M sodium acetate buffer, pH 5.2, and 8 mmol adepic dihydrazide (Lancaster Synthesis, Frankfurt/Main, D) were added. After stirring for 18 h at 25 °C, the reaction mixture was added to 160 ml of a cold 1:1 mixture of acetone and ethanol (v/v). The precipitated product was collected by centrifugation, re-dissolved in 40 ml water, and dialysed for 3 d against water (SnakeSkin dialysis tubing, 3.5 KD cut off, Perbio Science Deutschland GmbH, Bonn, D), and lyophilized.

### Example 2.3: Reaction of hydroxyethyl starch with 1,4-phenylene-bis-3-thiosemicarbazide

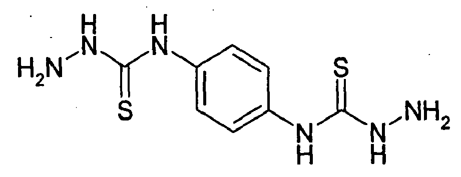

0.96 g of HES18/0.4 (MW = 18,000 D, DS=0.4) were dissolved in 8 ml aqueous 0.1 M sodium acetate buffer, pH 5.2, and 8 mmol 1,4-phenylene-bis-3-thiosemicarbazide (Lancaster Synthesis, Frankfurt/Main, D) were added. After stirring for 18 h at 25 °C, 8 ml water was added to the reaction mixture, and the suspension was centrifugated for 15 min at 4,500 rpm. The clear supernatant was decanted and subsequently added to 160 ml of a cold 1:1 mixture of acetone and ethanol (v/v). The precipitated product was collected by centrifugation, re-dissolved in 40 ml water, and centrifugated for 15 min at 4,500 rpm. The clear supernatant was dialysed for 3 d against water (SnakeSkin dialysis tubing, 3.5 KD cut off, Perbio Science Deutschland GmbH, Bonn, D), and lyophilized.

### Example 2.4: Reaction of hydroxyethyl starch with O-[2-(2-aminooxyethoxy)-ethyl]-hydroxyl amine

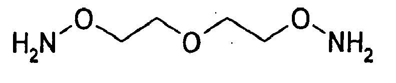

O-[2-(2-aminooxy-ethoxy)-ethyl]-hydroxyl amine was synthesized as described in Boturyn et al. Tetrahedron 53 (1997) p. 5485-5492 in 2 steps from commercially available materials.

0.96 g of HES 18/0.4 (MW = 18,000 D, DS=0.4) were dissolved in 8 ml aqueous 0.1 M sodium acetate buffer, pH 5.2, and 8 mmol O-[2-(2-aminooxy-ethoxy)-ethyl]-hydroxyl amine were added. After stirring for 18 h at 25 °C, the reaction mixture was added to 160 ml of a cold 1:1 mixture of acetone and ethanol (v/v). The precipitated product was collected by centrifugation, re-dissolved in 40 ml water, and dialysed for 3 d against water (SnakeSkin dialysis tubing, 3.5 KD cut off, Perbio Science Deutschland GmbH, Bonn, D), and lyophilized.

### Example 3 Formation of hydroxyethyl starch derivatives by reaction with the oxidised reducing end

### Example 3.1 Reaction of hydroxyethyl starch with carbohydrazide

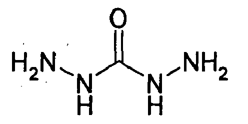

0.12 mmol Oxo-HES 10/0.4 (MW = 10,000 D, DS=0.4, prepared according to DE 196 28 705 A1) were dissolved in 3 ml absolute dimethyl sulfoxide (DMSO) and added dropwise under nitrogen to a mixture of 15 mmol of carbohydrazide (Sigma Aldrich, Taufkirchen, D) in 15 ml DMSO. After stirring for 88 h at 65 °C, the reaction mixture was added to 160 ml of a cold 1:1 mixture of acetone and ethanol (v/v). The precipitate was collected by centrifugation and was dialysed for 4 d against water (SnakeSkin dialysis tubing, 3.5 KD cut off, Perbio Science Deutschland GmbH, Bonn, D) and lyophilized.

### Example 3.2 Reaction of hydroxyethyl starch with 1,4-phenylene-bis-3-thiosemicarbazide

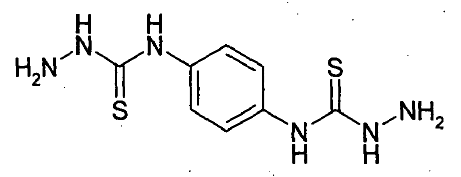

0.12 mmol Oxo-HES 10/0.4 (MW = 10,000 D, DS=0.4, prepared according to DE 196 28 705 A1) were dissolved in 3 ml absolute dimethyl sulfoxide (DMSO) and added dropwise under nitrogen to a mixture of 15 mmol of 1,4-phenylene-bis-3-thiosemicarbazide (Lancaster Synthesis, Frankfurt/Main, D) in 15 ml DMSO. After stirring for 88 h at 65 °C, the reaction mixture was added to 160 ml of a cold 1:1 mixture of acetone and ethanol (v/v). The precipitate was collected by centrifugation and was dialysed for 4 d against water (SnakeSkin dialysis tubing, 3.5 KD cut off, Perbio Science Deutschland GmbH, Bonn, D) and lyophilized.

### Example 3.3 Reaction of hydroxyethyl starch with hydrazine H₂N-NH₂

1,44 g (0.12 mmol) of Oxo-HES 10/0.4 (MW = 10,000 D, DS=0.4, prepared according to DE 196 28 705 A1) were dissolved in 3 ml absolute dimethyl sulfoxide (DMSO) and were added dropwise under nitrogen to a mixture of 0.47 ml (15 mmol) hydrazine in 15 ml DMSO. After stirring for 19 h at 40°C the reaction mixture was added to 160 ml of a 1:1 mixture of ethanol and acetone (v/v). The precipitated product was collected by centrifugation, redissolved in 40 mL of water and dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized.

### Example 3.4 Reaction of hydroxyethyl starch with hydroxylamine

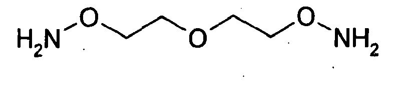

O-[2-(2-aminooxy-ethoxy)-ethyl]-hydroxylamine was synthesized as described by Boturyn et al in 2 steps from commercially available materials (Boturyn, Boudali, Constant, Defrancq, Lhomme, 1997, Tetrahedron, 53, 5485).

1.44 g (0.12 mmol) of Oxo-HES 10/0.4 (MW = 10,000 D, DS=0.4, prepared according to DE 196 28 705 A1) were dissolved in 3 ml absolute dimethyl sulfoxide (DMSO) and were added dropwise under nitrogen to a mixture of 2.04 g (15 mmol) O-[2-(2-aminooxy-ethoxy)-ethyl]-hydroxylamine in 15 ml DMSO. After stirring for 48 h at 65°C the reaction mixture was added to 160 ml of a 1:1 mixture of ethanol and acetone (v/v). The precipitated product was collected by centrifugation, redissolved in 40 ml of water and dialysed for 4 days against water (SnakeSkin dialysis tubing, 3.5 KD cut off, Perbio Science Deutschland GmbH, Bonn, Germany) and lyophilized.

### Example 3.5 Reaction of hydroxyethyl starch with adepic dihydrazide

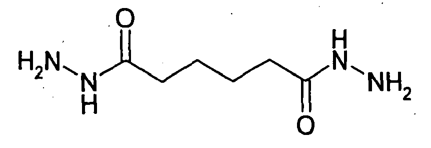

1.74 g (15 mmol) adepic dihydrazide (Lancaster Synthesis, Frankfurt/Main, D) were dissolved in 20 ml absolute dimethyl sulfoxide (DMSO) at 65°C and 1.44 g (0,12 mmol) of Oxo-HES 10/0.4 (MW = 10,000 D, DS=0.4, prepared according to DE 196 28 705 A1), dissolved in 3 ml absolute DMSO were added dropwise under nitrogen. After stirring for 68 h at 60°C the reaction mixture was added to 200 ml of water The solution containing the reaction product was dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized.

### Example 3.6 Reaction of hydroxyethyl starch with 1,4-diamino butane

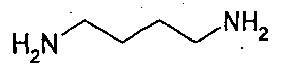

1.44 g (0.12 mmol) of Oxo-HES 10/0.4 (MW = 10,000 D, DS=0.4, prepared according to DE 196 28 705 A1) were dissolved in 3 ml dry dimethyl sulfoxide (DMSO) and were added dropwise under nitrogen to a mixture of 1.51 ml (15 mmol) 1,4-diaminobutane (Sigma Aldrich, Taufkirchen, D) in 15 ml DMSO. After stirring for 19 h at 40°C the reaction mixture was added to 160 ml of a 1:1 mixture of ethanol and acetone (v/v). The precipitate Amino-HES10KD/0.4 was collected by centrifugation, redissolved in 40 ml of water and dialysed for 4 days against water (SnakeSkin dialysis tubing, 3.5 KD cut off, Perbio Science Deutschland GmbH, Bonn, Germany) and lyophilized.

### Example 4 Oxidation of erythropoietin

Oxidized erythropoietin was produced as described in Example 8. As oxidised erythropoietin, EPO-GT-1-A as described in Example 8.11(c) was used (EPO-GT-1 without acid hydroylsis, treated with mild periodate oxidation).

### Example 5: Conjugation of hydroxyethyl starch derivatives with oxidized erythropoietin of example 4

### Example 5.1 Reaction of oxidized erythropoietin with the reaction product of example 2.1

Oxidized EPO (1.055 µg/µl) in 20 mM PBS buffer was adjusted to pH 5.3 with 5 M sodium acetate buffer, pH 5.2. To 19 µl of the EPO solution, 18 µl of a solution of the HES derivate as produced according to example 2.1 (MW 18 kD; 18.7 µg/µl in 0.1 M sodium acetate buffer, pH 5.2) was added, and the mixture was incubated for 16 h at 25 °C. After lyophilisation, the crude product was analyzed by SDS-Page with NuPAGE 10% Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig. 1. A successful conjugation is indicated by the migration of the protein band to higher molecular weights. The increased bandwidth is due to the molecular weight distribution of the HES derivatives used and the number of HES derivatives linked to the protein.

### Example 5.2 Reaction of oxidized erythropoietin with the reaction product of example 2.3

Oxidized EPO (1.055 µg/µl) in 20 mM PBS buffer was adjusted to pH 5.3 with 5 M sodium acetate buffer, pH 5.2. To 19 µl of the EPO solution, 18 µl of a solution of the HES derivate as produced according to example 2.3 (MW 18 kD; 18.7 µg/µl in 0.1 M sodium acetate buffer, pH 5.2) was added, and the mixture was incubated for 16 h at 25 °C. After lyophilisation, the crude product was analyzed by SDS-Page with NuPAGE 10% Bis-Tris Gels/MOPS buffer (Invitrogen, Carlsbad, CA, USA) as described in the instructions given by Invitrogen.

### Example 5.3 Reaction of oxidized erythropoietin with the reaction product of example 2.4

Oxidized EPO (1.055 µg/µl) in 20 mM PBS buffer was adjusted to pH 5.3 with 5 M sodium acetate buffer, pH 5.2. To 19 µl of the EPO solution, 18 µl of a solution of the HES derivate as produced according to example 2.4 (MW 18 kD; 18.7 µg/µl in 0.1 M sodium acetate buffer, pH 5.2) was added, and the mixture was incubated for 16 h at 25 °C. After lyophilisation, the crude product was analyzed by SDS-Page with NuPAGE 10% Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig. 2. A successful conjugation is indicated by the migration of the protein band to higher molecular weights. The increased bandwidth is due to the molecular weight distribution of the HES derivatives used and the number of HES derivatives linked to the protein.

### Example 5.4 Reaction of oxidized erythropoietin with the reaction product of example 3.1

Oxidized EPO (1.055 µg/µl) in 20 mM PBS buffer was adjusted to pH 5.3 with 5 M sodium acetate buffer, pH 5.2. To 19 µl of the EPO solution, 18 µl of a solution of the HES derivate as produced according to example 3.1 (MW 10 kD; 18.7 µg/µl in 0.1 M sodium acetate buffer, pH 5.2) was added, and the mixture was incubated for 16 h at 25 °C. After lyophilisation, the crude product was analyzed by SDS-Page with NuPAGE 10% Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig. 3. A successful conjugation is indicated by the migration of the protein band to higher molecular weights. The increased bandwidth is due to the molecular weight distribution of the HES derivatives used and the number of HES derivatives linked to the protein.

### Example 5.5 Reaction of oxidized erythropoietin with the reaction product of example 3.2

Oxidized EPO (1.055 µg/µl) in 20 mM PBS buffer was adjusted to pH 5.3 with 5 M. sodium acetate buffer, pH 5.2. To 19 µl of the EPO solution, 18 µl of a solution of the HES derivate as produced according to example 3.1 (MW 10 kD; 18.7 µg/µl in 0.1 M sodium acetate buffer, pH 5.2) was added, and the mixture was incubated for 16 h at 25 °C. After lyophilisation, the crude product was analyzed by SDS-Page with NuPAGE 10% Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig. 3. A successful conjugation is indicated by the migration of the protein band to higher molecular weights. The increased bandwidth is due to the molecular weight distribution of the HES derivatives used and the number of HES derivatives linked to the protein.

### Example 6 Formation of Thio-EPO by reduction of erythropoietin

**241.5** µg erythropoietin (EPO-GT-1, see Example 8) in 500 µl of a 0.1 M sodium borate buffer, 5 mM EDTA, 10 mM DTT (Lancaster, Morcambe, UK), pH 8.3, were incubated for 1 h at 37 °C. The DTT was removed by centrifugal filtration with a VIVASPIN 0.5 ml concentrator, 10 KD MWCO (VIVASCIENCE, Hannover, D) at 13,000 rpm, subsequent washing 3 times with the borate buffer and twice with a phosphate buffer (0.1 M, 9.15 M NaCl, 50 mM EDTA, pH 7.2). The gel is stained with Roti-Blue Coomassie staining reagent (Roth, Karlsruhe, D) overnight.

### Example 7: Conjugation of hydroxyethyl starch derivatives with thio-erythropoietin using a crosslinking compound

In each of the following examples, N-(alpha-maleimidoacetoxy) succinimide ester (AMAS) 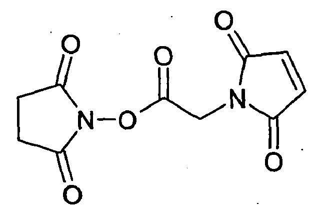 was used as crosslinking compound.

### Example 7.1 Reaction of thio-erythropoietin with the reaction product of example 2.1 and the crosslinking compound

To 50 nmol HES derivate as produced according to example 2.1 and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40 °C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig. 4. A successful conjugation is indicated by the migration of the protein band to higher molecular weights. The increased bandwidth is due to the molecular weight distribution of the HES derivatives used and the number of HES derivatives linked to the protein.

### Example 7.2 Reaction of thio-erythropoietin with the reaction product of example 2.2 and the crosslinking compound

To 50 nmol_HES derivate as produced according to example 2.2 and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40 °C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig 5. A successful conjugation is indicated by the migration of the protein band to higher molecular weights. The increased bandwidth is due to the molecular weight distribution of the HES derivatives used and the number of HES derivatives linked to the protein.

### Example 7.3 Reaction of thio-erythropoietin with the reaction product of example 2.3 and the crosslinking compound

To 50 nmol HES derivate as produced according to example 2.3 and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40°C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig. 5. A successful conjugation is indicated by the migration of the protein band to higher molecular weights. The increased bandwidth is due to the molecular weight distribution of the HES derivatives used and the number of HES derivatives linked to the protein.

### Example 7.4 Reaction of thio-erythropoietin with the reaction product of example 2.4 and the crosslinking compound

To 50 nmol HES derivate as produced according to example 2.4 and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40 °C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig 4. A successful conjugation is indicated by the migration of the protein band to higher molecular weights. The increased bandwidth is due to the molecular weight distribution of the HES derivatives used and the number of HES derivatives linked to the protein.

### Example 7.5 Reaction of thio-erythropoietin with the reaction product of example 1.1 and the crosslinking compound

To 50 nmol HES derivate as produced according to example 1.1, at incubation conditions of 80 °C and 17 h as well as of 25 °C and 3 d, and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40 °C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig. 5. A successful conjugation is indicated by the migration of the protein band to higher molecular weights. The increased bandwidth is due to the molecular weight distribution of the HES derivatives used and the number of HES derivatives linked to the protein.

### Example 7.6 Reaction of thio-erythropoietin with the reaction product of example 1.3 and the crosslinking compound

To 50 nmol HES derivate as produced according to example 1.3, at incubation conditions of 80 °C and 17 h as well as of 25 °C and 3 d, and dissolved in 200 µl of a 0.1 M sodium phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO were added. The clear solution was incubated for 80 min at 25 °C and 20 min at 40°C. Remaining AMAS was removed by centrifugal filtration with a VIVASPIN 0.5 ml concentrator, 5 KD MWCO (VIVASCIENCE, Hannover, D) at 13,000 rpm, washing 4 times and 30 min with the phosphate buffer.

To the residual solution, 15 µg of ThioEPO as produced according to example 5 (1 µg/µl in phosphate buffer) were added, and the mixture was incubated for 16 h at 25 °C. After lyophilisation, the crude product was analysed by SDS-Page with NuPAGE 10% Bis-Tris Gels/MOPS buffer (Invitrogen, Carlsbad, USA) as described in the instructions given by Invitrogen. The gel is stained with Roti-Blue Coomassie staining reagent (Roth, Karlsruhe, D) overnight.

The experimental result is shown in Fig 5. A successful conjugation is indicated by the migration of the protein band to higher molecular weights. The increased bandwidth is due to the molecular weight distribution of the HES derivatives used and the number of HES derivatives linked to the protein.

### Example 7.7 Reaction of thio-erythropoietin with the reaction product of example 3.1 and the crosslinking compound

To 50 nmol HES derivate, produced according to Example 3.1 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/µl in phosphate buffer) were added, and the mixture was incubated for 16 h at 25 °C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 7.8 Reaction of thio-erythropoietin with the reaction product of example 3.2 and the crosslinking compound

To 50 nmol HES derivate, produced according to Example 3.2 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/µl in phosphate buffer) were added, and the mixture was incubated for 16 h at 25 °C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 7.9 Reaction of thio-erythropoietin with the reaction product of example 3.3 and the crosslinking compound

To 50 nmol HES derivate, produced according to Example 3.3 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/µl in phosphate buffer) were added, and the mixture was incubated for 16 h at 25 °C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 7.10 Reaction of thio-erythropoietin with the reaction product of example 3.4 and the crosslinking compound

To 50 nmol HES derivate, produced according to Example 3.4 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/µl in phosphate buffer) were added, and the mixture was incubated for 16 h at 25°C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 7.11 Reaction of thio-erythropoietin with the reaction product of example 3.5 and the crosslinking compound

To 50 nmol HES derivate, produced according to Example 3.5 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/pl in phosphate buffer) were added, and the mixture was incubated for 16 h at 25 °C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 7.12 Reaction of thio-erythropoietin with the reaction product of example 3.6 and the crosslinking compound

To 50 nmol HES derivate, produced according to Example 36 and dissolved in 200 µl phosphate buffer (0.1 M, 9.15 M NaCl, 50 mM EDTA, pH 7.2), 10 µl of a solution of 2.5 µmol AMAS (Sigma Aldrich, Taufkirchen, D) in DMSO was added, and the clear solution was incubated for 80 min at 25 °C and 20 min at 40 °C. The AMAS was removed by centrifugal filtration with a VIVASPIN 0.5 ml concentrator, 5 KD MWCO (VIVASCIENCE, Hannover, Germany) at 13,000 rpm and washing 4 times for 30 min with the phosphate buffer.

To the residual solution, 15 µg Thio-EPO (1 µg/µl in phosphate buffer) were added, and the mixture was incubated for 16 h at 25 °C. After lyophilisation, the crude product was analysed by SDS-Page with NuPAGE 10 % Bis-Tris Gels/MOPS buffer (Invitrogen, Carlsbad, CA, USA) as described in the instructions given by Invitrogen. The gel is stained with Roti-Blue Coomassie staining reagent (Roth, Karlsruhe, D) overnight.

The experimental result is shown in Fig 6. A successful conjugation is indicated by the migration of the protein band to higher molecular weights. The increased bandwidth is due to the molecular weight distribution of the HES derivatives used and the number of HES derivatives linked to the protein.

### Example 8 Preparative production of HES-EPO conjugates

### Summary

HES-EPO conjugates were synthesized by coupling of HES derivatives (average mw of 18,000 Dalton; hydroxyethyl substitution degree of 0.4) to the partially (mild periodate) oxidized sialic acid residues on the oligosaccharide chains of recombinant human EPO. Based on carbohydrate structural analysis the modifications introduced did not affect the structural integrity of the core oligosaccharide chains since MALDI/TOF-MS of the mild acid treated HES-modified glycans revealed intact neutral N-acetyllactosamine-type chains which were indistinguishable from those observed in unmodified EPO product. The results obtained indicate that at least 3 modified HES-residues are attached per EPO molecule in the case of the EPO preparation which was subjected to modification without prior partial sialic acid removal. An EPO variant lacking about 50% of the sialic acid residues of the former protein showed a similar apparent high molecular weight mobility in SDS-PAGE (60-110 KDa vs 40 KDa for the BRP EPO standard). The HES modified EPO is stable under standard ion-exchange chromatography conditions at room temperature at pH 3-10.

The EPO-bioassay in the normocythaemic mouse system indicates that the HES-modified EPO has 2.5-3.0 fold higher specific activity (IU/mg) in this assay when compared to the International BRP EPO reference standard based on protein determination using the UV absorption value from the European Pharmacopeia and an RP-HPLC EPO protein determination method calibrated against the BRP EPO standard preparation.

### Example 8.1 Materials and methods

### (a) Liberation of N-linked oligosaccharides by digestion with N-glycosidase

Samples were incubated with 25 units (according to manufacturer's specification, Roche Diagnostics, Germany) of recombinant PNGase F over night at 37°C. Complete digestion was monitored by the specific mobility shift of the protein in SDS-PAGE. The released N-glycans were separated from the polypeptide by addition of 3 volumes of cold 100% ethanol and incubation at -20°C for at least 2 hours (Schroeter S et al., 1999). The precipitated protein was removed by centrifugation for 10 minutes at 4°C at 13000 rpm. The pellet was then subjected to two additional washes with 500 µl of ice-cold 75% ethanol. The oligosaccharides in the pooled supernatants were dried in a vacuum centrifuge (Speed Vac concentrator, Savant Instruments Inc., USA). The glycan samples were desalted using Hypercarb cartridges (25 mg or 100 mg of HyperCarb) as follows prior to use: the columns were washed with 3 x 500 µl of 80% acetonitrile (v/v) in 0.1% TFA followed by washes with 3 x 500 µl of water. The samples were diluted with water to a final volume of 300 *µ*l - 600 *µ*l before loading onto the cartridge which then was rigorously washed with water. Oligosaccharides were eluted with 1.2 ml (25 mg cartridges; 1.8 ml in the case of 100 mg cartridges) 25% acetonitrile in water containing 0.1% trifluoroacetic acid (v/v).The eluted oligosaccharides were neutralized with 2 M NH₄OH and were dried in a Speed Vac concentrator. In some cases desalting of N-glycosidase released oligosaccharides was performed by adsorption of the digestion mixture from samples < 100 µg of total (glyco)protein onto 100 mg Hypercarb cartridges.

### (b) Analysis of oligosaccharides by matrix-assisted laser desorption/ ionization time-of flight mass-spectrometry (MALDI/TOF/TOF-MS)

A Bruker ULTRAFLEX time-of-flight (TOF/TOF) instrument was used: native desialylated oligosaccharides were analyzed using 2,5-dihydroxybenzoic acid as UV-absorbing material in the positive as well as in the negative ion mode using the reflectron in both cases. For MS-MS analyses, selected parent ions were subjected to laser induced dissociation (LID) and the resulting fragment ions separated by the second TOF stage (LIFT) of the instrument. Sample solutions of 1 µl and an approximate concentration of 1-10 pmol·*µ*l⁻¹ were mixed with equal amounts of the respective matrix. This mixture was spotted onto a stainless steel target and dried at room temperature before analysis.

### Example 8.2 Preparation and characterization of recombinant human EPO (EPO-GT-1)

EPO was expressed from recombinant CHO cells as described (Mueller PP et al., 1999, Domer AJ et al., 1984) and the preparations were characterized according to methods described in the Eur. Phar. *(Ph. Eur. 4, Monography 01*/*2002:1316: Erythropoietin concentrated solution).* The final product had a sialic acid content of 12 nMol (+/- 1.5 nMol) per nMol of protein. The structures of N-linked oligosaccharides were determined by HPAEC-PAD and by MALDI/TOF-MS as described (Nimtz et al., 1999, Grabenhorst, 1999). The EPO preparations that were obtained contained di-, tri- and tetrasialylated oligosaccharides (2-12%, 15-28% and 60-80%, respectively, sulphated and pentasialylated chains were present in small amounts). The overall glycosylation characteristics of EPO preparations were similar to that of the international BRP EPO standard preparation.

The isoelectric focusing pattern of the recombinant EPO was comparable to that of the international BRP Reference EPO standard preparation showing the corresponding isoforms. 25% of the EPO protein lacked O-glycosylation at Ser₁₂₆ of the polypeptide chain.

### Example 8.3 Preparation of partially desialylated EPO forms

**EPO GT-1** protein (2.84 mg/ml) was heated to 80°C in 20 mM Na-phosphate buffer pH 7.0 and then 100 µl of 1 N H₂SO₄ was added per 1 ml of the EPO solution; incubation was continued for 5 min, 10 min and 60 min, respectively, yielding EPO preparations of different degree of sialylation. Quantitation of oligosaccharides with 0-4 sialic acids was performed after liberation of oligosaccharides with polypeptide N-glycosidase and isolation of N-linked chains was performed by desalting using Hypercarb cartridges (25 mg HyperSep Hypercarb; ThermoHypersil-Keystone, UK). EPO preparations were neutralized by addition of 1 N NaOH and were frozen in liquid N₂ and were stored at -20°C until further use.

### Example 8.4 Periodate oxidation of sialylated EPO forms

To 10 mg of untreated or mild acid treated EPO dissolved in 3.5 ml of 20 mM Na-phosphate buffer pH 7.0 was added 1.5 ml of 0.1 M Na-acetate buffer pH 5.5 and the mixture was cooled to 0°C in an ice-bath; 500 *µl* of 10 mM Na-periodate was added and the reaction mixture was kept in the dark for 60 min at 0°C. Then 10 *µl* of glycerol was added and incubation was continued for further 10 min in the dark. The partially oxidized EPO forms were separated from reagents by desalting using VIVASPIN concentrators (10,000 MWCO, PES Vivascience AG, Hannover, Germany) according to manufacturer's recommendation at 3000 rpm in a laboratory centrifuge equipped with a fixed angle rotor. After freezing in liquid nitrogen the EPO preparations were stored in a final volume of 4 ml at -20°C.

100 µg aliquots of the partially oxidized EPO preparation were subjected to N-glycosidase treatment and oligosaccharides were isolated using Hypercarb cartridges as described. Oligosaccharides were desialylated by mild acid treatment and were analyzed by HPAEC-PAD and their retention times were compared to those of authentic standard oligosaccharides as described (Nimtz et al., 1990 and 1993).

### Example 8.5 Reduction of EPO disulfides with dithioerythreitol

5 mg of EPO-GT-1 was incubated in 5 ml of 0.1 M Tris/HCl buffer pH 8.1 in the presence of 30 mM dithioerythreitol (DTT) at 37°C for 60 minutes; removal of DTT was achieved by using a Vivaspin concentrator at 4 °C, 4 cycles of buffer exchange. The final reduced EPO preparation was frozen in liquid nitrogen and stored at -20°C. in 50 mM Na-acetate buffer pH 5.5.

### Example 8.6 EPO protein determination

Quantitative determination of EPO protein was performed by measuring UV absorption at 280 nm according to the Eur. Phar. (European Pharmacopeia 4, Monography 01/2002: 1316: erythropoietin concentrated solution) in a cuvette with 1 cm path length. In addition, EPO was quantitated by applying a RP-HPLC method using a RP-C4 column (Vydac Protein C4, Cat.# 214TP5410, Grace Vydac, Ca, US); the HPLC method was calibrated using the erythropoietin BRP 1 reference standard (European Pharmacopeia, Conseil de l'Europe B.P. 907-F67029, Strasbourg Cedex 1).

### Example 8.7 Oxidation of desialylated EPO with galactose oxidase

4.485 mg of completely desialylated EPO was incubated in 20 mM Na-phosphate buffer pH 6.8 in the presence of 16 *µ*l catalase (6214 units/200 ml) and 80 µl of galactose oxidase (2250 units/ml from *Dactylium dendroides* (Sigma-Aldrich, Steinheim, Germany); incubation at 37°C was over night; 2 times 20 µl of galactose oxidase was added after 4 hours and after 8 hours after starting of the incubation.

### Example 8.8 Preparation of EPO samples for bioassays

### Purification of EPO from incubations of periodate- or galactose-oxidase-oxidized EPO protein preparations with activated HES

Purification of EPO samples (removal of unreacted HES derivatives) was carried out at room temperature. The EPO incubation mixtures (approximately 5 mg of EPO protein) were diluted 1:10 with buffer A (20 mM N-morpholine propane sulfonic acid [MOPS/NaOH] in H₂O bidest, pH 8.0) and were applied to a column containing 3 ml Q-Sepharose HP (Pharmacia Code no. 17-1014-03, Lot no. 220211) equilibrated with 10 column volumes (CV) of buffer A by using a flow rate of 0.5 ml/min. The column was washed with 6-8 CV of buffer A (flow rate = 0.8 ml/min) and elution was performed by using buffer B (20 mM morpholine ethane sulfonic acid [MES/NaOH], 0.5 M NaCl in H₂O bidest, pH 6.5) at a flow rate of 0.5 ml/min. EPO was detected by UV absorption at 280 nm and eluted in about 6 ml. The column was regenerated by using 3 CV of buffer C (20 mM MES, 1.5 M NaCl in H₂O adjusted to pH 6.5) and was re-equilibrated by using 10 CV of buffer A (flow rate = 0.7 ml/min).

Buffer exchange of EPO eluates obtained from the Q-Sepharose step was performed using Vivaspin concentrators and phosphate buffered saline (PBS) with each 3 centrifugation cycles per sample; samples were adjusted to 2 ml with PBS and were stored at -20°C.

Only <25% of the partially desialylated and subsequently mild periodate oxidized EPO forms that were subjected to HES-modification were obtained from the Q-Sepharose eluate since under the conditions employed the basic EPO forms did not bind Q-Sepharose and were found in the flow-through together with nonreacted HES derivatives.

### Example 8.9 High-pH anion-exchange chromatography with pulsed amperometric detection (HPAEC-PAD)

Purified native and desialylated oligosaccharides were analyzed by high-pH anion-exchange (HPAE) chromatography using a Dionex BioLC system (Dionex, USA) equipped with a CarboPac PA1 column (0.4 x 25 cm) in combination with a pulsed amperometric detector (PAD) (Schröter et al., 1999; Nimtz et al., 1999). Detector potentials (E) and pulse durations (T) were: E1: +50 mV, T1: 480 ms; E2: +500 mV, T2: 120 ms; E3: -500 mV, T3: 60 ms, and the output range was 500-1500 nA. The oligosaccharides were then injected onto the CarboPac PA1 column which was equilibrated with 100% solvent A. For desialylated oligosaccharides elution (flow rate: 1 ml.min⁻¹) was performed by applying a linear gradient (0-20%) of solvent B over a period of 40 min followed by a linear increase from 20-100% solvent B over 5 min. Solvent A was 0.2 M NaOH in bidistilled H₂O, solvent B consisted of 0.6 M NaOAc in solvent A. For native oligosaccharides the column was equilibrated with 100% solvent C (0.1 M NaOH in bidistilled H₂O) and elution (flow rate: 1 ml·min⁻¹) was performed by applying a linear gradient (0-35%) of solvent D over a period of 48 min followed by a linear increase from 35-100% solvent D over 10 min. Solvent D consisted of 0.6 M NaAc in solvent C.

### Example 8.10 Monosaccharide compositional analysis of N-glycans, HES-modified N-glycans and EPO protein by GC-MS

Monosaccharides were analyzed as the corresponding methyl glycosides after methanolysis, *N*-reacetylation and trimethylsilylation by GC/MS [Chaplin, M.F. (1982) A rapid and sensitive method for the analysis of carbohydrate. Anal. Biochem. 123, 336-341]. The analyses were performed on a Finnigan GCQ ion trap mass spectrometer (Finnigan MAT corp., San Jose, CA) running in the positive ion EI mode equipped with a 30 m DB5 capillary column. Temperature program: 2 min isotherm at 80°C, then 10 degrees min⁻¹ to 300°C.

Monosaccharides were identified by their retention time and characteristic fragmentation pattern. The uncorrected results of electronic peak integration were used for quantification. Monosaccharides yielding more than one peak due to anomericity and/or the presence of furanoid and pyranoid forms were quantified by adding all major peaks. 0.5 µg of myo-inositol was used as an internal standard compound.

### Example 8.11 Results

### Example 8.11(a) Characterization of N-glycans of mild acid treated (partially desialylated) EPO-GT-1

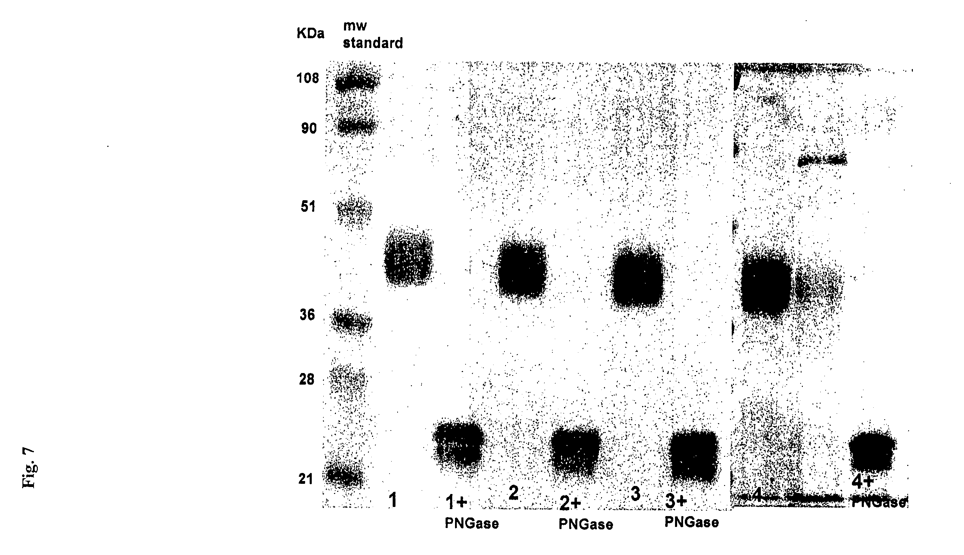

EPO-GT-1 preparations subjected to mild acid treatment for 5, 10 or 60 min. were analyzed by SDS-PAGE before and after liberation of N-linked oligosaccharides by incubation with N-glycosidase as shown in Figure 7. N-linked oligosaccharides were subjected to HPAEC-PAD oligosaccharide mapping (Figure 8). The untreated EPO-GT-1 contained >90% of N-linked oligosaccharides with 3 or 4 sialic acid residues whereas after 5 min. of incubation in the presence of mild acid <40% of carbohydrate chains had 3 or 4 sialic acid residues. HPAEC-PAD of the desialylated N-glycans revealed that the ratio of neutral oligosaccharides that were detected for the untreated EPO-GT-1 and remained stable in the preparations subjected to acid treatment for 5, 10 or 60 min. MALDI/TOF-MS of the desialylated glycans revealed that <90% of the proximal fucose was present after mild acid treatment of the protein.

### Example 8.11(b) Characterization of periodate treated EPO-GT-1

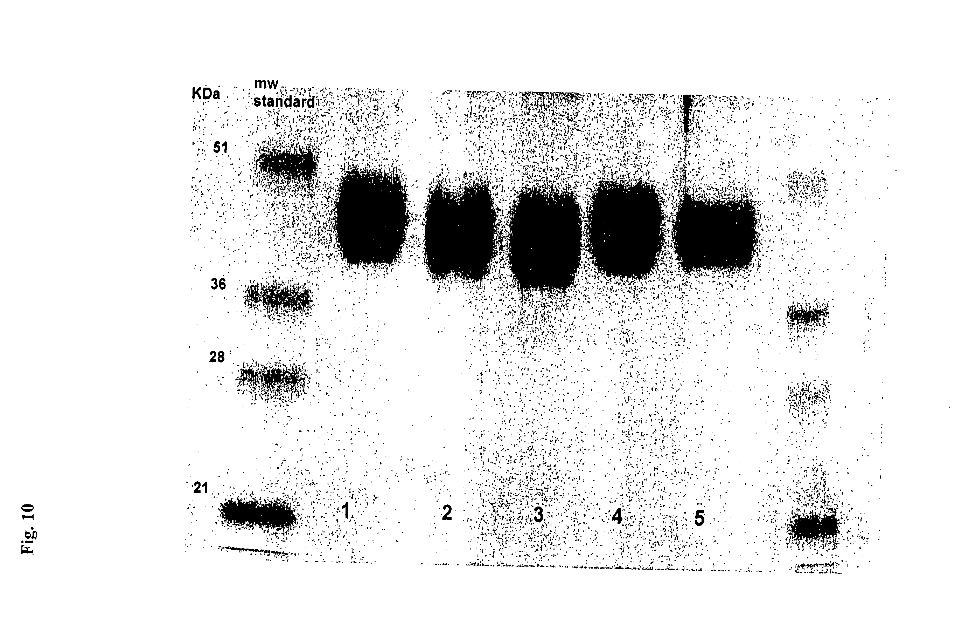

SDS-PAGE mobility of mild periodate treated EPO forms that were previously subjected to a 5 and 10 minute treatment with acid or were not treated are compared in Figure 10. The conditions used for periodate oxidation of sialic acids did not change the SDS-PAGE pattern of EPO preparations (compare Fig. 7). Oxidation of sialic acids resulted in HPAEC-PAD analysis a shift of oligosaccharides to earlier elution times (compare Figures **8** **and** **11**).

### Example 8.11(c) Characterization of HES-modified EPO derivatives

### (aa) Time course of HES modification of EPO-GT-1-A with hydroxylamine-modified HES derivative X, produced according to Example 2.4

400 *µ*g of hydroxylamine-modified HES derivative **X** was added to 20 *µ*g of **EPO-GT-1-A** (mild periodate oxidized EPO, not acid hydrolyzed prior to mild periodate oxidation) in 20 *µ*L of 0.5 M NaOAc buffer pH 5.5 and the reaction was stopped after 30 min, 2, 4, and 17 hours, respectively, by freezing samples in liquid nitrogen. Subsequently samples were stored at -20°C until further analysis.

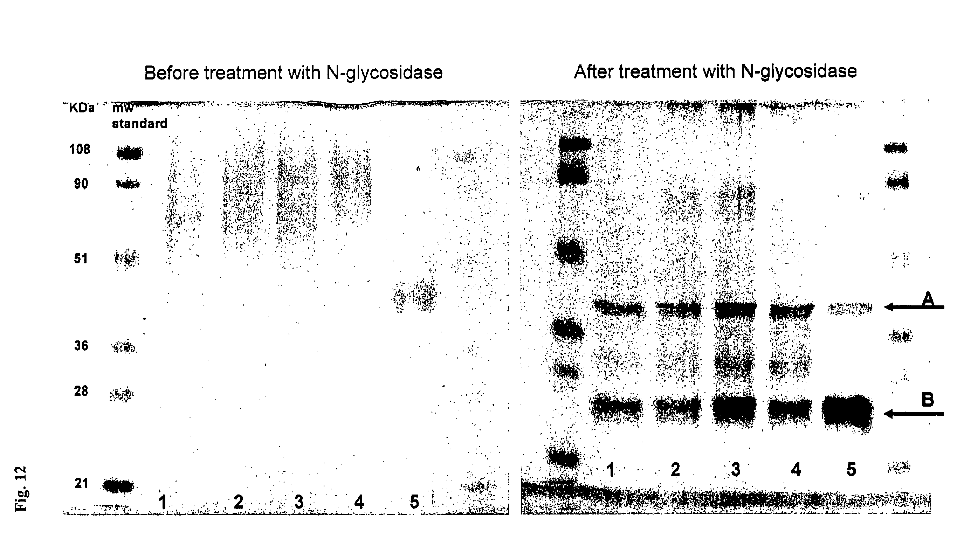

SDS-PAGE sample buffer was added and the samples were heated to 90°C and applied onto SDS-gels. As shown in Figure **12**, increasing incubation times resulted in an increased shift towards higher molecular weight of the protein. After 17 hours of incubation in the presence of the hydroxylamine-modified HES derivative **X** a diffuse Coomassie stained protein band was detected migrating in an area between 60 and 11 KDa, based on the position of molecular weight standards (see left part of Fig. **12**). Upon treatment with N-glycosidase most of the protein was shifted towards the position of de-*N*-glycosylated EPO (see Fig. **12****,** right gel; arrow A indicates migration position of N-glycosidase, arrow B indicates migration position of de-*N-*glycosylated EPO; the diffuse protein band visible in the region between the 28 KDa and 36 KDa molecular weight standards presumably represents EPO-forms which are modified by HES and the O-glycosylation site of the molecule. In view of the specificity of N-glycosidase we conclude from this result that in fact HES-modification occurs at the periodate oxidized sialic acid residues of glycans of the EPO protein.

### (bb) Characterization of HES-EPO conjugates

HES-EPO conjugates **I** (originating from EPO-GT-1 after mild periodate oxidation, i.e. from EPO-GT-1-A), **II** (resulting from EPO-GT-1 subjected to 5 min acid hydrolysis and mild periodate oxidation), **III** (resulting from EPO-GT-1 subjected to 10 min acid hydrolysis and mild periodate oxidation) were synthesized as described before. A control incubation (**K**) was included containing unmodified EPO-GT-1 under the same buffer conditions to which an equivalent amount of unmodified HES was added. The incubation mixtures were subjected to further purification for subsequent biochemical analysis of the HES-EPO derivatives.

Incubations HES-EPO conjugates **I**, **II** and **III** as well as the control incubation K were subjected to a Q-Sepharose purification step as described under "Material and Methods" **(Example 8.8)** in order to remove the excess of nonreacted HES-reagent which was expected in flow through of the ion-exchange column. Due to the high amounts of basic EPO forms contained in previously acid treated samples **II** and **III** we expected considerable amounts of modified EPO product from these incubations in the flow through. As is shown in Figure **13**, almost all of the EPO material from samples **I** was retained by Q-Sepharose column whereas only approximately 20-30% of the samples **III** and **II** was recovered in the fraction eluting with high salt concentration. All of the protein material from the incubations with HES derivative **X**, both in the flow-through and the fractions eluting with high salt, had apparent higher molecular weight in SDS-PAGE when compared to the control EPO.

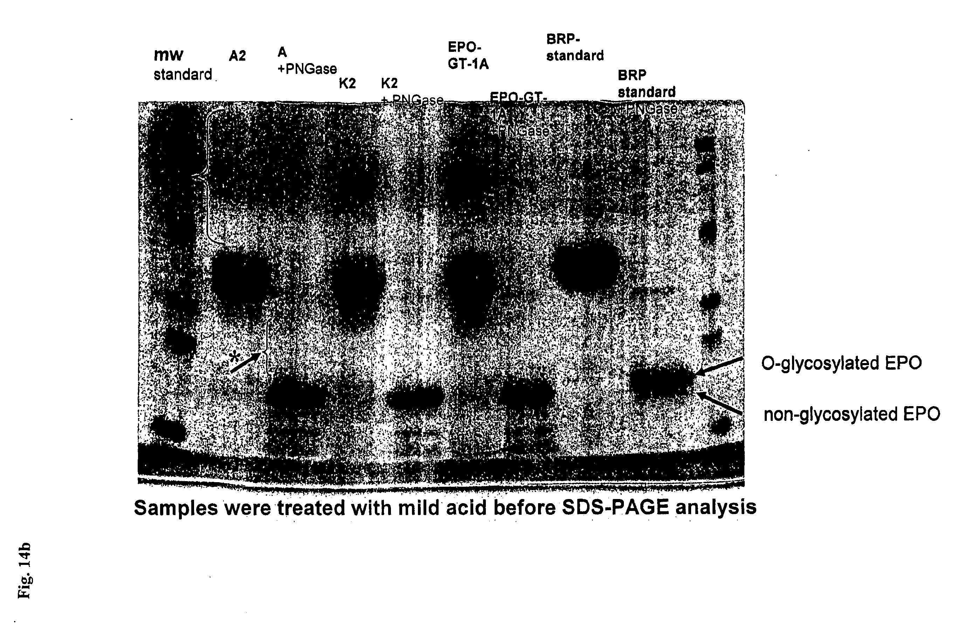

In order to characterize in more detail the HES-modified EPO sample **A** and **K** (see Figure **11**) were compared to periodate oxidized form **EPO-GT-1-A**. The samples were subjected to N-glycosidase treatment and as is depicted in Figures **14a** **and** **14b** the release of N-glycans resulted in the two low molecular weight bands at the position of the O-glycosylated and nonglycosylated EPO forms of the standard EPO preparation. In the case of sample **A** a further band migrating at the position of the 28 KDa mw standard was detected suggesting HES-modification at the O-glycan of this EPO variant (cf. **Example 8.11(c)(aa)).** This band (and also the heavily HES-modified high mw form of N-glycosylated EPO, see Figs. 14a and 14b) disappeared after subjecting the samples to mild hydrolysis which is in agreement with the view that HES modification was achieved at the periodate oxidised sialic acid residues of erythropoietin.

Aliquots of the N-glycosidase incubation mixtures were hydrolyzed using conditions enabling the complete removal of sialic acids residues (and also the sialic acid linked HES derivative) from oligosaccharides; after neutralization, the mixtures were then absorbed onto small Hypercarb columns for their desalting. The columns were washed rigorously with water followed by elution of bound neutral oligosaccharides with 40% acetonitrile in H₂O containing 0.1% of trifuloacetic acid. The resulting oligosaccharides were subjected to MALDI/TOF-MS. The spectra of the desialylated oligosaccharide fractions from sample A, EPO-GT-1-A and sample K showed identical masses for complex type oligosaccharides at m/z = 1810 Da (diantennary), 2175 = triantennary, 2540 = tetraantennary, 2906 = tetraantennary plus 1 N-acetyllactosamine repeat and 3271 = tetraantennary plus 2 N-acetyllactosamine repeats; small signals corresponding to lack of fucose (-146) and galactose (minus 162) were detected which are attributable to the acid hydrolysis conditions applied for sialic acid removal (see MALDI-Figures 17, 18 and 19).

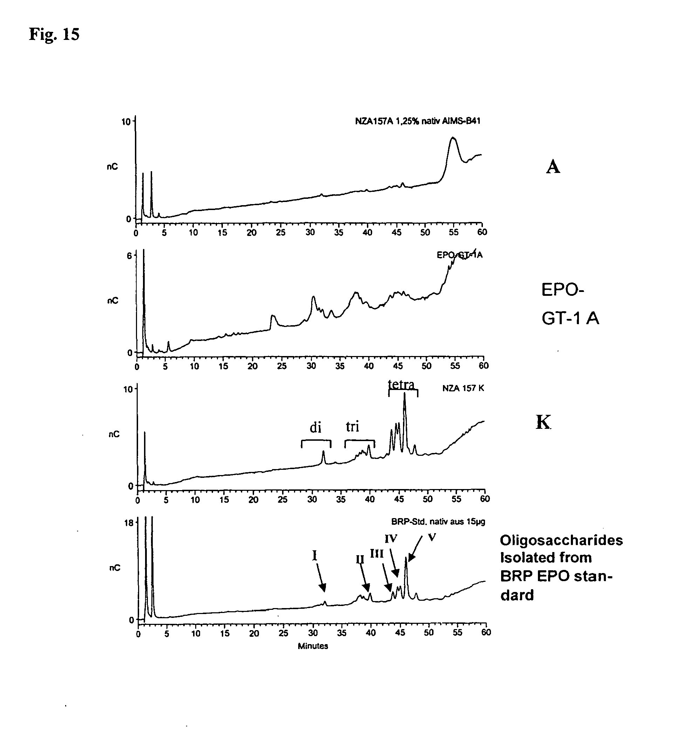

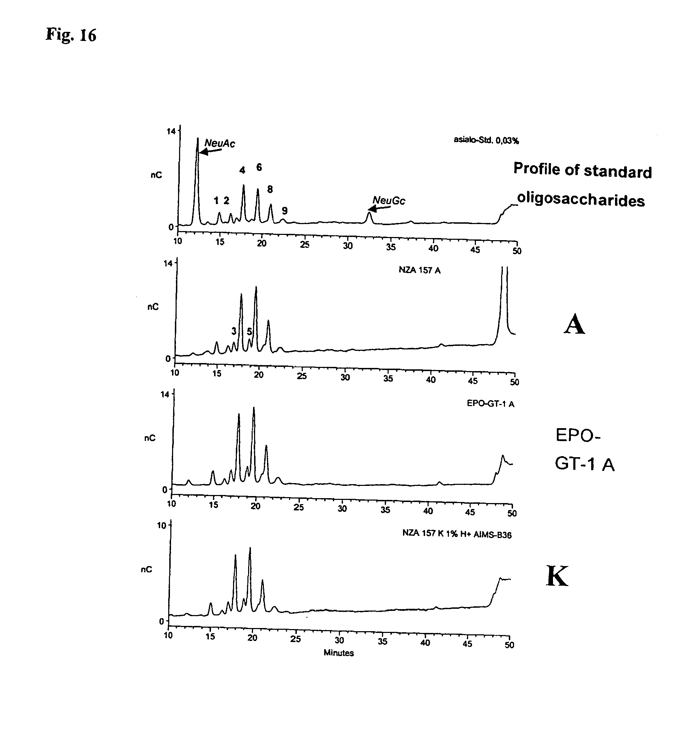

In a parallel experiment the N-glycosidase digestion mixture was absorbed onto 1 ml RP-C 18 cartridge (without prior acid hydrolysis of oligosaccharides) and elution was performed with 5% acetonitrile in water containing 0.1% TFA; under these conditions the EPO protein was completely retained onto the RP-material and oligosaccharides were washed off from the column with 5% acetonitrile in H₂O containing 0.1% TFA. The de-*N*-glycosylated EPO protein was eluted with 70% acetonitrile in H₂O containing 0.1% TFA. The oligosaccharide fractions from the RP-C18 step of N-glycosidase-treated sample **A**, EPO **GT-1-A** and sample **K** were neutralized and subjected to desalting using Hypercarb cartridges as described before. The isolated oligosaccharides were subjected to HPAEC-PAD mapping before (see Figures **15**) and after mild acid treatment under conditions which enabled quantitative removal of sialic acids from glycans (see Figures **16**).

The HPAEC-PAD profile for the native material obtained from the HES-modified sample **A** showed only neglectable signals for oligosaccharides whereas EPO GT-1-A-derived oligosaccharides exhibited the same glycan profile as the one shown in Fig. 11 (sample named EPO-GT-1 after mild periodate treatment). The elution profile of oligosaccharides obtained from the control EPO sample (**K**) yielded the expected pattern (compare profile in Figure **8**). For comparison, the native oligosaccharide profile of the international BRP-EPO standard is included for comparison and as reference standard.

After mild acid hydrolysis, all oligosaccharide preparations showed an identical elution profile of neutral oligosaccharide structures (see Figures **16**) with the expected qualitative and quantitative compositon of di-, tri- and tetraantennary complex-type carbohydrate chains as described in the methods section for the EPO preparation which was used as a starting material in the present study. This result demonstrates that the HES-modification of the EPO sample results in a covalent linkage of the HES derivative which is detached from the EPO-protein by N-glycosidase and is acid-labile since it is removed from the N-glycans using mild acid treatment conditions known to desialylate carbohydrates (see Figures **14a****+b**).

### (cc) Monosaccharide compositional analysis of HES-EPO and HES-EPO N-glycans by GC-MS

In order to further confirm HES-modification of EPO at the N-glycans of the molecule, EPO samples were digested with N-glycosidase and the EPO protein was adsorbed onto RP-C 18 cartridges whereas oligosaccharide material was washed off as described above. As shown in Table 2, glucose and hydroxyethylated glucose derivatives were detected only in the EPO protein which was subjected to HES-modification at cysteine residues and in oligosaccharide fractions of EPO sample A2.

### Example 8.11(d) In-vivo assay of the biological activity of HES-modified EPO

The EPO-bioassay in the normocythaemic mouse system indicates was performed according to the procedures described in the European Pharmacopeia; the laboratory that carried out the EPO assay was using the International BRP EPO reference standard preparation. For the HES-modified EPO **A2** preparation a mean value for the specific activity of **294,600 units per mg EPO of protein** was determined indicating an approximately 3-fold higher specific activity when compared to the International BRP EPO reference standard preparation that was included in the samples sent for activity assays.

The results of the study are summarized in Table 3.

### References for examples 1 to 8:

Nimtz M, Noll G, Paques EP, Conradt HS. Carbohydrate structures of a human tissue plasminogen activator expressed in recombinant Chinese hamster ovary cells. FEBS Lett. 1990 Oct. 1; 271(1-2):14-8
Domer AJ, Wasley LC, Kaufman RJ. Increased synthesis of secreted proteins induces expression of glucose-regulated proteins in butyrate-treated Chinese hamster ovary cells. J Biol Chem. 1989 Dec 5; 264 (34):20602-7
Mueller PP, Schlenke P. Nimtz M, Conradt HS, Hauser H Recombinant glycoprotein quality in proliferation-controlled BHK-21 cells. Biotechnol Bioeng. 1999 Dec 5; 65(5):529-36
Nimtz M, Martin W, Wray V, Kloppel KD, Augustin J, Conradt HS. Structures of sialylated oligosaccharides of human erythropoietin expressed in recobminant BHK-21 cells. Eur J Biochem. 1993 Apr. 1; 213(1):39-56
Hermentin P, Witzel R, Vliegenthart JF, Kamerling JP, Nimtz M, Conradt HS. A strategy for the mapping of N-glycans by high-ph anion-exchange chromatography with pulsed amperometric detection. Anal Biochem. 1992 Jun; 203(2):281-9
Schroter S, Derr P, Conradt HS, Nimtz M, Hale G, Kirchhoff C. Male specific modification of human CD52. J Biol Chem. 1999 Oct. 15;274(42):29862-73

### Example 9

### Production of recombinant EPO

### A) Production in mammalian cells

### Recombinant EPO was produced in CHO cells as follows:

A plasmid harbouring the human EPO cDNA was cloned into the eukaryotic expression vector (pCR3 and named afterwards pCREPO). Site directed mutagenesis was performed using standard procedures as described (Grabenhorst, Nimtz, Costa et al., 1998, In vivo specificity of human alpha 1,3/4-fucosyltransferases III-VII in the biosynthesis of Lewis(x) and sialyl Lewis(x) motifs on complex-type N-glycans - Coexpression studies from BHK-21 cells together with human beta-trace protein, J. Biol. Chem., 273(47), 30985-30994).

CHO cells stably expressing human EPO or amino acid variants (e.g. Cys-29→Ser/Ala, or Cys-33→Ser/Ala , Ser-126→Ala etc.) thereof were generated with the calcium phosphate precipitation method and selected with G418-sulfate as described (Grabenhorst et al.). Three days after transfection, the cells were subcultivated 1:5 and selected in DMEM containing 10% FBS and 1.5 g/liter G418 sulfate.

Using this selection procedure, usually 100-500 clones survived and where propagated in selection medium for a further time period of 2-3 weeks. Cell culture supernatants of confluently growing monolayers were then analyzed for EPO expression levels by Western blot analysis and by IEF/Western Blot analysis.

EPO was produced from stable subclones in spinner flasks or in 21 perfusion reactors. Different glycoforms of EPO with different amounts of NeuAc (e.g. 2-8, 4-10, 8-12 NeuAc residues) were isolated according to published protocols using combinations various chromatographic procedures as described below.

### Literature:

Grabenhorst, Conradt, 1999, The cytoplasmic, transmembrane, and stem regions of glycosyltransferases specify their in vivo functional sublocalization and stability in-the Golgi., J Biol Chem., 274(51), 36107-16; Grabenhorst, Schlenke, Pohl, Nimtz, Conradt, 1999, Genetic engineering of recombinant glycoproteins and the glycosylation pathway in mammalian host cells, Glycoconj J., 16(2), 81-97; Mueller, Schlenke, Nimtz, Conradt, Hauser, 1999, Recombinant glycoprotein product quality in proliferation-controlled BHK-21 cells, Biotechnology and bioengineering, 65(5), 529-536; Schlenke, Grabenhorst, Nimtz, Conradt, 1999, Construction and characterization of stably transfected BHK-21 cells with human-type sialylation characteristic, Cytotechnology, 30(1-3), 17-25.

### B) Production in insect cells

Recombinant human EPO was produced from insect cell lines SF9 and SF 21 after infection of cells with recombinant baculovirus vector containing the human EPO cDNA under control of the polyhedrin promoter as described in the literature.

Cells grown in serum-free culture medium were infected at cell density of 2x10⁶ or X10⁷ cells per mL and EPO titers were determined every day in the cell culture supernatants. EPO was purified by Blue sepharose chromatography, ion-exchange chromatography on Q-Sepharose and finally RP-HPLC on C₄-Phase.

Purity of the product was checked by SDS-PAGE and N-terminal sequencing. Detailled carbohydrate structural analysis (N- and O-glycosylation) was performed according to published procedures.

### Literature:

Grabenhorst, Hofer, Nimtz, Jager, Conradt, 1993, Biosynthesis and secretion of human interleukin 2 glycoprotein variants from baculovirus-infected Sf21 cells. Characterization ofpolypeptides and posttranslational modifications, Eur J Biochem., 215(1), 189-97; Quelle, Caslake, Burkert, Wojchowski, 1989, High-level expression and purification of a recombinant human erythropoietin produced using a baculovirus vector, Blood, 74(2), 652-7

### Example 10

### Formation of reactive HES derivatives

### 1. SH-reactive HES

### 1.1 Reaction of EMCH with Oxo-HES12KD to form SH-reactive HES12KD B

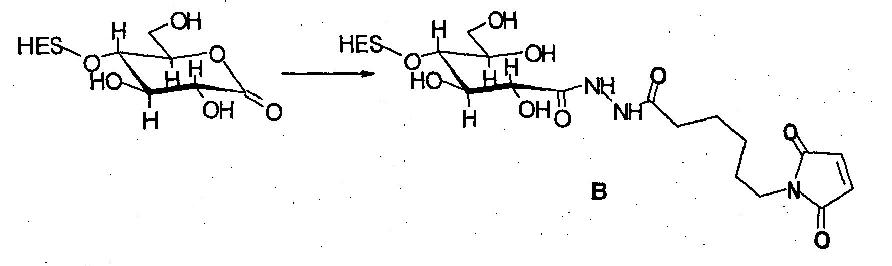

0.144 g (0.012 mmol) of Oxo-HES12KD (Fresenius German Patent DE 196 28 705 A1) were dissolved in 0.3 mL absolute dimethyl sulfoxide (DMSO) and were added dropwise under nitrogen to a mixture of 34 mg (0.15 mmol) EMCH (Perbio Science, Deutschland GmbH, Bonn, Germany) in 1.5 mL DMSO. After stirring for 19 h at 60°C the reaction mixture was added to 16 mL of a 1:1 mixture of ethanol and acetone. The precipitate was collected by centrifugation, redissolved in 3 mL DMSO and again precipitated as described. The SH-reactiv-HES12KD **B** was obtained by centrifugation and drying in vaccuo. The conjugation reaction with Thio-EPO is described in Example 11, 2.2.

### Alternatives:

In this reaction, all cross-linkers can be used, which exhibit a hydrazide- and a maleimide function, separated by a spacer. Further examples for molecules of that group, available from Perbio Science, Deutschland GmbH, Bonn, Germany, are shown in Table 1; marked with an "A". Furthermore, another group of cross-linkers exhibiting an activated disulfide function instead of a maleimide funcion could also be used.

### 1.2 Halogenacetamide-derivatives of HES glycosylamines

### a) Glycosylamine-formation¹

¹Manger, Wong, Rademacher, Dwek, 1992, Biochemistry, 31, 10733-10740; Manger, Rademacher, Dwek, 1992, Biochemistry, 31, 10724-10732

A 1 mg sample of HES12KD was dissolved in 3 mL of saturated ammonium bicarbonate. Additional solid ammonium bicarbonate was then added to maintain saturation of the solution during incubation for 120 h at 30°C. The Amino-HES12KD C was desalted by direct lyophilization of the reaction mixture.

### b) Acylation of the glycosylamine C with chloroacetic acid anhydride

A 1 mg sample of Amino-HES12KD C was dissolved in 1 mL of 1 M sodium bicarbonate and cooled on ice. To this was added a crystal of solid chloroacetic acid anhydride (~5 mg), and the reaction mixture was allowed to warm to room temperature. The pH was monitored and additional base was added if the pH dropped below 7.0. After two hours at room temperature a second aliquot of base and anhydride was added. After six hours the product Chloroacetamide-HES D1 (X = Cl) was desalted by passage over a mixed bed Amberlite MB-3(H)(OH) ion exchange resins.

### c) Acylation of the glycosylamine with bromoacetic anhydride²

²Blach Kiss, Tull, Withers, 1993,Carbohydr. Res., 250, 195

Bromoacetic anhydride was prepared as described by Thomas.³ A 1 mg sample of amino-HES 12KD C was dissolved in 0.1 mL of dry DMF and cooled on ice and 5 mg bromoacetic anhydride was added. The reaction mixture was brought slowly to room temperature and the solution was stirred for 3 h. The reaction mixture was added to 1 mL of a 1:1 mixture of ethanol and acetone with -20 °C. The precipitate was collected by centrifugation, redissolved in 0.1 mL DMF and again precipitated as described. The Bromoacetamide-HES **D2** (X = Br) was obtained by centrifugation and drying in vaccuo. The conjugation reaction with Thio-EPO is described in Example 11, 1.2.
³Thomas, 1977, Methodes Enzymol., 46, 362

### d) The corresponding Iodo-derivative D3 (X = I) was synthesised as described for D2. Instead bromoacetic anhydride N-succinimidyl iodoacetate was used

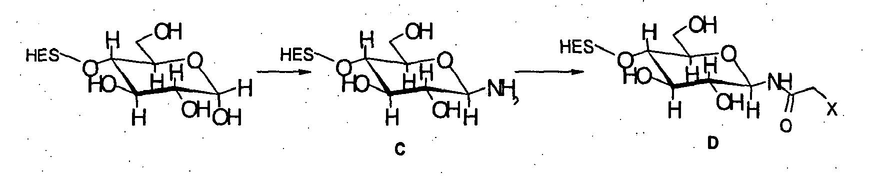

and all steps were performed in the dark.

### Alternatives:

For acylation of amino groups, other activated forms of halogen acidic acids can be used, e.g.
- bromides or -chlorides
- esters, e.g. N-hydroxysuccinimide ester, esters with substituted phenoles (p-nitrophenole, pentafluorophenole, trichlorophenole etc)

Furthermore, all cross-linkers having an amino reactive group and a halogen acetyl function, separated by a spacer, could be used. An example thereof is SBAP. This molecule and others are available from Perbio Science Deutschland GmbH, Bonn, Germany. They are marked in Table 1 with an "D". For the use as cross-linkers
for the ligation of amino-HES with thio-EPO without isolation of the halogenacetamid-HES derivatives see remarks in example 11, 1.2.

### 1.3 Halogenacetamide-derivatives of Amino-HES E¹

a) Reaction of 1,4-diaminobutane with Oxo-HES12KD to amino-HES 12KD **E⁴**
   ⁴S. Frie, Diplomarbeit, Fachhochschule Hamburg, 1998
   1.44 g (0.12 mmol) of Oxo-HES12KD were dissolved in 3 mL dry dimethyl sulfoxide (DMSO) and were added dropwise under nitrogen to a mixture of 1.51 mL (15 mmol) 1,4-diaminobutane in 15 mL DMSO. After stirring for 19 h at 40°C the reaction mixture was added to 160 mL of a 1:1 mixture of ethanol and acetone. The precipitate Amino-HES 12KD **E** was collected by centrifugation, redissolved in 40 mL of water an dialysed for 4 days against water (SnakeSkin dialysis tubing, 3.5 KD cut off, Perbio Science Deutschland GmbH, Bonn, Germany) and lyophilized. 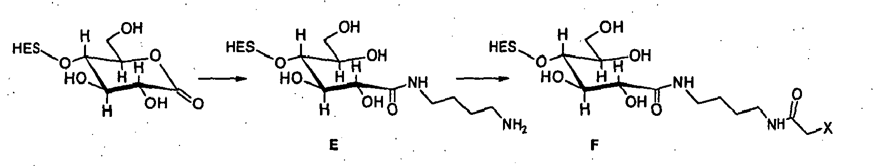
b) Chloroacetamide-HES12KD **F1** was prepared as described for Chloroacetamide-HES12KD **D1** in 1.3 above.
c) Bromoacetamide-HES 12KD **F2** (X = Br) was prepared as described for Bromoacetamide-HES12KD **D2** in 1.3 above. The conjugation reaction with Thio-EPO is described in Example 11, 1.2.
d) The corresponding Iodo-derivative **F3** (X = I) was not isolated before its reaction with Thio-EPO. The experiment is described in Example 11, 1.1.

### Alternatives:

### See 1.2 above

### 2. CHO-Reactive HES

### 2.1 Hydrazide-HES

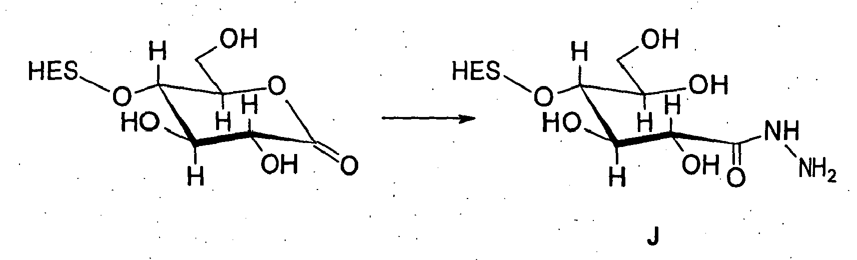

a) Reaction of hydrazine with Oxo-HES12KD
   1,44 g (0.12 mmol) of Oxo-HES12KD were dissolved in 3 mL absolute dimethyl sulfoxide (DMSO) and were added dropwise under nitrogen to a mixture of 0.47 mL (15 mmol) hydrazine in 15 mL DMSO. After stirring for 19 h at 40°C the reaction mixture was added to 160 mL of a 1:1 mixture of ethanol and acetone. The precipitated product **J** was collected by centrifugation, redissolved in 40 mL of water and dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 12, 2.2. 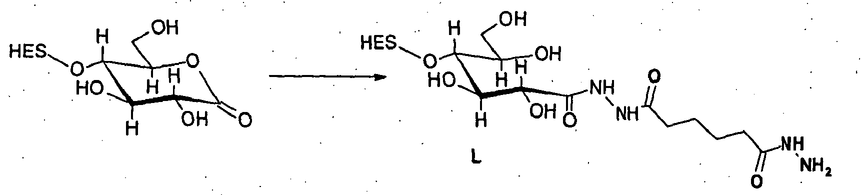
b) Reaction of adipic dihydrazide with Oxo-HES12KD
   1.74 g (15 mmol) adepic dihydrazide were dissolved in 20 mL absolute dimethyl sulfoxide (DMSO) at 65°C and 1,44 g (0,12 mmol) of Oxo-HES 12KD, dissolved in 3 mL absolute DMSO were added dropwise under nitrogen. After stirring for 68 h at 60°C the reaction mixture was added to 200 mL of water The solution containing **L** was dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 12, 2.2.

### Alternatives:

Furthermore, derivatives can be used, wherein 2 hydrazid groups are separated by any spacer.

### 3. Further Amino-HES12KD derivatives I and H¹

Ammonolysis of **D** or **F** was performed separately by dissolving a 1 mg sample of each halogeneacetamide in 0.1 mL of saturated ammonium carbonate. Additional solid ammonium carbonate was then added to maintain saturation of the solution during incubation of 120 h at 30°C. The reaction mixture was added to 1 mL of a 1:1 mixture of ethanol and acetone with -20°C. The precipitate was collected by centrifugation, redissolved in 0.05 mL water and again precipitated as described. The product aminoHES **H** or **I** was obtained by centrifugation and drying in vaccuo. The conjugation reaction with oxidised Glyco-EPO is described in Example 12,4.1.

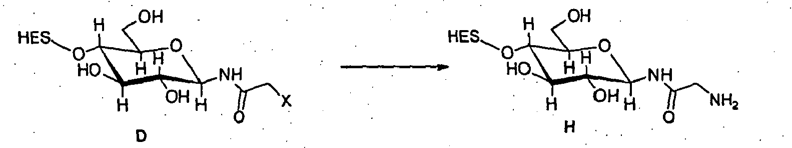

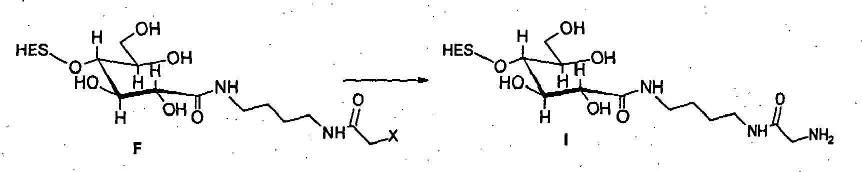

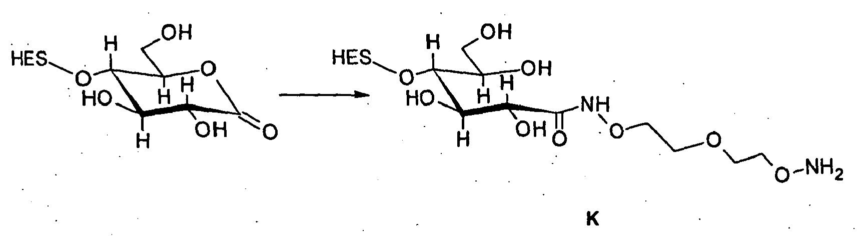

### 4. Hydroxylamine-modified HES12KD K

O-[2-(2-aminooxy-ethoxy)-ethyl]-hydroxylamine was synthesized as described by Boturyn et al in 2 steps from commercially available materials.⁵ 1,44 g (0.12 mmol) of Oxo-HES 12KD were dissolved in 3 mL absolute dimethyl sulfoxide (DMSO) and were added dropwise under nitrogen to a mixture of 2.04 g (15 mmol) O-[2-(2-aminooxy-ethoxy)-ethyl]-hydroxylamine in 15 mL DMSO. After stirring for 48 h at 65°C the reaction mixture was added to 160 mL of a 1:1 mixture of ethanol and acetone. The precipitated product **K** was collected by centrifugation, redissolved in 40 mL of water and dialysed for 4 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 12,3.1.
⁵Boturyn, Boudali, Constant, Defrancq, Lhomme, 1997, Tetrahedron, 53, 5485

### Alternatives:

Furthermore, derivatives could be used, wherein the two hydroxylamine groups are separated by any spacer.

### 5. Thio-HES12KD

### 5.1 Addition to Oxo-HES12KD

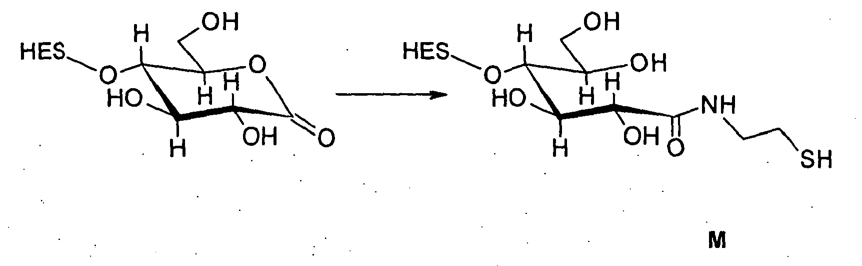

1,44 g (0.12 mmol) of Oxo-HES12KD were dissolved in 3 mL absolute dimethyl sulfoxide (DMSO) and were added to a mixture of 1.16 g (15 mmol) cysteamine in 15 mL DMSO under nitrogen dropwise. After stirring for 24 h at 40°C the reaction mixture was added to 160 mL of a 1:1 mixture of ethanol and acetone. The precipitated product **M** was collected by centrifugation, redissolved in 40 mL of water and dialysed for 2 days against a 0.5 % (v/v) triethylamine in water solution and for 2 days against water (SnakeSkin dialysis tubing, 3.5 KD cut off, Perbio Science Deutschland GmbH, Bonn, Germany) and lyophilized. The conjugation reaction with oxidised Glyco-EPO is described in Example 12,2.1.

### Alternatives:

Derivatives could be used, wherein the amino group and the thio-function are separated by any spacer. Furthermore, the amino group in the derivatives could be replaced by a hydrazine, a hydrazid or a hydroxylamine. The thio-function could be protected in the form of e.g. a disulfide or a trityl-derivative. However, in this case, a further deprotection step must be preformed before the conjugation, which would release a component being analogous to **M**.

### 5.2 Modifikation of Amino-HES12KD E, H or I

### a) Modification with SATA/SATP

1,44 g (0.12 mmol) of Amino-HES 12KD **E**, **H** or **I** were dissolved in 3 mL absolute dimethyl sulfoxide (DMSO) and were added to a mixture of 139 mg (0.6 mmol) SATA in 5 mL DMSO under nitrogen dropwise. After stirring for 24 h at room temperature the reaction mixture was added to 160 mL of a 1:1 mixture of ethanol and acetone. The precipitated product **N** was collected by centrifugation, redissolved in 40 mL of water and dialysed for 2 days against water (SnakeSkin dialysis tubing, 3.5 KD cut off, Perbio Science Deutschland GmbH, Bonn, Germany) and lyophilized.

The deprotection was performed in a 50 mM sodium phosphate buffer, containing 25 mM EDTA and 0.5M hydroxylamine, pH7.5 for 2 hours at room temperature and the product **O** was purified by dialysis against a 0.1 M sodium acetate buffer pH 5.5, containing 1 mM EDTA. The deprotection reaction was performed immediately before the conjugation reaction which is 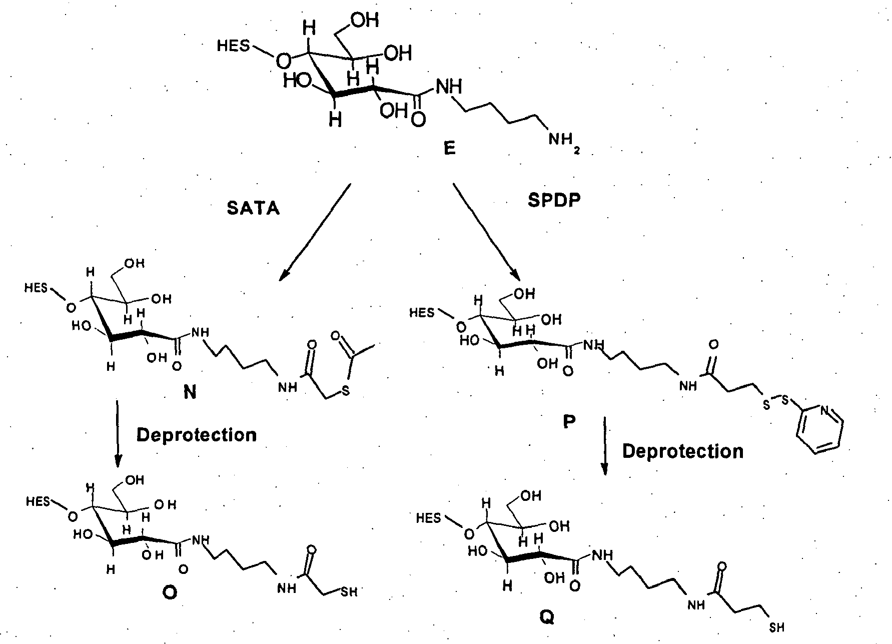 described in Example 12,2.1.

### b) Modification with SPDP

1,44 g (0.12 mmol) of Amino-HES12KD **E**, **H** or **I** were dissolved in 3 mL absolute dimethyl sulfoxide (DMSO) and were dropwise added to a mixture of 187 mg (0.6 mmol) SPDP in 5 mL DMSO under nitrogen. After stirring for 24 h at room temperature the reaction mixture was added to 160 mL of a 1:1 mixture of ethanol and acetone. The precipitated product **P** was collected by centrifugation, redissolved in 40 mL of water and dialysed for 2 days against water (SnakeSkin dialysis tubing, 3.5 KD cut off, Perbio Science Deutschland GmbH, Bonn, Germany) and lyophilized.

The deprotection was performed in a solution of 12 mg dithiothreitol (DTT) per 0.5 mL 100 mM sodiumacetate buffer, containing 100 mM sodium chloride at pH 4.5 for 30 min at room temperature and the product **Q** was purified by dialysis against a 0.1 M sodium acetate buffer pH 5.5, containing 1 mM EDTA. The deprotection reaction was performed immediately before the conjugation reaction which is described in Example 12, 2.1.

### Alternatives:

For the conversion of amino- to thiol-groups, either in free form or protected, several reagants are available. After the modification, the products could be isolated. Alternatively, as accepted for the use of cross-linkers, they could be directly used for the conjugation reaction, preferably after a purification step. For the isolation and storage of thio-HES derivatives, the synthesis of thio-HES derivatives in a protected form may be useful. For this, all derivatives being analogous to SATA could be used, which have an active ester-function and a thioester-function, separated by any spacer. SATP, being a further member of this group, is found in Table 1, marked with an "H". The derivatives being analogous to SPDP could have an acitve ester-function and a disulfide-function, separated by any spacer. Further members of these groups are found in Table 1, marked with an "F". Further analogous derivatives could have an active ester-function and a thiol-function, protected as a trityl derivative, separated by any spacer.

### Example 11

### Conjugation reactions with Thio-EPO

### 1. Reaction of Thio-EPO with a halogenacetamide-modified SH-reactive HES

### 1.1 Example Protocol 1

Conjugation of ThioEPO to Amino-HES12KD (**E**, **H** or **I**) with a Cross-linker containing a NHS-active-ester and an iodoacetamide group, e.g. SIA.⁶
⁶Cumber, Forrester, Foxwell, Ross, Thorpe, 1985, Methods Enzymol., 112, 207

### Materials

A. Borate buffer. Composition was 50 mM sodium borate, pH 8.3, 5 mM EDTA.
B. PBS, phosphate buffered saline: 10 mM sodium phosphate, 150 mM NaCl, pH 7.4.
C. AminoHES 12KD **E**, **H** or **I**. Prepared at 1 mg/mL in borate buffer.
D. Crosslinker stock solution: 14 mg SIA were dissolved in 1 mL DMSO
E. D-Salt^{™} Dextran Desalting Columns, 2 x 5 mL bed volume (Perbio Science Deutschland GmbH, Bonn, Germany)
F. Coomassie® Protein Assay Reagent (Perbio Science Deutschland GmbH, Bonn, Germany)
G. ThioEPO solution: 5 mg/mL of ThioEPO **1** in borate buffer.
H. Microconcentrator: Microcon YM-3 (amicon, Milipore GmbH, Eschborn, Germany)

### Method

100 µL SIA solution was added to 400 µL of the aminoHES12KD **E** solution and was allowed to react with agitation for 0.5 hours at room temperature. The excess crosslinker was removed by centrifuging the sample at 14000 x g for 60 minutes using a microconcentrator. After centrifuging the sample was brought up to its original volume in borate buffer and this process was repeated two more times. The residual solution was added to 1 mL of ThioEPO solution and the reaction mixture was incubated for 16 hour at room temperature. Reactivity of the excess iodoacetamide was quenched at the end of the incubation period by the addition of cysteine to a final concentration of 10 mM. The reaction mixture was applied to a desalting column equilibrated with PBS buffer and the protein content of the fractions were monitored with a Coomassie protein assay reagent. All fractions containing the protein conjugate were pooled and the the conjugate was obtained by lyophylisation after dialysis against water over night.

### Alternatives:

In this reaction, all cross-linkers could be used, which have a succinimide- or a sulfosuccinimide function and a iodoacetamide function separated by a spacer. Further examples are found in Table 1. They are marked with a "C" and are avialable from Perbio Science Deutschland GmbH, Bonn, Germany.

### 1.2 Example Protocol 2

Conjugation of ThioEPO **1** to SH reactiveHES12KD bromoacetamide **D2, F2** or iodoacetamide **D3.**⁷
⁷de Valasco, Merkus, Anderton, Verheul, Lizzio, Van der Zee, van Eden, Hoffmann, Verhoef, Snippe, 1995, Infect. Immun., 63, 961

### Materials

A. Phosphate buffer. Composition was 100 mM sodium phosphate, pH 6.1, 5 mM EDTA.
B. PBS, phosphate buffered saline: 10 mM sodium phosphate, 150 mM NaCl, pH 7.4.
C. SH reactiveHES12KD bromoacetamide **D2**. Prepared at 10 mg/mL in phosphate buffer.
D. D-Salt^{™} Dextran Desalting Columns, 2 x 5 mL bed volume (Perbio Science Deutschland GmbH, Bonn, Germany)
E. Coomassie® Protein Assay Reagent (Perbio Science Deutschland GmbH, Bonn, Germany)
F. ThioEPO solution: 5 mg/mL of ThioEPO **1** in phosphate buffer.

### Method

1 mL SH reactiveHES12KD bromoacetamide **D2** solution and 1 mL of ThioEPO solution were combined and the reaction mixture was incubated for 48 hours at room temperature. Reactivity of the excess bromoacetamide was quenched at the end of the incubation period by the addition of cysteine to a final concentration of 10 mM. The reaction mixture was applied to a desalting column, equilibrated with PBS buffer. The protein content of the fractions were monitored with a Coomassie protein assay reagent, all fractions containing the protein conjugate were pooled and the the conjugate was obtained by lyophylisation after dialysis against water over night.

### Alternatives:

Instead of the isolation of the SH reactive HES12KD-bromoacetamid **D2**, amino HES12KD **(E, H, I)** could be linked with a cross-linker via a succinimide- and a bromoacetamid function (see 1.1 above). SBAP is a member of this group of cross-linkers and is found in Table 1, marked with a "D".

### 2. Reaction of Thio-EPO with a maleimide-modified SH-reactive HES

### 2.1 Example Protocol 4

Conjugation of ThioEPO to Maleimido-HES12KD **B**.

### Materials

A. Maleimido-HES12KD **B**: 10 mg/mL in 0.1 M sodium acetate buffer, pH 5.5
B. ThioEPO solution: 5 mg/mL of ThioEPO in phosphate/NaCl-buffer
C. Phosphate/NaCI: 0.1 M sodium phosphate, 50mM NaCI, pH 7.0
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline: 10 mM sodium phosphate, 150 mM NaCl, pH 7.4.

### Method

1 mL SH-reactive-HES12KD **B** solution and 1 mL of ThioEPO **1** solution were combined and the reaction mixture was incubated for 2 hours at room temperature. Reactivity of the excess maleimides was quenched at the end of the incubation period by the addition of cysteine to a final concentration of 10 mM. The reaction mixture was applied to Sephadex® G-200 (1.5 x 45 cm) equilibrated with PBS buffer and 1 mL fractions were collected. The protein content of the fractions were monitored with a Coomassie protein assay reagent. All fractions containing the protein conjugate were pooled and the the conjugate was obtained by lyophylisation after dialysis against water over night.

### 2.2 Example Protocol 12

Conjugation of ThioEPO to aminoHES12KD (**E, H, I**) with a Cross-linker containing a NHS-active-ester and a maleimide group, e.g. SMCC

### Materials

A: Microconcentrator: Microcon YM-10 (amicon, Milipore GmbH, Eschborn, Germany).
B. PBS, phosphate buffered saline:10 mM sodium phosphate, 150 mM NaCl, pH 7.4.
C. AminoHES12KD **E, H** or **I**. Prepared at 10 mg/mL in PBS buffer.
D. SMCC solution: 1 mg SMCC were dissolved in 50 µL DMSO
E. D-Salt^{™} Dextran Desalting Columns, 2 x 5 mL bed volume (Perbio Science Deutschland GmbH, Bonn, Germany)
F. Coomassie® Protein Assay Reagent (Perbio Science Deutschland GmbH, Bonn, Germany)
G. ThioEPO **1** solution: 5 mg/mL of ThioEPO **1** in PBS buffer.

### Method

To 50 µL SMCC solution 400 µL of the aminoHES12KD **E** solution was added and the reaction mixture was allowed to react with agitation for 80 min at room temperature and for 10 min at 46°C. The excess crosslinker was removed by centrifugation of the reaction mixture through a microconcentrator at 14000 x g for 60 min. The volume was brought up to 450 µL with PBS buffer and the process was repeated two more times. After the last centrifugation, the residual solution was brought up to 450 µL with PBS and was added to 1 mL of ThioEPO solution and the reaction mixture was incubated for 16 hours at room temperature. Reactivity of the excess maleimide was quenched at the end of the incubation period by the addition of cysteine to a final concentration of 10 mM. The reaction mixture was applied to a desalting column equilibrated with PBS buffer. The protein content of the fractions were monitored with a Coomassie protein assay reagent, all fractions containing the protein conjugate were pooled and the conjugate was obtained by lyophylisation after dialysis against water over night.

### Alternatives:

In this reaction, all cross-linkers could be used which have a succinimide- or a sulfosuccinimide function and a maleimide-function, separated by a spacer. Further examples for this group of molecules, available from Perbio Science Deutschland GmbH, Bonn, Germany, are found in Table 1, marked with an "E". There is a further group of cross-linkers, which have instead of a maleimide function an activated disulfide function. These cross-linkers could also be used for the conjugation. However, the disulfide bond of the conjugate is cleavable under reductive conditions. Members of this group are marked in Table 1 with a "F". A third group of cross-linkers uses instead of a maleimide function a vinylsulfon function as a SH-reactive group. A member of this group "SVSB" is marked in Table 1 with a "G".

### Example 12

### Conjugation reactions with oxidized EPO

### 1. Oxidation of Glyco-EPO

### 1.1 Oxidation of Glyco-EPO with sodium meta-periodate: Example Protocol 5

### Materials

A. Glyco-EPO solution: 10 mg/mL of Glyco-EPO in acetate buffer
B. Sodium meta-periodate solution: 10 mM or 100 mM sodium periodate in acetate buffer, prepared fresh. Keep in dark. Using these solutions, the final concentration of sodium periodate in the oxidation mixture is 1 mM or 10 mM, respectively.
C. acetate buffer: 0.1 M sodium acetate buffer, pH 5.5
D. Glycerol
E. Microconcentrator: Microcon YM-3 (amicon, Milipore GmbH, Eschborn, Germany)

### Method

All steps were performed in the dark.

To 1 mL of cold Glyco-EPO solution 0.1 mL of cold sodium meta-periodate solution were added and the the oxidation reaction was allowed to proceed for 1 hour in the dark. If the Glyco-EPO to be oxidized contained sialic acid residues, then the oxidation conditions were 1 mM sodium periodate, 0°C. Otherwise, 10 mM sodium periodate at room temperature was used. To stop the oxidation glycerol was added to a final concentration of 15 mM and incubated for 5 minutes at 0°C. The excess reagents and by-products were remove by centrifuging of the product at 14000 x g for 60 minutes using a microconcentrator. After centrifuging, sample was brought up to its original volume in the buffer used in the next modification step, e.g. in the acetate buffer. This process was repeated two more times.

### 1.2 Enzymatic oxidation of Glyco-EPO: Example Protocol 6

The enzymatic oxidation of EPO is described elsewhere (Chamow et al., 1992, J. Biol. Chem., 267, 15916-15922).

### 2. Conjugation with Hydrazine/Hydrazide-Derivatives

### 2.1 Example Protocol 7

Conjugation of oxidised Glyco-EPO to Thio-HES12KD **M, O** or **Q** with a Cross-linker containing a hydrazide and a maleimide functional group, e.g.M₂C₂H (Perbio Science, Deutschland GmbH, Bonn, Germany).

### Materials

A. M₂C₂H stock: 10 mg/mL M₂C₂H in DMSO, prepared fresh
B. Oxidised Glyco-EPO solution from 6.1.1: 5 mg/mL of Glyco-EPO in acetate buffer
C. Thio-HES 12KD **M**, **O** or **Q**: 10 mg/mL in phosphate/NaCl buffer
D. Acetate buffer: 0.1 M sodium acetate buffer, pH 5.5
E. Phosphate/NaCl: 0.1 M sodium phosphate, 50 mM NaCl, pH 7.0
F. Microconcentrator: Microcon YM-3 (amicon, Milipore GmbH, Eschborn, Germany)
G. Gel filtration column: for example, Sephadex® G-200 (1.5 x 45 cm)
H. Coomassie® Protein Assay Reagent (Perbio Science Deutschland GmbH, Bonn, Germany)
I. PBS, phosphate buffered saline:10 mM sodium phosphate, 150 mM NaCl, pH 7.4

### Method

M₂C₂H stock solution was added to 1 mL of oxidized Glyco-EPO to a final concentration of 1 mM and was allowed to react with agitation for 2 hours at room temperature. The excess crosslinker was removed by centrifuging the sample at 14000 x g for 60 minutes using a microconcentrator. After centrifuging the sample was brought up to its original volume in phosphate/NaCI buffer and this process was repeated two more times. To the M₂C₂H-modified Glyco-EPO 1 mL of Thio-HES12KD M, O or Q solution was added and the reaction mixture was incubated for 16 hours at room temperature. Reactivity of the excess maleimides was quenched at the end of the incubation period by the addition of cysteine. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the conjugate was obtained by lyophylisation after dialysis against water over night.

### Procedural Notes

The hydrazone adduct is slightly less stable at extremes of pH. For applications that may involve treatment at low pH, we reduced the hydrazone by treatment with 30 mM sodium cyanoborohydride in PBS buffer to a hydrazine. For most applications, this extra step was unnecessary.

### 2.2 Example Protocol 8

Direct conjugation of oxidised Glyco-EPO to Hydrazido-HES 12KD **L**.

### Materials

A. Oxidised Glyco-EPO solution from 6.1.1: 5 mg/mL of Glyco-EPO in acetate buffer
B. Hydrazido-HES 18KD **L** or **J**: 10 mg/mL in acetate buffer
C. Acetate buffer: 0.1 M sodium acetate buffer, pH 5.5
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline: 10 mM sodium phosphate, 150 mM NaCl, pH 7.4

### Method

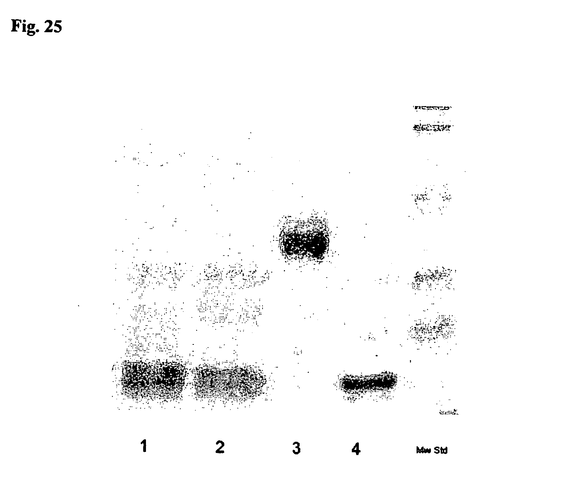

1 mL of Hydrazido-HES12KD L solution and 1 mL of oxidized Glyco-EPO solution were combined and the reaction mixture was allowed to react with agitation for 16 hours at room temperature. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the the conjugate was obtained by lyophylisation after dialysis against water over night. The result of the conjugation is shown in Figure 24. The observed molecular shift demonstrates that the conjugation was successful. The smear results from the heterogenity of HES. Figure 25 demonstrates that HES is conjugated to a carbohydrate moiety of a carbohydrate side chain.

### Procedural Notes

The hydrazone adduct is slightly less stable at extremes of pH. For applications that may involve treatment at low pH, we reduced the hydrazone by treatment with 30 mM sodium cyanoborohydride in PBS buffer to a hydrazine. For most applications, this extra step was unnecessary.

### 3. Conjugation with Hydroxylamine-Derivatives⁸

⁸Rose, 1994, Am. Chem. Soc., 116, 30

### 3.1 Example Protocol 9

Conjugation of oxidized Glyco-EPO to Hydroxylamino-HES12KD K

### Materials

A. Oxidised Glyco-EPO solution from 6.1.1: 5 mg/mL of Glyco-EPO in acetate buffer
B. Hydroxylamino-HES12KD **K**: 10 mg/mL in acetate buffer
C. Acetate buffer: 0.1 M sodium acetate buffer, pH 5.5
D. Gel filtration column: for example, Sephadex® G-200 (1.5 x 45 cm)
E. Coomassie® Protein Assay Reagent (Perbio Science Deutschland GmbH, Bonn, Germany)
F. PBS, phosphate buffered saline: 10 mM sodium phosphate, 150 mM NaCl, pH 7.4

### Method

1 mL of Hydroxylamino-HES 12KD K solution and 1 mL of oxidized Glyco-EPO solution were combined and the reaction mixture was allowed to react with agitation for 16 hours at room temperature. The reaction mixture was applied to Sephadex® G-200 (1.5 x 45 cm) equilibrated with PBS and 1 mL fractions were collected. The protein content of the fractions were monitored with a Coomassie protein assay reagent, all fractions containing the protein conjugate were pooled and the conjugate was obtained by lyophylisation after dialysis against water over night. The result of the conjugation is shown in Figure 24. The observed molecular shift in lane 2 demonstrates that the conjugation was successful. The smear results from the heterogenity of HES. Figure 25 demonstrates that HES is conjugated to a carbohydrate moiety of a carbohydrate side chain.

### Example 13

### Characterisation of galactose oxidase treated EPO N-glycans

Recombinant EPO or partially desialylated EPO forms (generated by limited mild acid hydroysis) were incubated with galactose oxidase in the presence of catalase at 37°C from 30 min - 4 hours at 37°C in 0.05 M Na-phosphate buffer pH 7.0. Progress of the reaction was monitored by removal of 50 µg aliquots of the EPO and subsequent treatment of the protein with polypeptide N-glycanase.

Liberated N-linked oligosaccharides (monitored by SDS-PAGE detection of the de-N-glycosylated polypeptide) were subjected to HPAEC-PAD mapping as described ( Grabenhorst et al., 1999, Nimtz et al., 1993/1994; Schlenke et al., 1999) before and after removal of sialic acids. Quantitation of oxidised galactose residues in individual EPO oligosaccharides was performed by the typical shift observed in HPAEC-PAD and was also verified by MALDI/TOF MS of the oligosaccharide mixtures.

### Example 14

### Characterisation of HAS modified EPO

Separation of HAS modified EPO forms from nonreacted EPO and HAS-precursor molecules was achieved by gel filtration using e.g. Ultrogel AcA 44 / 54 or similar gel filtration media. Alternatively, nonreacted HAS was removed by immuno affinity
isolation of EPO on a 4 mL column containing a monoclonal antibody coupled to Affigel (BioRad) and subsequent separation of unmodified EPO by gel filtration (e.g. using a matrix enabling the separation of globular proteins of a relative molecular mass between 20 kDa and 200 kDa).

HAS modified EPOs were identified by SDS-PAGE analysis (using 12.5 or 10% acrylamide gels) through detection of their higher molecular weight compared to unmodified EPO upon staining of gels with Coomassie Brillant Blue. The higher molecular weight of HAS modified EPO polypeptides was also identified by Western Blot analysis of samples using a polyclonal antibody raised against recombinant human EPO.

N-glycan modification of EPO forms was demonstrated by their successful removal from the EPO protein with polypeptide N-glycanase (recombinant N-glycosidase from Roche, Germany employing 25 units / mg EPO protein at 37°C for 16 hours); analysis by SDS-PAGE resulted in a typical shift of the EPO protein to a migration position of the N-glycosidase treated unmodified EPO of approximately 20 KDa.

Modification of the single desialylated and glacatose oxidase treated EPO O-glycan at Ser 126 was demonstrated by SDS-PAGE migration of the de-N-glycosylated product by detection of its migration position compared to nonreacted de-N-glycosylated EPO. If required, modified EPO was fractionated by RP-HPLC on a C8-phase before SDS-PAGE analysis. HAS O-glycan modification of EPO was also analysed by β-elimination of the O-glycan and detection of the de-O-glycosylated form of EPO in Western blots using a polyclonal antibody raised against recombinant human EPO.

### Example 15

### Quantitation of EPO and modified EPO forms

EPO forms where quantitated by UV measurements as described in Ph.Eur (2000, Erythropoietini solutio concentrata, 1316, 780-785) and compared to the international BRP reference EPO standard. Alternatively, EPO concentrations were determined by a RP-HPLC assay using a RP-C4-column and absorption at 254 nm employing 20, 40 , 80 and 120 µg of the BRP standard EPO reference preparation for calibration.

### Example 16

### In-vitro biological actvity of HES-modified recombinant human EPO:

Purified HES-modified EPO was tested for activity using the erythropoietin bioactivity assay as described by Krystal [Krystal, 1984, Exp. Heamatol., 11, 649-660].

Anemia was induced in NMRI mice by treatment with phenylhydrazine hydrochloride and spleen cells were collected and used as described in [Fibi et al., 1991, Blood, 77, 1203 ff.]. Dilutions of EPO were incubated with 3x10⁵ cells/well in 96-well microtiter plates. After 24 hours at 37° C in a humified atmosphere (5% CO₂) cells were labelled for 4 hours with 1 µCi of ³H-thymidine per well. Incorporated radioactivity was determined by liquid scintillation counting. The International reference EPO standard (BRP-standard) was used for comparison.

Alternatively, EPO bioactivity was measured by an in vitro assay using the EPO-sensitive cell line TF-1 (Kitamura et. al., [J. cell Phys., 140. 323-334]. Exponentially growing cells were washed free of growth factors and were incubated in the presence of serial dilutions of the EPO for further 48 hours. Proliferation of the cells was assessed by using the MTT reduction assay as described by Mosmann [Mosman, 1983, J.Immunol. Methods, 65, 55-63].

### Example 17

### In-vivo activity determination of EPO and HAS-modified EPO forms:

In vivo activity determinations were performed in normocythemic mice by measuring the increase of reticulocytes after 4 days after animals received the foreseen dose of EPO or modified EPO forms. Assays were performed using the BRP EPO standard which was calibrated against the WHO EPO standard in the polycythemic mouse assay. EPO samples were diluted in phosphate buffered saline containing 1 mg/ml of bovine serum albumin (Sigma).

0.5 ml of the EPO test solution in Dulbecco's buffered saline (corresponding to an EPO protein equivalent of a 100, 80, 40 or 20 IU/ml of the BRP standard EPO) were infected subcutaneously per animal. Blood samples were taken after 4 days after injection and reticulocytes were stained with acridine orange; quantitation of reticulocytes was performed by flow-cytometry by counting a total of 30,000 blood cells within 5 hours after the blood sample was taken (see Ph. Eur, 2000, Erythropoietini solutio concentrata, 1316, pages 780-785) and European Pharmacopoeia (1996/2000, attachment 2002).

### Example 18

### In-vivo half-life Determinations

Rabbits were injected intravenously with specified amounts of unmodified or HAS-modified EPO forms. Blood samples were obtained at specified times, and serum was prepared. Serum erythropoietin levels were determined by *in vitro* bioassay or by an EPO-specific commercial ELISA.

### Example 19

### In vivo pharmakokinetics

In mice: Each animal received 300 IU EPO/kg subcutaneously. Seven days after the post-treatment hematocrit of each animal was determined. A substantial increase in hematocrit was observed 9in all animals treated with modified EPO, an expected result in view o the relatively short half-life of untreated EPO. The mean change in hematocrit of the modified EPO-treated group was significantly different from that of the untreated EPO group and that of the control group.

In rabbits: Rabbits were treated with a single dose of unmodified or HAS-modified EPO corresponding to 200 or up to 800 ng/kg body weight. After 2, 6, 16, 24 and 48 hours blood samples were analyzed by using a commercial EPO-specific ELISA for determination of plasma concentrations. Mean plasma EPO concentrations were determined and the average initial half-lives (α-phase) and the terminal half-lives (β-phase) were calculated from the ELISA values as described: (Zettlmissl et al., 1989, J. Biol. Chem., 264, 21153-21159).

### Literature:

Sytkowski, Lunn, Risinger, and Davis, 1999, An Erythropoietin Fusion Protein Comprised of Identical Repeating Domains Exhibitis Enhanced Biological Properites, J. Biol. Chem., 274, 24773-24778.

### Example 20

### Assessment of the in vitro biological activity of HES-modified recombinant human IL-2

Modified IL2 was recovered by gelfiltration on Ultrogel AcA 54. Aliquots of corresponding fraction were sterile filtrated and IL2 bioactivity was determined by using the IL2 dependent murine CTLL-2 cell line [Gillis, Ferm, On, and Smith, 1978, J.Immunol., 120, 2027-2032]. Activity was related to the international reference IL2 standard preparation.

**Table 1**

| **Abreviation** | **Chemical Name** | **Type** | |
|---|---|---|---|
| AMAS | N-(α-Maleimidoacetoxy) succinimide ester | E | 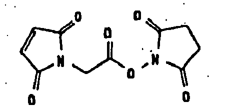 |
| BMPH | N-(β-Maleimidopropionic acid) hydrazide·TFA | A | 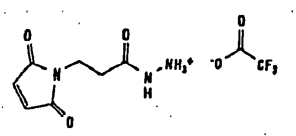 |
| BMPS | N-(β-Maleimidopropyloxy) succinimide ester | E | 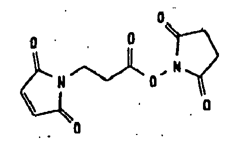 |
| EMCH | N-(ε-Maleimidocaproic acid) hydrazide | A | 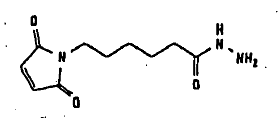 |
| EMCS | N-(ε-Maleimidocaproyloxy) succinimide ester | E | 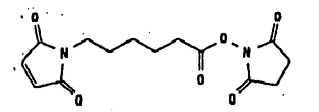 |
| GMBS | N-γ-Maleimidobutyryloxy-succinimide ester | E | 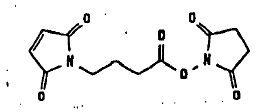 |
| KMUH | N-(κ-Maleimidoundecanoic acid) hydrazide | A | 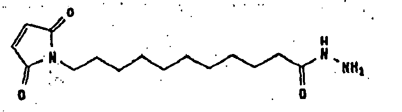 |
| LC-SMCC | Succinimidyl 4-(N-maleimidomethyl) cyclohexane-1-carboxy-(6-amido-caproate) | E | 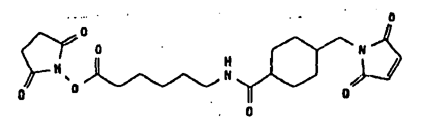 |
| LC-SPDP | Succinimidyl 6-(3'-[2-pyridyl-dithio]propionamido) hexanoate | F | 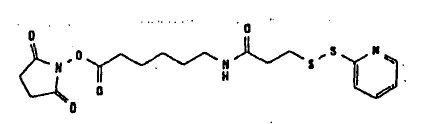 |
| MBS | m-Maleimidobenzoyl-N-hydroxysuccinimide ester | E | 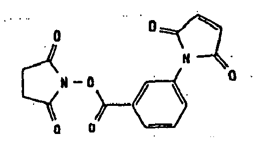 |
| M₂C₂H | 4-(N-Maleimidomethyl)-cyclohexane-1-carboxyl-hydrazide·HCl·1/2 dioxane | A | 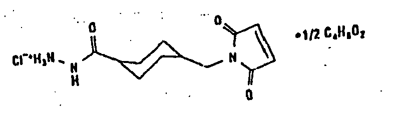 |
| MPBH | 4-(4-N-Maleimidophenyl)-butyric acid hydazide·HCl | A | 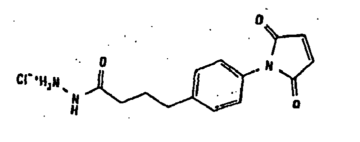 |
| SATA | N-Succinimidyl S-acetylthio-acetate | H | 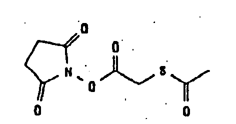 |
| SATP | N-Succinimidyl S-acetylthio-propionate | H | 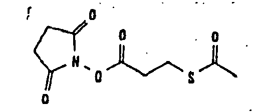 |
| SBAP | Succinimidyl 3-(bromoacetamido) propionate | D | 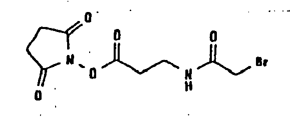 |
| SIA | N-Succinimidyl iodoacetate | C | 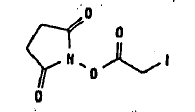 |
| SIAB | N-Succinimidyl(4-iodoacetyl)aminobenzoate | C. | 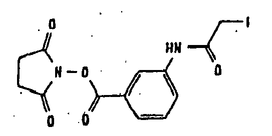 |
| SMCC | Succinimidyl 4-(N-maleimidomethyl) cyclohexane-1-carboxylate | E | 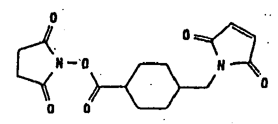 |
| SMPB | Succinimidyl 4-(p-maleimidophenyl)butyrate | E | 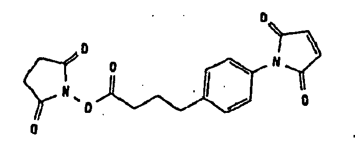 |
| SMPH | Succinimidyt-6-(β-maleimidopropionamido) hexanoate | E | 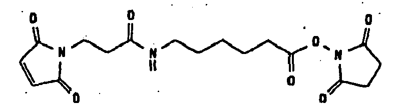 |
| SMPT | 4-Succinimidyloxy-carbonyl-methyl-α-(2-pyridyldithio)toluene | F | 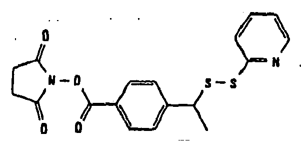 |
| SPDP | N-Succinimidyl 3-(2-pyridyldithio)propionate | F | 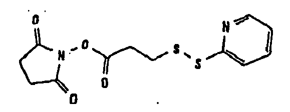 |
| Sulfo-EMCS | N-(ε-Maleimidocaproyloxy) sulfosuccinimide ester | E | 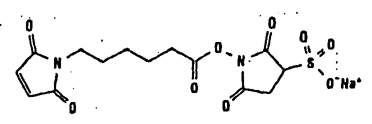 |
| Sulfo-GMBS | N-γ-Maleimidobutryloxy-sulfosuccinimide ester | E | 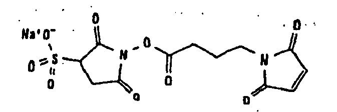 |
| Sulfo-KMUS | N-(κ-Maleimidoundecanoyloxy)-sulfosuccinimide ester | E | 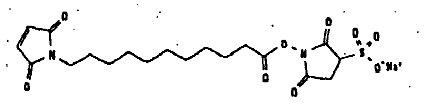 |
| Sulfo-LC-SPDP | Sulfosuccinimidyl 6-(3'-[2-pyridyldithio]propionamido) hexanoate | F | 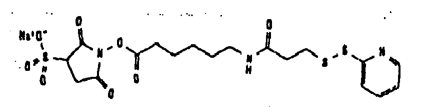 |
| Sulfo-MBS | m-Maleimidobenzoyl-N-hydroxysulfosuccinimide ester | E | 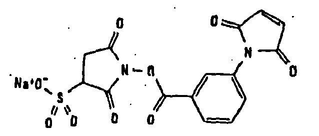 |
| Sulfo-SIAB | Sulfosuccinimidyl(4-iodoacetyl)aminobenzoate | C | 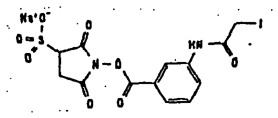 |
| Sulfo-SMCC | Sulfosuccinimidyl 4-(N-maleimidomethyl) cyclohexane-1-carboxylate | E | 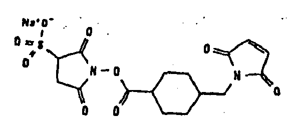 |
| Sulfo-SMPB | Sulfosuccinimidyl 4-(p-maleimidophenyl)butyrate | E | 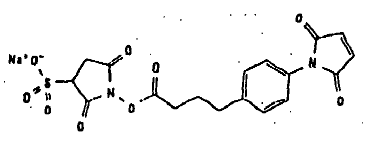 |
| Sulfo-LC-SMPT | Suflosuccinimidyl 6-(α-methyl-α-[2-pyridyldithio]-toluamido)hexanoate | F | 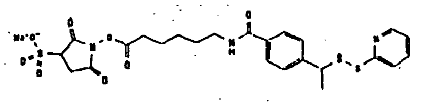 |
| SVSB | N-Succinimidyl-(4-vinylsulfonyl)benzoate | G | 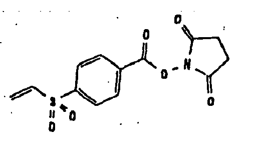 |

**Table 2 Monosaccharide compositional analysis of glycans from HES-modified EPO and control samples**

| ****Mono- saccharide** | **I.** Glycans from **A2** | **II.** Glycans from **EPO-GT-1A** | **III.** Glycans from **K2** | **III.** Glycans from **A2** | **IV.** Glycans from **EPO-GT-1A** | **V.** Glycans from **K2** | **VI.** Cystein modified **EPO protein*** |
|---|---|---|---|---|---|---|---|
| fucose | 1,935 | 3,924 | 2,602 | 2,246 | 4,461 | 2,601 | 2,181 |
| mannose | 6,028 | 11,020 | 9,198 | 6,379 | 11,668 | 6,117 | 6,260 |
| galactose | 8,886 | 19,935 | 14,427 | 10,570 | 16,911 | 11,555 | 10,386 |
| glucose | 17,968 | --- | --- | 21,193 | trace | trace | 33,021 |
| GlcNAc | 7,839 | 21,310 | 14,440 | 11,360 | 15,953 | 10,503 | 10,498 |
| GlcHe1 | 5,583 | --- | --- | 5,926 | --- | --- | 14,857 |
| GlcHe2 | 1,380 | --- | --- | 1,552 | --- | --- | 3,775 |
| NeuNAc | 5,461 | 822 | 4,504 | 3,895 | 4,871 | 13,562 | 13,003 |
| inositol | 1,230 | 2,310 | 1,620 | 2,050 | 1,320 | 1,134 | 1,087 |

| | | | | | | | |
|---|---|---|---|---|---|---|---|
| * the equivalent of Cys-HES-modified EPO protein was subjected to compositional analysis; the EPO protein was isolated from the HES-incubation mixture by chromatography on a Q-Sepharose column as described above and was desalted by centrifugation using a Vivaspin 5 separation device. ** Monosaccharide determinations were performed from single GC runs of the pertrimethylsilylated methylglycosides; the electronical integration values of peaks are given without correction for losses during the derivatisation procedure and recoveries of each compound. | | | | | | | |

**Table 3**

| **Sample No.** | **Sample description** | **Calculated specific activity of EPO sample** (based on A280 nm and RP-HPLC determination) |
|---|---|---|
| 850247 | 1. HES-modified EPO A2 | **344,000 U/mg** |
| 850248 | 2. EPO-GT-1-A | **82,268 U/mg** |
| 850249 | 3. Control EPO K2 | **121,410 U/mg** |
| 850250 | 4. BRP EPO standard | **86,702 U/mg** |
| 850251 | 1. diluted with 4 volume of PBS | **309,129 U/mg** |
| 850252 | 2. diluted with 4 volume of PBS | **94,500 U/mg** |
| 850253 | 3. diluted with 4 volume of PBS | **114,100 U/mg** |
| 850254 | 4. diluted with 4 volume of PBS | **81,200 U/mg** |
| 850255 | 1. diluted with 4 volume of PBS | **230,720 U/mg** |

In the following, preferred methods and hydroxyalkyl starch derivatives of the present invention are listed resulting from the following embodiments 1 to 52 including the combinations of these embodiments as explicitly given:
1. A method of producing a hydroxyalkyl starch derivative, said hydroxyalkyl starch having a structure according to formula (I) 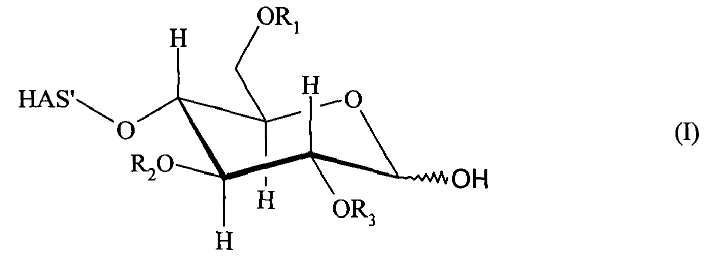 comprising reacting
   - hydroxyalkyl starch of formula (I) at its optionally oxidized reducing end or
   - a hydroxyalkyl starch derivative, obtainable by reacting hydroxyalkyl starch of formula (I) at its optionally oxidized reducing end with a compound (D), said compound (D) comprising
      -- at least one functional group Z₁ capable of being reacted with the optionally oxidized reducing end of the hydroxyalkyl starch, and
      -- at least one functional group W,
      with a compound (L) comprising
   - at least one functional group Z₁ capable of being reacted with said hydroxyalkyl starch, or at least one functional group Z₂ capable of being reacted with functional group W comprised in said hydroxyalkyl starch derivative, and
   - at least one functional group X capable of being reacted with a functional group Y of a further compound (M),
   wherein said functional group Y is selected from the group consisting of an aldehyd group, a keto group, a hemiacetal group, an acetal group, and a thio group.
2. The method of embodiment 1 wherein R₁, R₂ and R₃ are independently hydrogen or a linear or branched hydroxyalkyl group.
3. The method of embodiment 2 wherein R₁, R₂ and R₃ are independently hydrogen or a 2-hydroxyethyl group.
4. The method of any of embodiments 1 to 3 wherein the hydroxyalkyl starch is hydroxyethyl starch.
5. The method of any of embodiments 1 to 4 wherein the functional group Z₁ comprises the structure -NH-.
6. The method of embodiment 5 wherein Z₁ is selected from the group consisting of 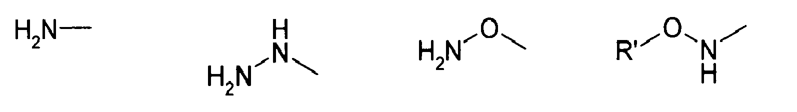 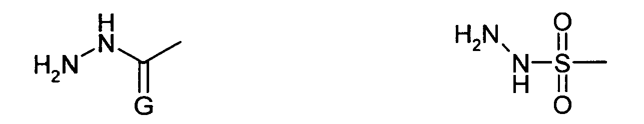 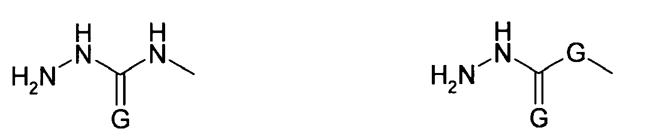 wherein G is O or S and, if present twice, independently O or S, and R' is methyl.
7. The method of any of embodiments 1 to 6 wherein the functional group Y is selected from the group consisting of an aldehyd group, a keto group, a hemiacetal group, and an acetal group, and the functional group X comprises the structure -NH-.
8. The method of embodiment 7 wherein X is selected from the group consisting of 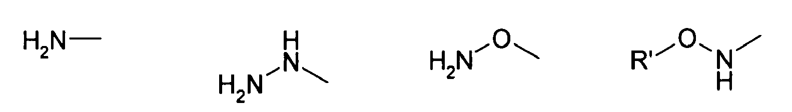 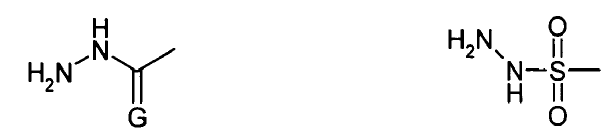 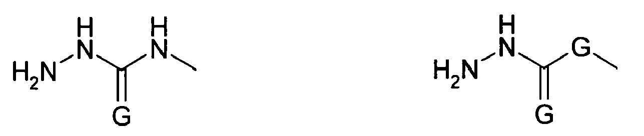 wherein G is O or S and, if present twice, independently O or S, and R' is methyl.
9. The method of any of embodiments 1 to 6 wherein the functional group Y is -SH and the functional group X is selected from the group consisting of 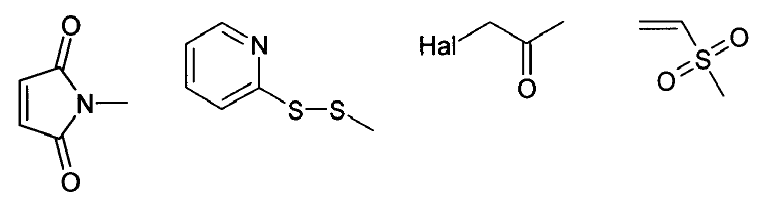 wherein Hal is Cl, Br or I.
10. The method of any of embodiments 1 to 9 wherein the functional group W or the functional group Z₂ is -SH and the functional group Z₂ or the functional group
   W is selected from the group consisting of 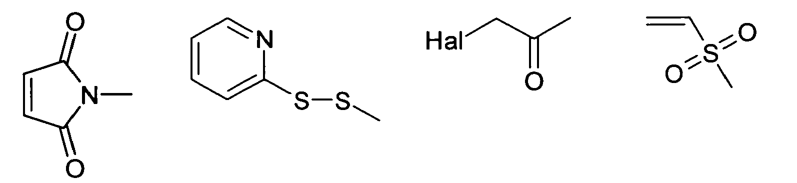 wherein Hal is Cl, Br, or I.
11. The method of any of embodiments 1 to 9 wherein the functional group W or the functional group Z₂ is selected from the group consisting of an activated ester or a carboxy group which is optionally transformed into an activated ester and the functional group Z₂ or the functional group W is selected from the group consisting of 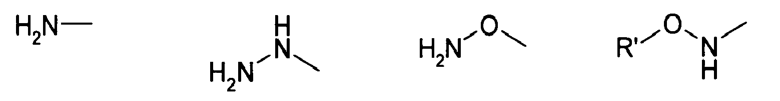 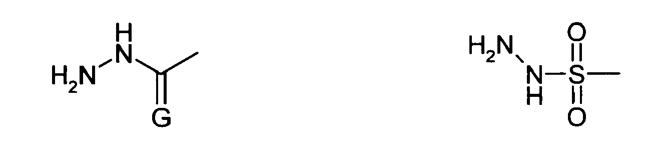 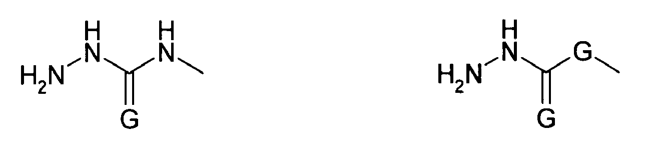 wherein G is O or S and, if present twice, independently O or S, and R' is methyl.
12. The method of any of embodiments 1 to 11 wherein the reducing end of the hydroxyalkyl starch is not oxidized prior to the reaction with compound (D) or compound (L), said hydroxyalkyl starch thus having a structure according to formula (I) 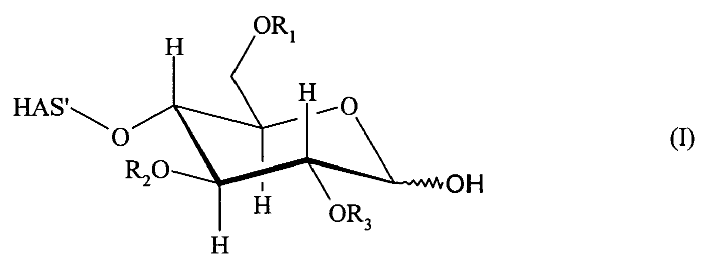
13. The method of any of embodiments 1 to 11 wherein the reducing end of the hydroxyalkyl starch is oxidized prior to the reaction with compound (D) or compound (L), said hydroxyalkyl starch thus having a structure according to formula (IIa) 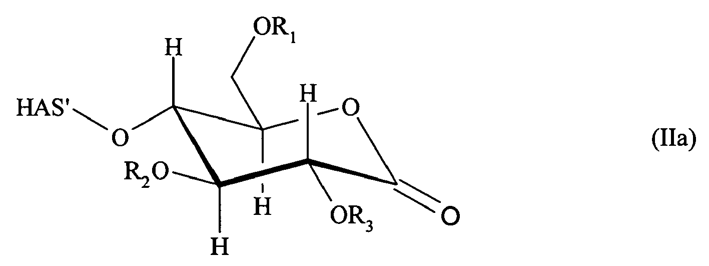 and/or according to formula (IIb) 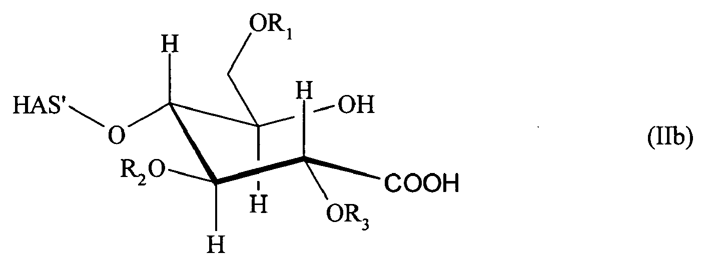
14. The method of embodiment 13 wherein the reducing end is oxidized by an alkaline iodine solution.
15. The method of any of embodiments 1 to 9 and 12 to 14 wherein hydroxyalkyl starch is reacted with a compound (L) via the reaction of functional group Z₁ with the optionally oxidized reducing end of the hydroxyalkyl starch and the resulting reaction product is reacted with a further compound (M) via the reaction of the functional group X comprised in compound (L) with the functional group Y comprised in compound (M).
16. The method of any of embodiments 1 to 9 and 12 to 14 wherein hydroxyalkyl starch is reacted with a compound (L) via the reaction of functional group Z₁ with the optionally oxidized reducing end of the hydroxyalkyl starch, where compound (L), prior to the reaction with hydroxyalkyl starch, is reacted with a further compound (M) via the reaction of functional group X comprised in compound (L) with the functional group Y comprised in compound (M).
17. The method of any of embodiments 1 to 14 wherein hydroxyalkyl starch is reacted with a compound (D) via the reaction of the functional group Z₁ comprised in compound (D), with the optionally oxidized reducing end of the hydroxyalkyl starch to give a first hydroxyalkyl starch derivative, and where the first hydroxyalkyl starch derivative is reacted with a compound (L) via the reaction of functional group Z₂ comprised in compound (L) with the functional group W comprised in compound (D) to give a second hydroxyalkyl starch derivative.
18. The method of embodiment 17 wherein the second hydroxyalkyl starch derivative is reacted with a further compound (M) via the reaction of functional group X comprised in compound (L) with the functional group Y comprised in compound (M).
19. The method of any of embodiments 1 to 18 wherein hydroxyalkyl starch is reacted with a compound (D) via the reaction of functional group Z₁ comprised in compound (D) with the optionally oxidized reducing end of the hydroxyalkyl starch to give a first hydroxyalkyl starch derivative, and where the first hydroxyalkyl starch derivative is reacted, via the reaction of the functional group W, comprised in compound (D), and the functional group Z₂, comprised in compound (L), with compound (L), where compound (L), prior to the reaction with the first hydroxyalkyl starch derivative, is reacted with a further compound (M) via the reaction of functional group X comprised in compound (L) with the functional group Y comprised in compound (M).
20. The method of any of embodiments 1 to 19 wherein the at least one further compound (M) is a polypeptide.
21. The method of embodiment 20 wherein the polypeptide is erythropoietin.
22. A hydroxyalkyl starch derivative obtainable by a method of producing a hydroxyalkyl starch derivative, said hydroxyalkyl starch having a structure according to formula (I) 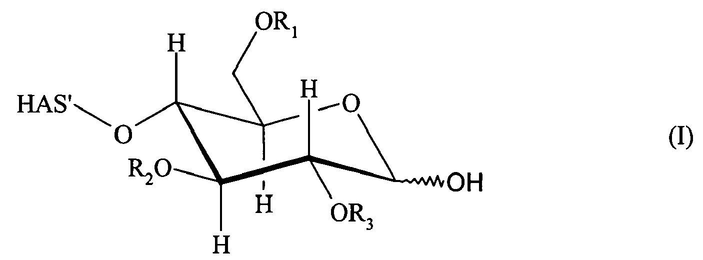 comprising reacting
   - hydroxyalkyl starch of formula (I) at its optionally oxidized reducing end or
   - a hydroxyalkyl starch derivative, obtainable by reacting hydroxyalkyl starch of formula (I) at its optionally oxidized reducing end with a compound (D), said compound (D) comprising
      -- at least one functional group Z₁ capable of being reacted with the optionally oxidized reducing end of the hydroxyalkyl starch, and
      -- at least one functional group W,
      with a compound (L) comprising
   - at least one functional group Z₁ capable of being reacted with said hydroxyalkyl starch, or at least one functional group Z₂ capable of being reacted with functional group W comprised in said hydroxyalkyl starch derivative, and
   - at least one functional group X capable of being reacted with a functional group Y of a further compound (M),
   wherein said functional group Y is selected from the group consisting of an aldehyd group, a keto group, a hemiacetal group, an acetal group, or a thio group.
23. The hydroxyalkyl starch derivative of embodiment 22 wherein R₁, R₂ and R₃ are independently hydrogen or a linear or branched hydroxyalkyl group.
24. The hydroxyalkyl starch derivative of embodiment 23 wherein R₁, R₂ and R₃ are independently hydrogen or a 2-hydroxyethyl group.
25. The hydroxyalkyl starch derivative of any of embodiments 22 to 24 wherein the hydroxyalkyl starch is hydroxyethyl starch.
26. The hydroxyalkyl starch derivative of any of embodiments 22 to 25 wherein the functional group Z₁ comprises the structure -NH-.
27. The hydroxyalkyl starch derivative of embodiment 26 wherein Z₁ is selected from the group consisting of 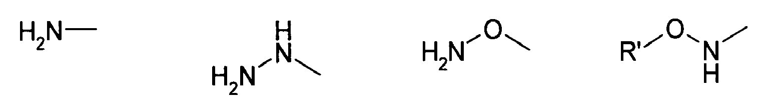 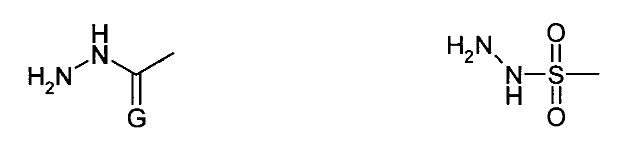 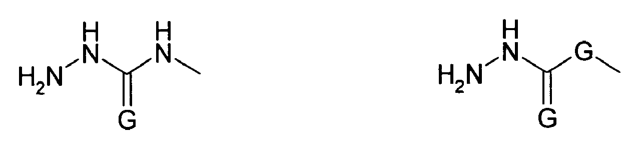 wherein G is O or S and, if present twice, independently O or S, and R' is methyl.
28. The hydroxyalkyl starch derivative of any of embodiments 22 to 27 wherein the functional group Y is selected from the group consisting of an aldehyd group, a keto group, a hemiacetal group, and an acetal group, and the functional group X comprises the structure -NH-.
29. The hydroxyalkyl starch derivative of embodiment 28 wherein X is selected from the group consisting of 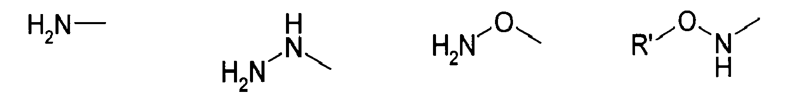 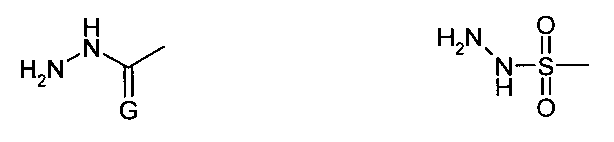 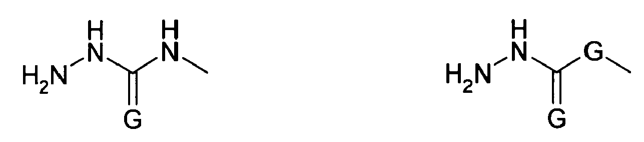 wherein G is O or S and, if present twice, independently O or S, and R' is methyl.
30. The hydroxyalkyl starch derivative of any of embodiments 22 to 27 wherein the functional group Y is -SH and the functional group X is selected from the group consisting of 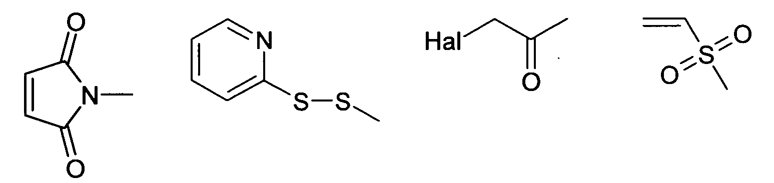 wherein Hal is Cl, Br or I.
31. The hydroxyalkyl starch derivative of any of embodiments 22 to 30 wherein the functional group W or the functional group Z₂ is -SH and the functional group
   Z₂ or the functional group W is selected from the group consisting of 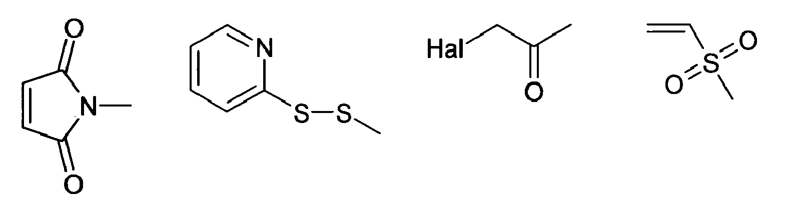 wherein Hal is Cl, Br, or I.
32. The hydroxyalkyl starch derivative of any of embodiments 22 to 30 wherein the functional group W or the functional group Z₂ is selected from the group consisting of an activated ester or a carboxy group which is optionally transformed into an activated ester and the functional group Z₂ or the functional group W is selected from the group consisting of 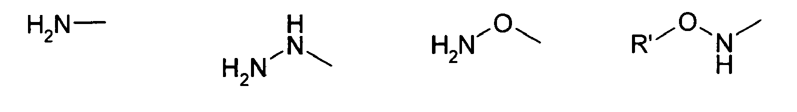 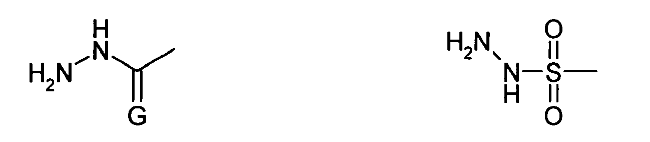 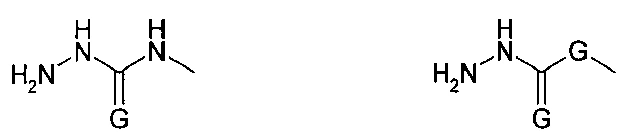 wherein G is O or S and, if present twice, independently O or S, and R' is methyl.
33. The hydroxyalkyl starch derivative of any of embodiments 22 to 32 wherein the reducing end of the hydroxyalkyl starch is not oxidized prior to the reaction with compound (D) or compound (L), said hydroxyalkyl starch thus having a structure according to formula (I) 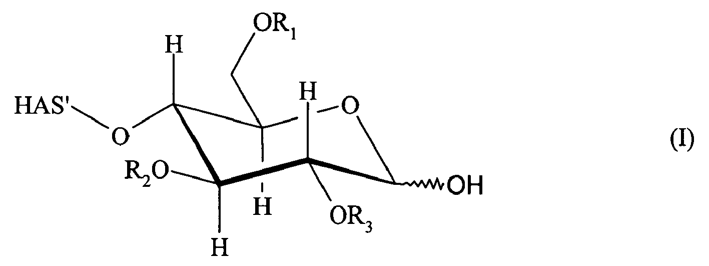
34. The hydroxyalkyl starch derivative of any of embodiments 22 to 32 wherein the reducing end of the hydroxyalkyl starch is oxidized prior to the reaction with compound (D) or compound (L), said hydroxyalkyl starch thus having a structure according to formula (IIa) 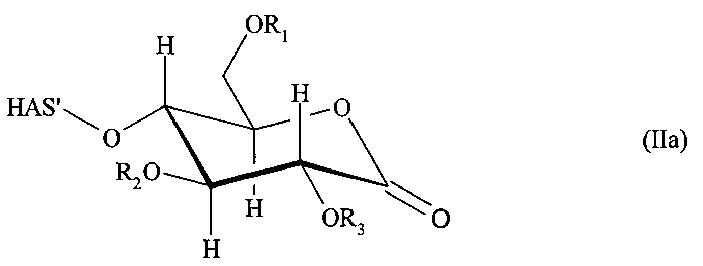 and/or according to formula (IIb) 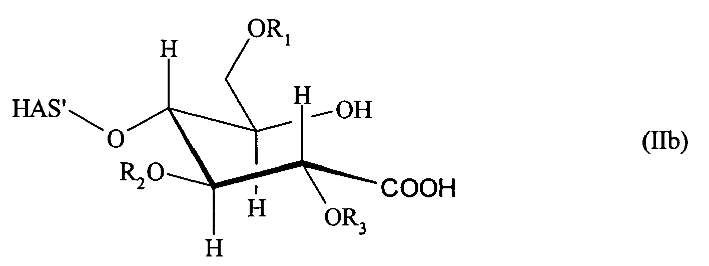
35. The hydroxyalkyl starch derivative of embodiment 34 wherein the reducing end is oxidized by an alkaline iodine solution.
36. The hydroxyalkyl starch derivative of any of embodiments 22 to 30 and 33 to 35 wherein hydroxyalkyl starch is reacted with a compound (L) via the reaction of functional group Z₁ with the optionally oxidized reducing end of the hydroxyalkyl starch and the resulting reaction product is reacted with a further compound (M) via the reaction of the functional group X comprised in compound (L) with the functional group Y comprised in compound (M).
37. The hydroxyalkyl starch derivative of any of embodiments 22 to 30 and 33 to 35 wherein hydroxyalkyl starch is reacted with a compound (L) via the reaction of functional group Z₁ with the optionally oxidized reducing end of the hydroxyalkyl starch, where compound (L), prior to the reaction with hydroxyalkyl starch, is reacted with a further compound (M) via the reaction of functional group X comprised in compound (L) with the functional group Y comprised in compound (M).
38. The hydroxyalkyl starch derivative of any of embodiments 22 to 35 wherein hydroxyalkyl starch is reacted with a compound (D) via the reaction of the functional group Z₁ comprised in compound (D), with the optionally oxidized reducing end of the hydroxyalkyl starch to give a first hydroxyalkyl starch derivative, and where the first hydroxyalkyl starch derivative is reacted with a compound (L) via the reaction of functional group Z₂ comprised in compound (L) with the functional group W comprised in compound (D) to give a second hydroxyalkyl starch derivative.
39. The hydroxyalkyl starch derivative of embodiment 38 wherein the second hydroxyalkyl starch derivative is reacted with a further compound (M) via the reaction of functional group X comprised in compound (L) with the functional group Y comprised in compound (M).
40. The hydroxyalkyl starch derivative of any of embodiments 22 to 35 wherein hydroxyalkyl starch is reacted with a compound (D) via the reaction of functional group Z₁ comprised in compound (D) with the optionally oxidized reducing end of the hydroxyalkyl starch to give a first hydroxyalkyl starch derivative, and where the first hydroxyalkyl starch derivative is reacted, via the reaction of the functional group W, comprised in compound (D), and the functional group Z₂, comprised in compound (L), with compound (L), where compound (L), prior to the reaction with the first hydroxyalkyl starch derivative, is reacted with a further compound (M) via the reaction of functional group X comprised in compound (L) with the functional group Y comprised in compound (M).
41. The hydroxyalkyl starch derivative of any of embodiments 22 to 40 wherein the at least one further compound (M) is a polypeptide.
42. The hydroxyalkyl starch derivative of embdoiment 41 wherein the polypeptide is erythropoietin.
43. A pharmaceutical composition comprising, in a therapeutically effective amount, a hydroxyalkyl starch derivative obtainable by a method of producing a hydroxyalkyl starch derivative, said hydroxyalkyl starch having a structure according to formula (I) 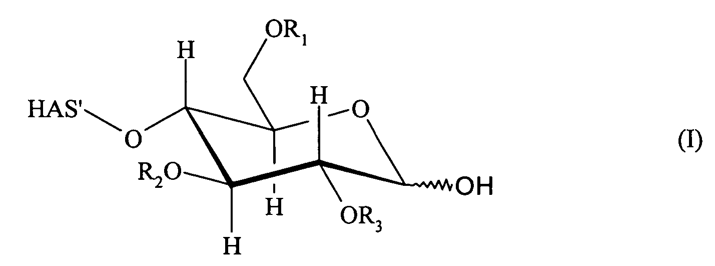 comprising reacting,
   - hydroxyalkyl starch of formula (I) at its optionally oxidized reducing end or
   - a hydroxyalkyl starch derivative, obtainable by reacting hydroxyalkyl starch of formula (I) at its optionally oxidized reducing end with a compound (D), said compound (D) comprising
      -- at least one functional group Z₁ capable of being reacted with the optionally oxidized reducing end of the hydroxyalkyl starch, and
      -- at least one functional group W,
      with a compound (L) comprising
   - at least one functional group Z₁ capable of being reacted with said hydroxyalkyl starch, or at least one functional group Z₂ capable of being reacted with functional group W comprised in said hydroxyalkyl starch derivative, and
   - at least one functional group X capable of being reacted with a functional group Y of a further compound (M),
   wherein said functional group Y is selected from the group consisting of an aldehyd group, a keto group, a hemiacetal group, an acetal group, or a thio group, said method of producing a hydroxyalkyl starch derivative further comprising reacting the reaction product comprising hydroxyalkyl starch, compound (L) and optionally compound (D) with a further compound (M) wherein the at least one further compound is a polypeptide.
44. The pharmaceutical composition of embodiment 43 wherein the polypeptide is AT III.
45. The pharmaceutical composition of embodiment 43 wherein the polypeptide is erythropoietin.
46. The pharmaceutical composition of any of embodiments 43 to 45 wherein the functional group Y is -SH and compound (L) is a compound of general formula Z₁-L'-X where the functional group Z₁ is selected from the group consisting of 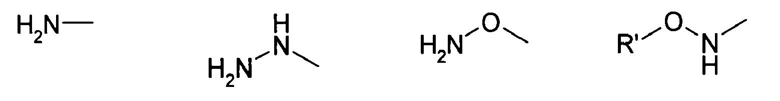 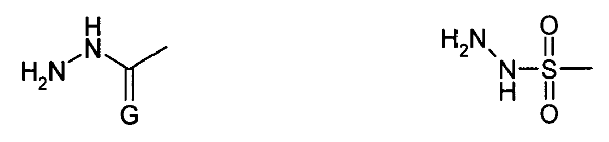 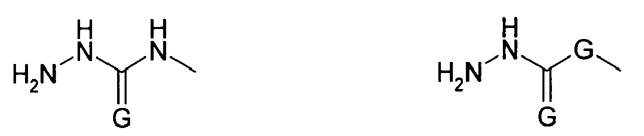 wherein G is O or S and, if present twice, independently O or S, and R' is methyl, and where the functional group X is selected from the group consisting of 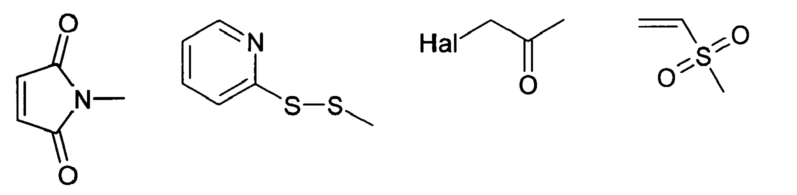 wherein Hal is Cl, Br or I, and where L' is an organic chain bridging Z₁ and X or where L' is absent.
47. The pharmaceutical composition of any of embodiments 43 to 45 wherein the functional group Y is selected from the group consisting of an aldehyd group, a keto group, a hemiacetal group, and an acetal group, and compound (L) is a compound of general formula Z₁-L'-X where the functional group Z₁ is selected from the group consisting of 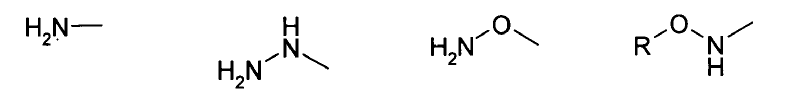 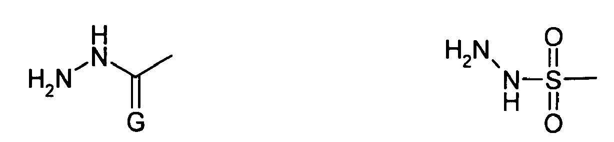 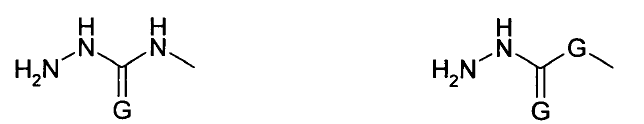 wherein G is O or S and, if present twice, independently O or S, and R is alkyl, cycloalkyl, aryl, aralkyl, arylcycloalkyl, alkaryl, or cycloalkylaryl, and where the functional group X is selected from the group consisting of 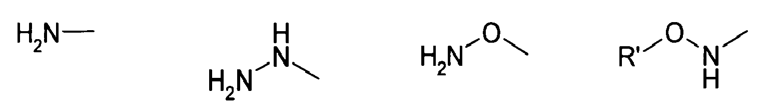 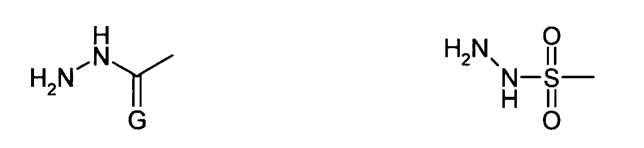 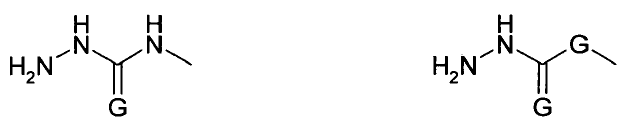 wherein G is O or S and, if present twice, independently O or S, and R' is methyl, and where L' is an organic chain bridging Z₁ and X or where L' is absent.
48. The pharmaceutical composition of any of embodiments 43 to 45 wherein the functional group Y is -SH, compound (D) is a compound of general formula Z₁-D'-W, and compound (L) is a compound of general formula Z₂-L'-X, where the functional group Z₁ is selected from the group consisting of 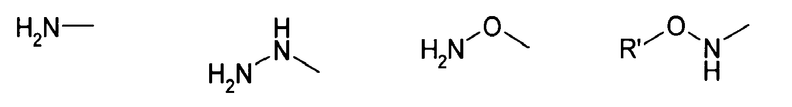 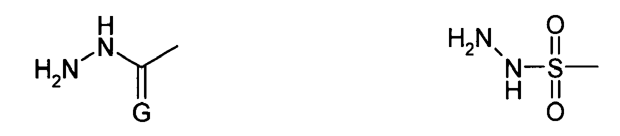 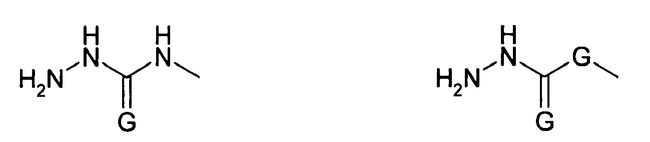 wherein G is O or S and, if present twice, independently O or S, and R is methyl, where the functional group X is selected from the group consisting of 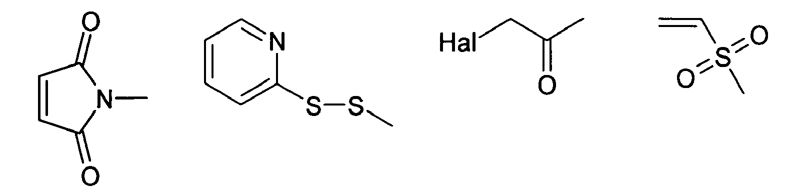 wherein Hal is Cl, Br or I, where the functional group W or the functional group Z₂ is -SH and the functional group Z₂ or the functional group W is selected from the group consisting of 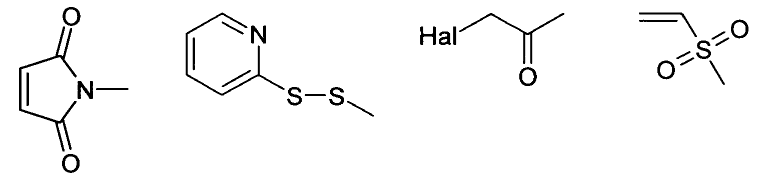 wherein Hal is Cl, Br, or I., or where the functional group W or the functional group Z₂ is selected from the group consisting of an activated ester or a carboxy group which is optionally transformed into an activated ester and the functional group Z₂ or the functional group W is selected from the group consisting of 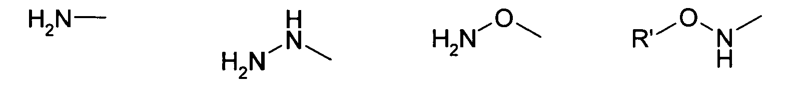 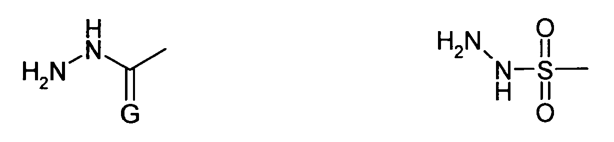 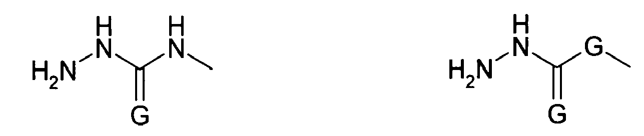 wherein G is O or S and, if present twice, independently O or S, and R' is methyl, and where D' is an organic chain bridging Z₁ and W or where D' is absent and where L' is an organic chain bridging Z₂ and X or where L' is absent.
49. The pharmaceutical composition of any of embodiments 43 to 45 wherein the functional group Y is selected from the group consisting of an aldehyd group, a keto group, a hemiacetal group, and an acetal group, compound (D) is a compound of general formula Z₁-D'-W, and compound (L) is a compound of general formula Z₂-L'-X, where the functional group Z₁ is selected from the group consisting of 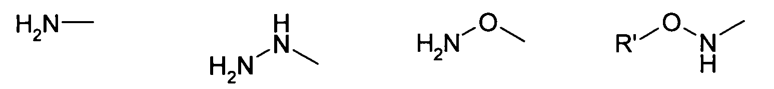 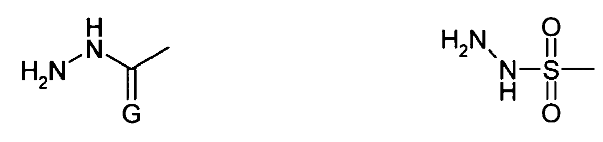 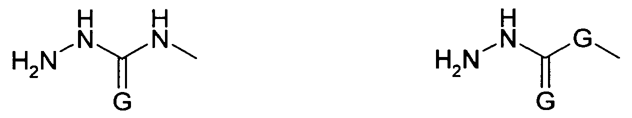 wherein G is O or S and, if present twice, independently O or S, and R' is methyl, where the functional group X is selected from the group consisting of 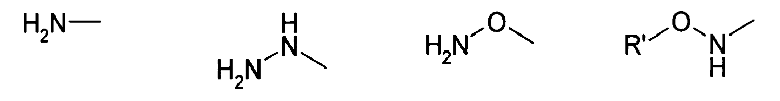 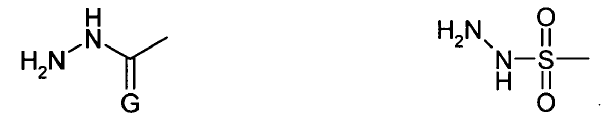 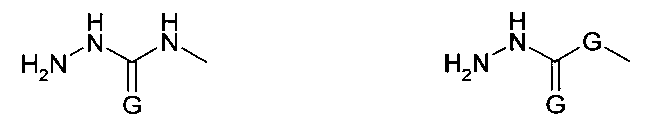 wherein G is O or S and, if present twice, independently O or S, and R' is methyl, where the functional group W or the functional group Z₂ is -SH and the functional group Z₂ or the functional group W is selected from the group consisting of 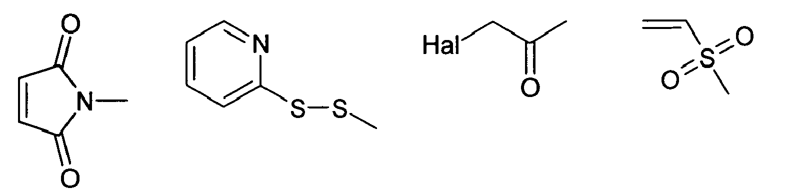 wherein Hal is Cl, Br, or I., or where the functional group W or the functional group Z₂ is selected from the group consisting of an activated ester or a carboxy group which is optionally transformed into an activated ester and the functional group Z₂ or the functional group W is selected from the group consisting of 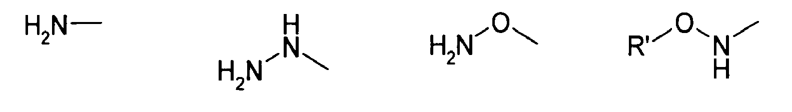 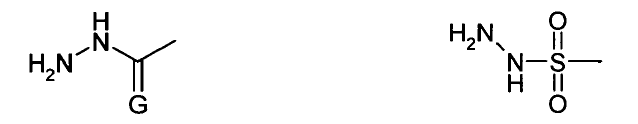 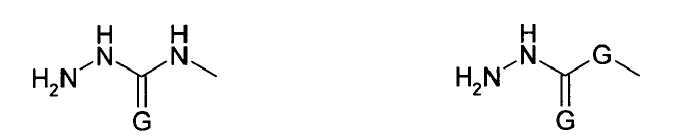 wherein G is O or S and, if present twice, independently O or S, and R' is methyl, , and where D' is an organic chain bridging Z₁ and W or where D' is absent and where L' is an organic chain bridging Z₂ and X or where L' is absent.
50. The pharmaceutical composition of embodiment 43 wherein hydroxyethyl starch is reacted in an aqueous medium with a compound according to the following formula 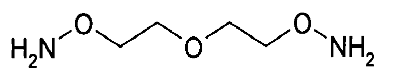 and the reaction product is reacted with erythropoietin.
51. The pharmaceutical composition of embodiment 50 wherein the erythropoietin is oxidised with sodium periodate prior to the reaction.
52. The pharmaceutical composition of embodiment 50 wherein the erythropoietin is partially desialylated and subsequently oxidised with sodium periodate prior to the reaction.

## Claims

1. A method of producing a hydroxyalkyl starch derivative, said hydroxyalkyl starch having a structure according to formula (I) 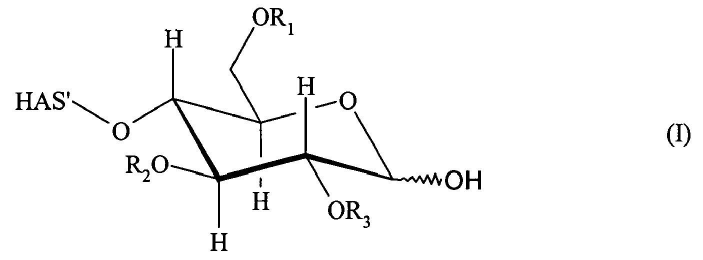 comprising reacting
- hydroxyalkyl starch of formula (I) at its optionally oxidized reducing end with a compound (L), comprising at least one functional group Z₁ capable of being reacted with said hydroxyalkyl starch, and at least one functional group X capable of being reacted with a functional group Y of a further compound (M), wherein the formation of a chemical linkage between compound (L) and hydroxyalkyl starch is achieved by reaction of the functional group Z₁ with the optionally oxidized reducing end of the hydroxyalkyl starch, and wherein Z₁ is 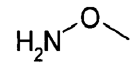 or
- a hydroxyalkyl starch derivative, obtainable by reacting hydroxyalkyl starch of formula (I) at its non-oxidized reducing end with a compound (D), comprising at least one functional group Z₁ capable of being reacted with the non-oxidized reducing end of the hydroxyalkyl starch, and at least one functional group W, with a compound (L), comprising at least one functional group Z₂ capable of being reacted with the functional group W comprised in said hydroxyalkyl starch derivative, and at least one functional group X capable of being reacted with a functional group Y of a further compound (M), wherein the formation of a chemical linkage between compound (D) and hydroxyalkyl starch is achieved by reaction of the functional group Z₁ with the non-oxidized reducing end of the hydroxyalkyl starch, wherein the functional group Z₁ comprises the structure -NH-,
wherein said functional group Y of the further compound (M) is a thio group.

2. The method as claimed in claim 1, wherein R₁, R₂ and R₃ are independently hydrogen or a linear or branched hydroxyalkyl group, preferably wherein R₁, R₂ and R₃ are independently hydrogen or a 2-hydroxyethyl group, more preferably wherein the hydroxyalkyl starch is hydroxyethyl starch.

3. The method as claimed in claim 1 or 2, wherein the functional group X is selected from the group consisting of 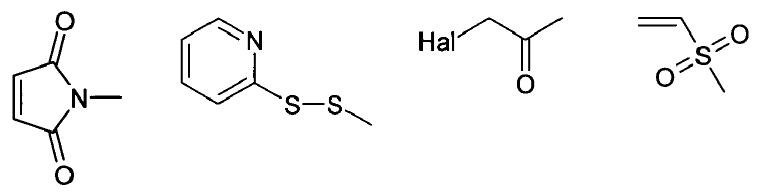 wherein Hal is Cl, Br or I.

4. The method as claimed in any of claims 1 to 3, wherein hydroxyalkyl starch is reacted with a compound (D) via the reaction of the functional group Z₁ comprised in compound (D), and wherein the functional group Z₁ comprised in compound D is is selected from the group consisting of 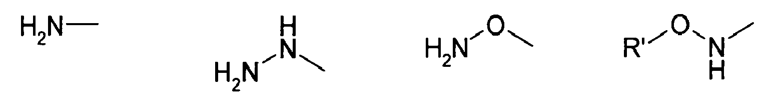 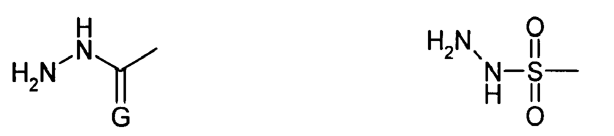 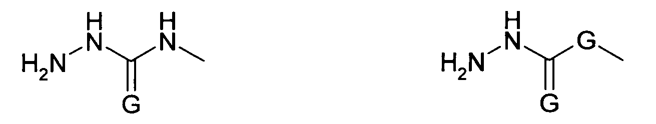 wherein G is O or S and, if present twice, independently O or S, and R' is methyl.

5. The method as claimed in claim 4, wherein hydroxyalkyl starch is reacted with compound (D) via the reaction of the functional group Z₁ comprised in compound (D) in an aqueous medium, wherein Z₁ is H₂N- and wherein the reaction is a reductive amination.

6. The method as claimed in claim 4, wherein Z₁ is

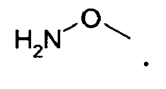

7. The method as claimed in any of claims 1 to 6, wherein the functional group W or the functional group Z₂ is -SH and the functional group Z₂ or the functional group W is selected from the group consisting of 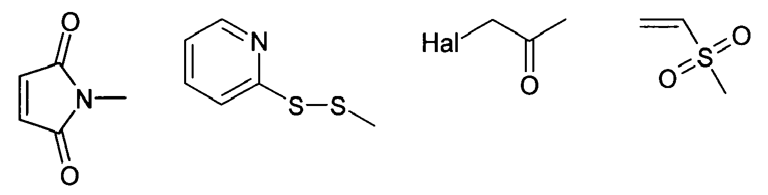 wherein Hal is Cl, Br, or I, preferably wherein the functional group W is -SH and the functional group Z₂ is selected from the group consisting of 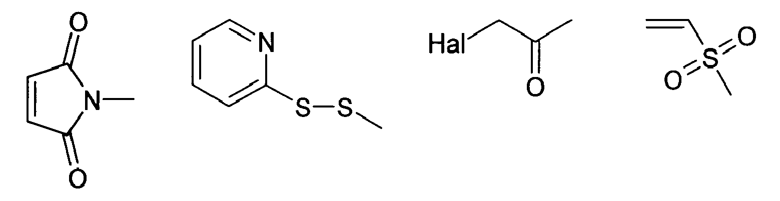 wherein Hal is Cl, Br, or I.

8. The method as claimed in any of claims 1 to 7, wherein the hydroxyalkyl starch is reacted with a compound (D) via the reaction of the functional group Z₁ comprised in compound (D) with the non-oxidized reducing end of the hydroxyalkyl starch to give a first hydroxyalkyl starch derivative, wherein the first hydroxyalkyl starch derivative is reacted with a compound (L) via the reaction of functional group Z₂ comprised in compound (L) with the functional group W comprised in compound (D) to give a second hydroxyalkyl starch derivative, and wherein the second hydroxyalkyl starch derivative is reacted with a further compound (M) via the reaction of functional group X comprised in compound (L) with the functional group Y comprised in compound (M).

9. The method as claimed in any of claims 1 to 3, wherein hydroxyalkyl starch is reacted with a compound (L) via the reaction of functional group Z₁ with the optionally oxidized reducing end of the hydroxyalkyl starch and the resulting reaction product is reacted with a further compound (M) via the reaction of the functional group X comprised in compound (L) with the functional group Y comprised in compound (M).

10. The method as claimed in any of claims 1 to 3 and 9, wherein the reducing end of the hydroxyalkyl starch of formula (I) is not oxidized prior to the reaction with compound (L).

11. The method as claimed in any of claims 1 to 10, wherein the at least one further compound (M) is a polypeptide.

12. A hydroxyalkyl starch derivative obtainable by a method as claimed in any of claims 1 to 11.

13. A pharmaceutical composition comprising, in a therapeutically effective amount, a hydroxyalkyl starch derivative as claimed in claim 12.
